# EUROPEAN PATENT SPECIFICATION

(11) **EP 2 658 996 B1**
(45) Date of publication and mention of the grant of the patent: **19.10.2016**
(21) Application number: 11802763.0
(22) Date of filing: 28.12.2011
(51) Int. Cl.: C12Q 1/68

(54) **COMPLEX SET OF MIRNAS AS NON-INVASIVE BIOMARKERS FOR PROSTATE DISEASES**
KOMPLEXER SATZ AUS MI-RNAS ALS NICHTINVASIVE BIOMARKER FÜR PROSTATAERKRANKUNGEN
ENSEMBLE COMPLEXE DE MI-ARNS EN TANT QUE BIOMARQUEURS NON INVASIFS DE MALADIES DE LA PROSTATE

(30) Priority: 30.12.2010 EP 10197448
(43) Date of publication of application: 06.11.2013
(73) Proprietor: Hummingbird Diagnostics GmbH, 69120 Heidelberg (DE)
(72) Inventor: LEIDINGER, Petra, 66687 Wadern-Nunkirchen (DE); KELLER, Andreas, 66346 Püttlingen (DE); BOISGUERIN, Valesca, 55118 Mainz (DE); BEIER, Markus, 69469 Weinheim (DE); MEESE, Eckhart, 66882 Hütschenhausen (DE)
(74) Representative: Geling, Andrea
(86) International application number: PCT/EP2011/074169
(87) International publication number: WO 2012/089772

(56) References cited:
- WO-A2-2010/135692
- US-B1- 6 171 796
- ERDMANN K ET AL: "1300 DOWN-REGULATION OF SELECTED MICRORNAS CORRELATES WITH EXPRESSION OF PROSTATE CANCER ASSOCIATED GENES", JOURNAL OF UROLOGY, LIPPINCOTT WILLIAMS & WILKINS, BALTIMORE, MD, US, vol. 183, no. 4, 1 April 2010 (2010-04-01) , page E503, XP027008457, ISSN: 0022-5347 [retrieved on 2010-04-01]
- M OZEN ET AL: "Widespread deregulation of microRNA expression in human prostate cancer", ONCOGENE, vol. 27, no. 12, 13 March 2008 (2008-03-13), pages 1788-1793, XP055003528, ISSN: 0950-9232, DOI: 10.1038/sj.onc.1210809
- HANNA E. RAUHALA ET AL: "miR-193b is an epigenetically regulated putative tumor suppressor in prostate cancer", INTERNATIONAL JOURNAL OF CANCER, vol. 127, no. 6, 1 September 2010 (2010-09-01), pages 1363-1372, XP055003500, ISSN: 0020-7136, DOI: 10.1002/ijc.25162
- LI MING ET AL: "Expression of 6 microRNAs in prostate cancer and its significance", CHINESE JOURNAL OF CLINICAL ONCOLOGY,, vol. 6, no. 1, 1 February 2009 (2009-02-01), pages 21-28, XP001539911, ISSN: 1672-7118, DOI: DOI:10.1007/S11805-009-0021-2
- PORKKA KATI P ET AL: "MicroRNA expression profiling in prostate cancer", CANCER RESEARCH, vol. 67, no. 13, July 2007 (2007-07), pages 6130-6135, XP002653363, ISSN: 0008-5472
- MARTIN SPAHN ET AL: "Expression of microRNA-221 is progressively reduced in aggressive prostate cancer and metastasis and predicts clinical recurrence", INTERNATIONAL JOURNAL OF CANCER, vol. 127, no. 2, 1 July 2010 (2010-07-01), pages NA-NA, XP055003499, ISSN: 0020-7136, DOI: 10.1002/ijc.24715

## Description

### TECHNICAL FIELD OF THE INVENTION

The present invention relates to a method for diagnosing of benign prostatic hyperplasia (BPH) based on the determination of differential expression profiles of sets of miRNAs comprising hsa-miR-675 and hsa-miR-221 in blood cell samples from BPH patients when compared to prostate cancer patients. Furthermore, the present invention relates to the use of a set of polynucleotides for detecting a set of at least two miRNAs comprising hsa-miR-675 and hsa-miR-221 in said method for diagnosing of BPH in a blood cell sample. In addition, the present invention relates to the use of a kit for diagnosing of BPH comprising means for determining expression profiles of sets of at least two miRNAs comprising hsa-miR-675 and hsa-miR-221 in said method for diagnosing BPH in a blood cell sample and at least one reference. Further, the present invention relates to use of a set of at least two miRNAs comprising hsa-miR-675 and hsa-miR-221 in said method for diagnosing of BPH in a blood cell sample from a subject.

### BACKGROUND OF THE INVENTION

Today, biomarkers play a key role in early diagnosis, risk stratification, and therapeutic management of various diseases. While progess in biomarker research has accelerated over the last 5 years, the clinical translation of disease biomarkers as endpoints in disease management and as the foundation for diagnostic products still poses a challenge.

MicroRNAs (miRNAs) are a new class of biomarkers. They represent a group of small noncoding RNAs that regulate gene expression at the posttranslational level by degrading or blocking translation of messenger RNA (mRNA) targets. MiRNAs are important players when it comes to regulate cellular functions and in several diseases, including cancer.

So far, miRNAs have been extensively studied in tissue material. It has been found that miRNAs are expressed in a highly tissue-specific manner. Disease-specific expression of miRNAs have been reported in many human cancers employing primarily tissue material as the miRNA source. In this context miRNAs expression profiles were found to be useful in identifying the tissue of origin for cancers of unknown primary origin.

Since recently it is known that miRNAs are not only present in tissues but also in other body fluid samples, including human blood. Nevertheless, the mechanism why miRNAs are found in body fluids, especially in blood, or their function in these body fluids is not understood yet.

Various miRNA biomarkers found in tissue material have been proposed to be correlated with certain diseases, e.g. cancer. However, there is still a need for novel miRNAs as biomarkers for the detection and/or prediction of these and other types of diseases. Especially desirable are non-invasive biomarkers, that allow for quick, easy and cost-effective diagnosis/prognosis which cause only minimal stress for the patient eliminating the need for surgical intervention

Particularly, the potential role of miRNAs as non-invasive biomarkers for the diagnosis and/or prognosis of BPH has not been systematically evaluated yet. In addition, many of the miRNA biomarkers presently available for diagnosing of diseases have shortcomings such as reduced sensitivity, not sufficient specificity or do not allow timely diagnosis or represent invasive biomarkers. Accordingly, there is still a need for novel and efficient miRNAs or sets of miRNAs as markers, effective methods and kits for the non-invasive diagnosis and/or prognosis of diseases such as BPH.

WO2010/135692 discloses miRNAs that may be used as serum or plasma biomarkers for characterizing prostate diseases in a patient. In particular for differentiating BPH patients from prostate cancer patients WO2010/135692 discloses miRNA biomarkers that are derived from analyzing serum samples.

The inventors of the present invention assessed for the first time the expression of miRNAs on a whole-genome level in subjects with BPH as non-invasive biomarkers from body fluids, preferably in blood. They surprisingly found that miRNAs are significantly dysregulated in blood of BPH subjects in comparison to healthy controls or in comparison to prostate cancer subjects. Thus, miRNAs are appropriated non-invasive biomarkers for diagnosing of BPH. This finding is surprising, since there is nearly no overlap of the miRNA biomarkers found in blood and the miRNA biomarkers found in tissue material representing the origin of the disease. The inventors of the present invention surprisingly found miRNA biomarkers in body fluids, especially in blood, that have not been found to be correlated to BPH when tissues material was used for this kind of analysis. Therefore, the inventors of the invention identified for the first time miRNAs as non-invasive surrogate biomarkers for diagnosis and/or prognosis of BPH. The inventors of the present invention identified single miRNAs which predict BPH with high specificity, sensitivity and accuracy. The inventors of the present invention also pursued a multiple biomarker strategy, thus implementing sets of miRNA biomarkers for diagnosing of BPH leading to added specificity, sensitivity, accuracy and predictive power, thereby circumventing the limitations of single biomarker. In detail, by using a machine learning algorithms, they identified unique sets of miRNAs (miRNA signatures) that allow for non-invasive diagnosis of BPH with even higher power, indicating that sets of miRNAs (miRNA signatures) derived from a body fluid sample, such as blood from a subject (e.g. human) can be used as novel non-invasive biomarkers.

### SUMMARY OF THE INVENTION

In a first aspect, the invention provides a method for diagnosing of BPH comprising the steps of:
(i) determining an expression profile of a set of at least two miRNAs in a blood cell sample from a patient, and
(ii) comparing said expression profile to a reference,
wherein the set of miRNAs comprises hsa-miR-675 (SEQ ID NO:9) and hsa-miR-221 (SEQ ID NO:32) that are differentially regulated in blood cell samples from BPH patients as compared to prostate cancer patients and wherein the comparison of said expression profile to said reference allows to discriminate between BPH and prostate cancer.

In a second aspect, the invention provides the use of a set of polynucleotides for detecting a set of at least two miRNAs, comprising hsa-miR-675 (SEQ ID NO:9) and hsa-miR-221 (SEQ ID NO:32), in a method for diagnosing of BPH in a blood cell sample according to the first aspect of the invention.

In a third aspect, the invention provides the use of a kit for diagnosing of BPH in a method according to the first aspect of the invention comprising
(a) means for determining the miRNA expression profile of a set of at least two miRNAs that comprise hsa-miR-675 (SEQ ID NO:9) and hsa-miR-221 (SEQ ID NO:32) comprising :
   i. a set of at least two polynucleotides for detecting a set of at least two miRNAs comprising hsa-miR-675 (SEQ ID NO:9) and hsa-miR-221 (SEQ ID NO:32) or
   ii. a set of at least two primer pairs for detecting a set of at least two miRNAs comprising hsa-miR-675 (SEQ ID NO:9) and hsa-miR-221 (SEQ ID NO:32)
      and
(b) at least one reference.

In a fourth aspect, the invention provides a use of a set of at least two miRNAs comprising hsa-miR-675 (SEQ ID NO:9) and hsa-miR-221 (SEQ ID NO:32), isolated from a blood cell sample in a method for diagnosing BPH according to the first aspect of the invention.

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used. The present invention encompasses (target) miRNAs which are dysregulated in biological samples such as blood or tissue of BPH patients in comparison to healthy controls. Said (target) miRNAs are preferably selected from the group consisting of SEQ ID NO: 1 to 182 and 352-353.

The term "miRBase" refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

As used herein, the term "nucleotides" refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar.

Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.

In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. The present invention encompasses polynucleotides in form of single polynucleotide strands as probes for binding to, hybridizing with or detecting complementary sequences of (target) miRNAs for diagnosing of BPH. Said (target) miRNAs are preferably selected from the group consisting of SEQ ID NO: 1 to 182 and 352-353.

Because of the conservation of miRNAs among species, for example between humans and other mammals, e.g. animals such as mice, monkey or rat, the polynucleotide(s) of the invention may not only be suitable for detecting a miRNA(s) of a specific species, e.g. a human miRNA, but may also be suitable for detecting the respective miRNA orthologue(s) in another species, e.g. in another mammal, e.g. animal such as mouse or rat.

The term "antisense", as used herein, refers to nucleotide sequences which are complementary to a specific DNA or RNA sequence. The term "antisense strand" is used in reference to a nucleic acid strand that is complementary to the "sense" strand.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which can be made detectable. A label may be incorporated into nucleic acids at any position, e.g. at the 3' or 5' end or internally. The polynucleotide for detecting a miRNA (polynucleotide probe) and/or the miRNA itself may be labeled. For detection purposes, the miRNA(s) or miRNA*(s) may be employed unlabeled, directly labeled, or indirectly labeled, such as with biotin to which a streptavidin complex may later bind.

The term "stringent hybridization conditions", as used herein, means conditions under which a first nucleotide sequence (e.g. polynucleotide in its function as a probe for detecting a miRNA or miRNA*) will hybridize to a second nucleotide sequence (e.g. target sequence such as nucleotide sequence of a miRNA or miRNA*), such as in a complex mixture of nucleotide sequences. Stringent conditions are sequence-dependent and will be different in different circumstances. Stringent conditions may be selected to be about 5 to 10°C lower than the thermal melting point (Tm) for the specific sequence at a defined ionic strength, pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to 1.0 M sodium ion concentration (or other salts) at pH 7.0 to 8.3 and the temperature is at least about 20°C for short probes (e.g. about 10-35 nucleotides) and up to 60°C for long probes (e.g. greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %, Tween 20, 0.1 x TE buffer, 0.5 mg/ml BSA, 0.1 mg/ml herring sperm DNA, incubating at 42°C with wash in 05x SSPE and 6x SSPE at 45°C.

The term "sensitivity", as used herein, means a statistical measure of how well a binary classification test correctly identifies a condition, for example how frequently it correctly classifies a heart and cardiovascular system disease into the correct type out of two or more possible types (e.g. heart and cardiovascular system disease type and healthy type). The sensitivity for class A is the proportion of cases that are determined to belong to class "A" by the test out of the cases that are in class "A". A theoretical, optimal prediction can achieve 100% sensitivity (i.e. predict all patients from the sick group as sick).

The term "specificity", as used herein, means a statistical measure of how well a binary classification test correctly identifies a condition, for example how frequently it correctly classifies a heart and cardiovascular system disease into the correct type out of two or more possible types. The specificity for class A is the proportion of cases that are determined to belong to class "not A" by the test out of the cases that are in class "not A". A theoretical, optimal prediction can achieve 100% specificity (i.e. not predict anyone from the healthy group as sick).

The term "accuracy", as used herein, means a statistical measure for the correctness of classification or identification of sample types. The accuracy is the proportion of true results (both true positives and true negatives).

The term "biological sample", as used in the context of the present invention, refers to any biological sample containing miRNA(s). Said biological sample may be a biological fluid, tissue, cell(s) or mixtures thereof. For example, biological samples encompassed by the present invention are body fluids, tissue (e.g. section or explant) samples, cell culture samples, cell colony samples, single cell samples, collection of single cell samples, blood samples (e.g. whole blood or a blood fraction such as serum or plasma or blood cell fractions such as red blood cells, platelets, white blood cells, T-cells, NK-cells, regulatory T-cells, B-cells, granulocytes etc.), urine samples, or samples from other peripheral sources. Said biological samples may be mixed or pooled, e.g. a biological sample may be a mixture of blood and urine samples. A "biological sample" may be provided by removing cell(s), cell colonies, an explant, or a section from a subject suspected to be affected by BPH, but may also be provided by using a previously isolated sample. For example, a tissue sample may be removed from a subject suspected to be affected by BPHs by conventional biopsy techniques or a blood sample may be taken from a subject suspected to be affected by BPH by conventional blood collection techniques. The biological sample, e.g. tissue or blood sample, may be obtained from a subject suspected to be affected by BPH prior to initiation of the therapeutic treatment, during the therapeutic treatment and/or after the therapeutic treatment.

The term "body fluid sample", as used in the context of the present invention, refers to liquids originating from the body of a subject. Said body fluid samples include, but are not limited to, blood, urine, sputum, breast milk, cerebrospinal fluid, cerumen (earwax), endolymph, perilymph, gastric juice, mucus, peritoneal fluid, pleural fluid, saliva, sebum (skin oil), semen, sweat, tears, vaginal secretion, vomit including components or fractions thereof. Said body fluid samples may be mixed or pooled, e.g. a body fluid sample may be a mixture of blood and urine samples or blood and tissue material. A "body fluid sample" may be provided by removing a body liquid from a subject, but may also be provided by using previously isolated sample material. Preferably, the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

In the context of the present invention said "body fluid sample" allows for a non-invasive diagnosis/and or prognosis of a subject.

The term "blood sample", as used in the context of the present invention, refers to a blood sample originating from a subject. The "blood sample" may be derived by removing blood from a subject by conventional blood collecting techniques, but may also be provided by using previously isolated and/or stored blood samples. For example a blood sample may be whole blood, plasma, serum, PBMC (peripheral blood mononuclear cells), blood cellular fractions including red blood cells (erythrocytes), white blood cells (leukocytes) or subtractions thereof ( e.g. T-cells, NK-cells, regulatory T-cells, B-cells, granulocytes), platelets (thrombocytes), or blood collected in blood collection tubes (e.g. EDTA-, heparin-, citrate-, PAXgene-, Tempus-tubes) including components or fractions thereof. For example, a blood sample may be taken from a subject suspected to be affected or to be suspected to be affected by BPH, prior to initiation of a therapeutic treatment, during the therapeutic treatment and/or after the therapeutic treatment.

Preferably, the blood sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

In the context of the present invention said "body fluid sample" allows for a non-invasive diagnosis/and or prognosis of a subject.

Preferably, when the blood sample is collected from the subject the RNA-fraction, especially the the miRNA fraction, is guarded against degradation. For this purpose special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction and/or the miRNA fraction are employed.

The biological sample, preferably the body fluid sample may be from a subject (e.g. human or mammal) that has been therapeutically treated or that has not been therapeutically treated. In one embodiment, the therapeutical treatment is monitored on the basis of the detection of the miRNA or set of miRNAs by the polynucleotide or set of polynucleotides of the invention. It is also preferred that total RNA or a subfraction thereof, isolated (e.g. extracted) from a biological sample of a subject (e.g. human or animal), is used for detecting the miRNA or set of miRNAs by the polynucleotide or set of polynucleotides or primer pairs of the invention.

The term "non-invasive", as used in the context of the present invention, refers to methods for obtaining a biological sample, particularly a body fluid sample, without the need for an invasive surgical intervention or invasive medical procedure. In the context of the present invention, a blood drawn represents a non-invasive procedure, therefore a blood-based test (utilizing blood or fractions thereof) is a non-invasive test. Other body fluid samples for non-invasive tests are e.g. urine, sputum, tears, mothers mild, cerumen, sweat, saliva, vaginal secretion, vomit, etc..

The term "minimal invasive", as used in the context of the present invention, refers to methods for obtaining a biological sample, particularly a body fluid sample, with a minimal need for an invasive surgical intervention or invasive medical procedure.

The term "biomarker", as used in the context of the present invention, represents a characteristic that can be objectively measured and evaluated as an indicator of normal and disease processes or pharmacological responses. A biomarker is a parameter that can be used to measure the onset or the progress of disease or the effects of treatment. The parameter can be chemical, physical or biological.

The term "surrogate biomarker", as used in the context of the present invention, represents biomarker intended to substitute for a clinical endpoint. It is a measure of a clinical condition or a measure of effect of a certain treatment that may correlate with the real clinical condition (e.g. healthy, diseased) but doesn't necessarily have a guaranteed relationship. An ideal surrogate biomarker is a laboratory substitute for a clinically meaningful result, and should lie directly in the causal pathway linking disease to outcome. Surrogate biomarkers are used when the primary endpoint is undesired (e.g. death). A commonly used example is cholesterol : while elevated cholesterol levels increase the likelihood for heart disease, the relationship is not linear - many people with normal cholesterol develop heart disease, and many with high cholesterol do not. "Death from heart disease" is the endpoint of interest, but "cholesterol" is the surrogate biomarker.

The term "diagnosis" as used in the context of the present invention refers to the process of determining a possible disease or disorder and therefore is a process attempting to define the (clinical) condition of a subject. The determination of the expression level of a set of miRNAs according to the present invention correlates with the (clinical) condition of a subject. Preferably, the diagnosis comprises (i) determining the occurrence/presence of BPH, (ii) monitoring the course of BPH, (iii) staging of BPH, (iv) measuring the response of a patient with BPH to therapeutic intervention, and/or (v) segmentation of a subject suffering from BPH.

The term "prognosis" as used in the context of the present invention refers to describing the likelihood of the outcome or course of a disease or a disorder.

Preferably, the prognosis comprises (i) identifying of a subject who has a risk to develop BPH, (ii) predicting/estimating the occurrence, preferably the severity of occurrence of BPH, and/or(iii) predicting the response of a subject with BPH to therapeutic intervention.

The term "(clinical) condition" (biological state or health state), as used herein, means a status of a subject that can be described by physical, mental or social criteria. It includes so-called "healthy" and "diseased" conditions. For the definition of "healthy" and "diseased" conditions it is referred to the international classification of diseases (ICD) of the WHO (http://www.int/classifications/icd/en/index.html). When one condition is compared according to a preferred embodiment of the method of the present invention, it is understood that said condition is BPH or a specific form of BPH. When two or more conditions are compared according to another preferred embodiment of the method of the present invention, it is understood that this is possible for all conditions that can be defined and is not limited to a comparison of a diseased versus healthy comparison and extends to multiway comparison, under the proviso that at least one condition is BPHs, preferably a specific form of BPH.

The term "miRNA expression profile" as used in the context of the present invention, represents the determination of the miRNA expression level or a measure that correlates with the miRNA expression level in a biological sample. The miRNA expression profile may be generated by any convenient means, e.g. nucleic acid hybridization (e.g. to a microarray), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX) and the like, that allow the analysis of differential miRNA expression levels between samples of a subject (e.g. diseased) and a control subject (e.g. healthy, reference sample). The sample material measure by the aforementioned means may be total RNA, labeled total RNA, amplified total RNA, cDNA, labeled cDNA, amplified cDNA, miRNA, labeled miRNA, amplified miRNA or any derivatives that may be generated from the aforementioned RNA/DNA species. By determining the miRNA expression profile, each miRNA is represented by a numerical value. The higher the value of an individual miRNA, the higher is the expression level of said miRNA, or the lower the value of an individual miRNA, the lower is the expression level of said miRNA.

The "miRNA expression profile", as used herein, represents the expression level/expression data of a single miRNA or a collection of expression levels of at least two miRNAs, preferably of least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more, or up to all known miRNAs.

The term "differential expression" of miRNAs as used herein, means qualitative and/or quantitative differences in the temporal and/or local miRNA expression patterns, e.g. within and/or among biological samples, body fluid samples, cells, or within blood. Thus, a differentially expressed miRNA may qualitatively have its expression altered, including an activation or inactivation in, for example, blood from a diseases subject versus blood from a healthy subject. The difference in miRNA expression may also be quantitative, e.g. in that expression is modulated, i.e. either up-regulated, resulting in an increased amount of miRNA, or down-regulated, resulting in a decreased amount of miRNA. The degree to which miRNA expression differs need only be large enough to be quantified via standard expression characterization techniques, e.g. by quantitative hybridization (e.g. to a microarray), amplification (PCR, RT-PCR, qRT-PCR, high-throughput RT-PCR), ELISA for quantitation, next generation sequencing (e.g. ABI SOLID, Illumina Genome Analyzer, Roche 454 GS FL), flow cytometry (e.g. LUMINEX) and the like.

Nucleic acid hybridization may be performed using a microarray/biochip or *in situ* hybridization. *In situ* hybridization is preferred for the analysis of a single miRNA or a set comprising a low number of miRNAs (e.g. a set of at least 2 to 50 miRNAs such as a set of 2, 5, 10, 20, 30, or 40 miRNAs). The microarray/biochip, however, allows the analysis of a single miRNA as well as a complex set of miRNAs (e.g. a all known miRNAs or subsets therof).

For nucleic acid hybridization, for example, the polynucleotides (probes) according to the present invention with complementarity to the corresponding miRNAs to be detected are attached to a solid phase to generate a microarray/biochip (e.g. 184 polynucleotides (probes) which are complementary to the 184 miRNAs having SEQ ID NO: 1 to 182 and 352-353. Said microarray/biochip is then incubated with a biological sample containing miRNAs, isolated (e.g. extracted) from the body fluid sample such as blood sample from a subject such as a human or an animal, which may be labelled, e.g. fluorescently labelled, or unlabelled. Quantification of the expression level of the miRNAs may then be carried out e.g. by direct read out of a label or by additional manipulations, e.g. by use of a polymerase reaction (e.g. template directed primer extension, MPEA-Assay, RAKE-assay) or a ligation reaction to incorporate or add labels to the captured miRNAs.

Alternatively, the polynucleotides which are at least partially complementary (e.g.a set of chimeric polynucleotides with each a first stretch being complementary to a set of miRNA sequences and a second stretch complementary to capture probes bound to a solid surface (e.g. beads, Luminex beads)) to miRNAs having SEQ ID NO: 1 to 182 and 352-353. are contacted with the biological sample containing miRNAs (e.g a body fluid sample, preferably a blood sample) in solution to hybridize. Afterwards, the hybridized duplexes are pulled down to the surface (e.g a plurality of beads) and successfully captured miRNAs are quantitatively determined (e.g. FlexmiR-assay, FlexmiR v2 detection assays from Luminex).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 2 to 50 miRNAs such as a set of 2, 5, 10, 20, 30, or 40 miRNAs), whereas high-throughput RT-PCR technologies (e.g. OpenArray from Applied Biosystems, SmartPCR from Wafergen, Biomark System from Fluidigm) are also able to measure large sets (e.g a set of 10, 20, 30, 50, 80, 100, 200 or more) to all known miRNAs in a high parallel fashion. RT-PCR is particularly suitable for detecting low abandoned miRNAs.

The aforesaid real time polymerase chain reaction (RT-PCR) may include the following steps:
(i) extracting the total RNA from a biological sample or body fluid sample such as a blood sample (e.g. whole blood, serum, or plasma) of a subjects such as human or animal, and obtaining cDNA samples by RNA reverse transcription (RT) reaction using universal or miRNA-specific primers; or collecting a body fluid sample such as urine or blood sample (e.g. whole blood, serum, or plasma) of a patient such as human or animal, and conducting reverse transcriptase reaction using universal or miRNA-specific primers (e.g. looped RT-primers) within the body fluid sample such as urine or blood sample (e.g. whole blood, serum, or plasma) being a buffer so as to prepare directly cDNA samples, (ii) designing miRNA-specific cDNA forward primers and providing universal reverse primers to amplify the cDNA via polymerase chain reaction (PCR), (iii) adding a fluorescent dye (e.g. SYBR Green) or a fluorescent probe (e.g. Taqman probe) probe to conduct PCR, and (iv) detecting the miRNA(s) level in the body fluid sample such as urine or blood sample (e.g. whole blood, serum, or plasma).

A variety of kits and protocols to determine an expression profile by real time polymerase chain reaction (RT-PCR) such as real time quantitative polymerase chain reaction (RT qPCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI7300. Alternative kits are available from Ambion, Roche, Qiagen, Invitrogen, SABiosciences, Exiqon etc.

The term "subject", as used in the context of the present invention, means a patient or individual or mammal suspected to be affected by BPH. The patient may be diagnosed to be affected by BPH, i.e. diseased, or may be diagnosed to be not affected by BPH, i.e. healthy. The subject may also be diagnosed to be affected by a specific form of BPH. The subject may further be diagnosed to develop BPH or a specific form of BPH as the inventors of the present invention surprisingly found that miRNAs representative for BPH are already present in the biological sample, e.g. blood sample, before BPH occurs or during the early stage of BPH. It should be noted that a subject that is diagnosed as being healthy, i.e. not suffering from BPH or from a specific form of BPH, may possibly suffer from another disease not tested/known. The subject may be any mammal, including both a human and another mammal, e.g. an animal such as a rabbit, mouse, rat, or monkey. Human subjects are particularly preferred. Therefore, the miRNA from a subject may be a human miRNA or a miRNA from another mammal, e.g. an animal miRNA such as a mouse, monkey or rat miRNA, or the miRNAs comprised in a set may be human miRNAs or miRNAs from another mammal, e.g. animal miRNAs such as mouse, monkey or rat miRNAs.

The term "control subject", as used in the context of the present invention, may refer to a subject known to be affected with BPH (positive control), i.e. diseased, or to a subject known to be not affected with BPH (negative control), i.e. healthy. It may also refer to a subject known to be effected by another disease/condition (see definition "(clinical) condition"). It should be noted that a control subject that is known to be healthy, i.e. not suffering from BPH, may possibly suffer from another disease not tested/known. The control subject may be any mammal, including both a human and another mammal, e.g. an animal such as a rabbit, mouse, rat, or monkey. Human "control subjects" are particularly preferred.

The term "set comprising at least two miRNAs representative for BPH", as used herein, refers to refers to at least two fixed defined miRNAs comprised in a set which are known to be differential between subjects (e.g. humans or other mammals such as animals) suffering from BPH (diseased state) and control subjects (e.g. humans or other mammals such as animals and are, thus, representative for BPH. Said " set comprising at least two miRNAs representative for BPH" are preferably selected from the group consisting of SEQ ID NO: 1 to 182 and 352-353, a fragment thereof, and a sequence having at least 80% sequence identity thereto.

The term "BPH", as used herein refers to benign prostatic hyperplasia, which means a benign enlargement of the prostate. BPH can be a progressive disease, especially if left untreated. Though the prostate continues to grow during most of a man's life, the enlargement doesn't usually cause problems until late in life. BPH rarely causes symptoms before age 40, but more than half of men in their sixties and as many as 90 percent in their seventies and eighties have some symptoms of BPH. But more importantly, the symptoms of BPH and prostate cancer are often similar. Often, blood tests are performed to rule out prostatic malignancy. Nevertheless, the testing the prostate specific antigen (PSA) does not meet the performance needs. Therefore, there is a urgent need for diagnostic tests that allow to discriminate between BPH and prostate cancer with high accuracy, sensitivity and specificity.

The inventors of the present invention surprisingly found that miRNAs are significantly dysregulated in body fluid samples such as blood of BPH subjects in comparison to a cohort of healthy controls and incomparison to a cohort of prostate cancer patients and thus, miRNAs are appropriated biomarkers for diagnosing of BPH in a non-invasive fashion or minimal-invasive fashion. Furthermore, the sets of miRNAs of the present invention lead to high performance in diagnosing of BPH, thus expose very high specificity, sensitivity and accuracy, both in comparison to healthy controls and in comparison to prostate cancer patients. They succeeded in determining the miRNAs that are differentially regulated in body fluid samples from patients having BPH compared to a cohort of healthy controls and compared to a cohort of prostate cancer patients. Additionally, the inventors of the present invention performed hypothesis tests (e.g. t-test, limma-test) or other measurements (e.g. AUC, mutual information) on the expression level of the found miRNAs, in all controls (healthy subjects or prostate cancer subjects respectively) and subjects suffering from BPH. These tests resulted in a significance value (p-value) for each miRNA. This p-value is a measure for the diagnostic power of each of these single miRNAs to discriminate, for example, between the two clinical conditions: healthy controls (healthy subjects - not suffering from BPH), or diseased, ( suffering from BPH) or alternatively between prostate cancer patients (not suffering from BPH) or diseased (suffering from BPH) . Since a manifold of tests are carried out, one for each miRNA, the p-values may be too optimistic and, thus, over-estimate the actual discriminatory power. Hence, the p-values are corrected for multiple testing by the Benjamini Hochberg approach.

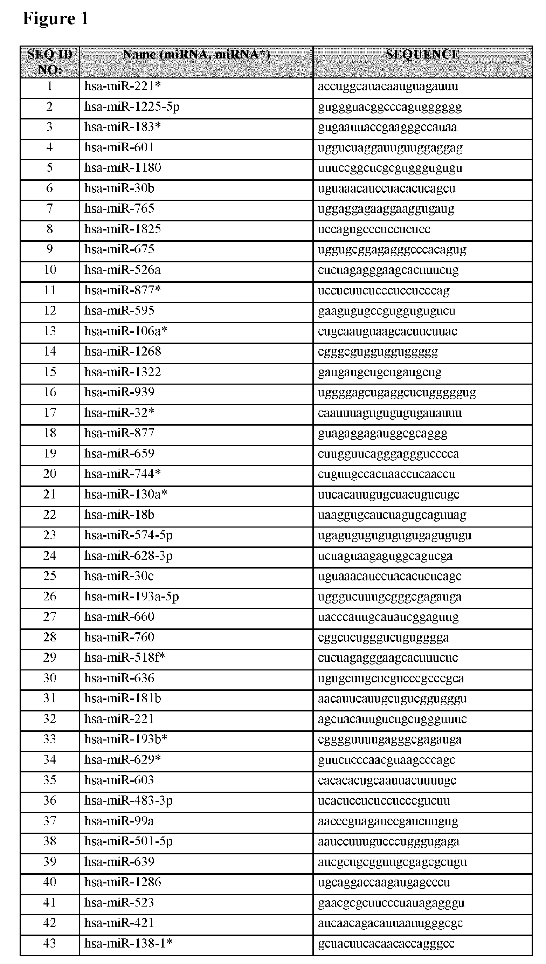

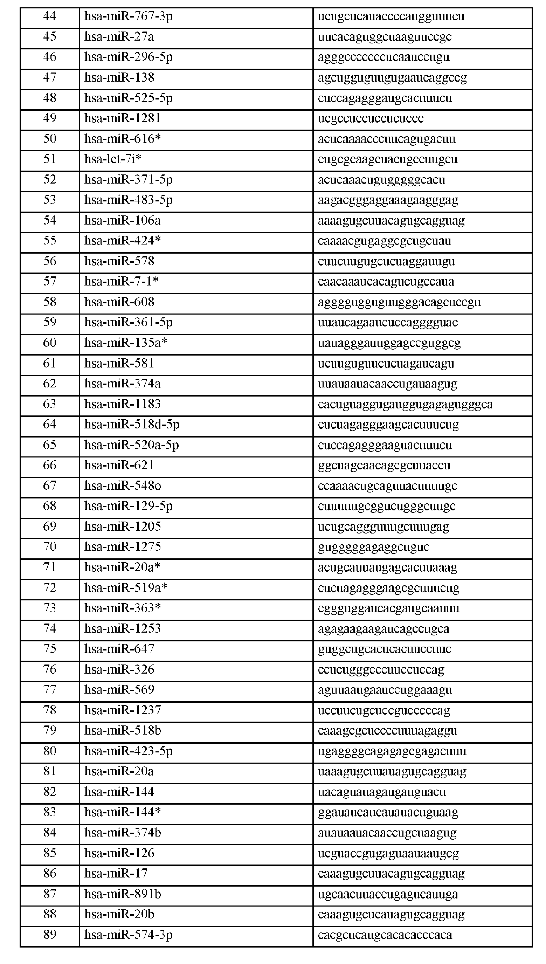

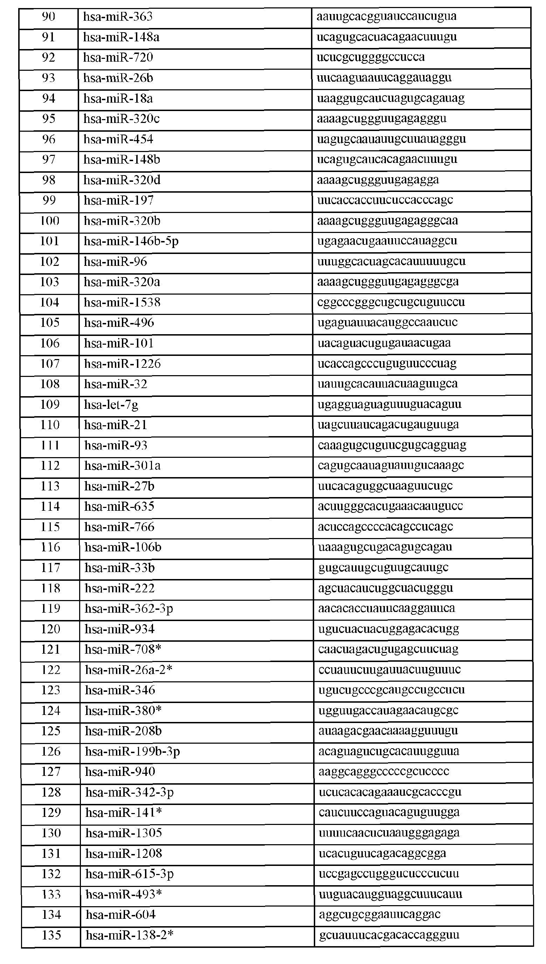

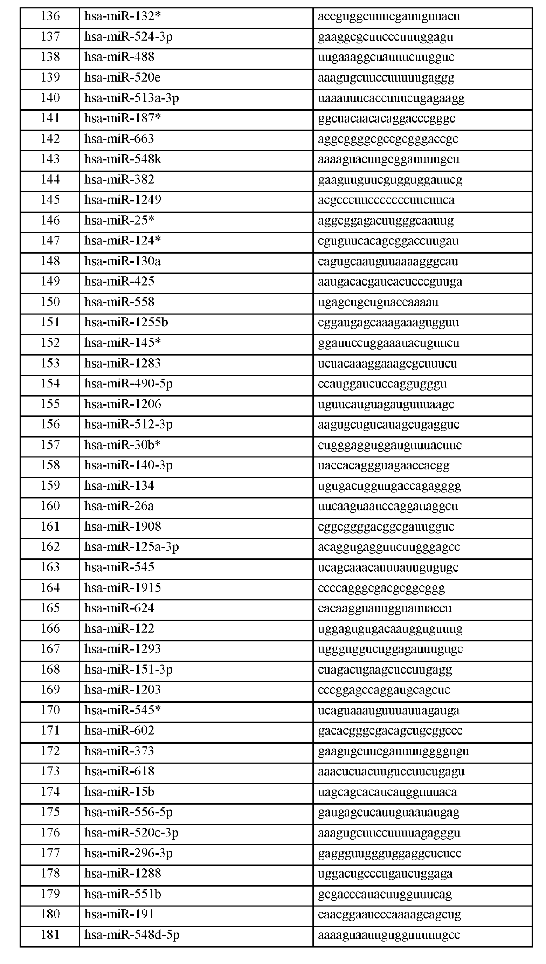

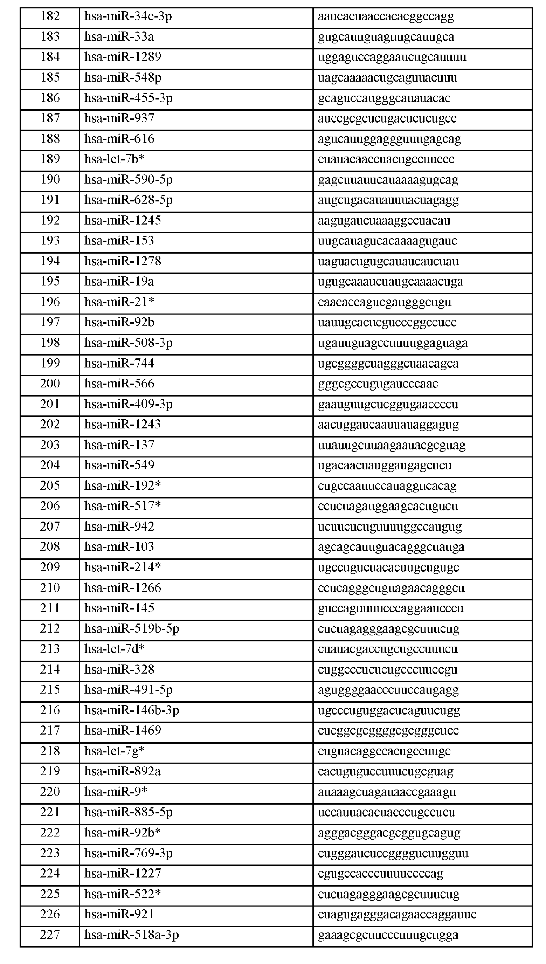

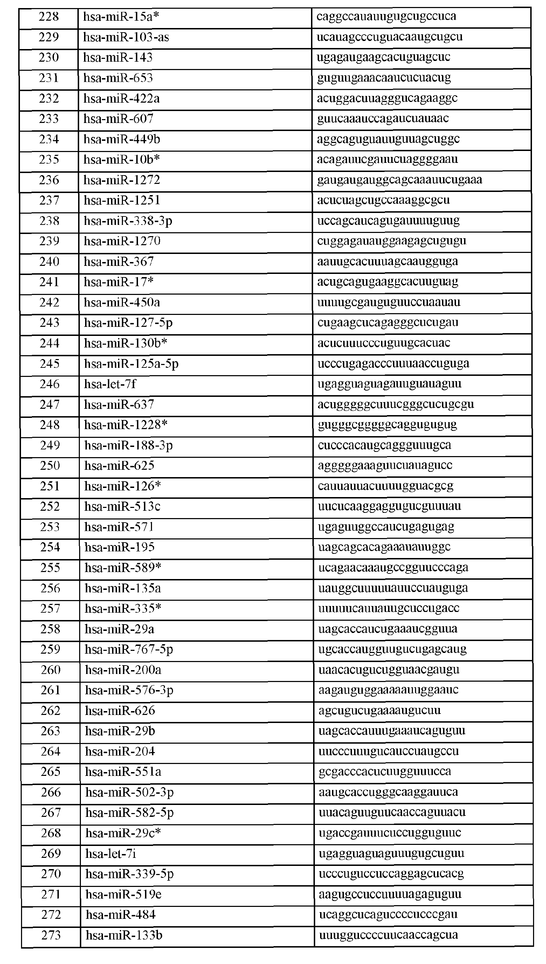

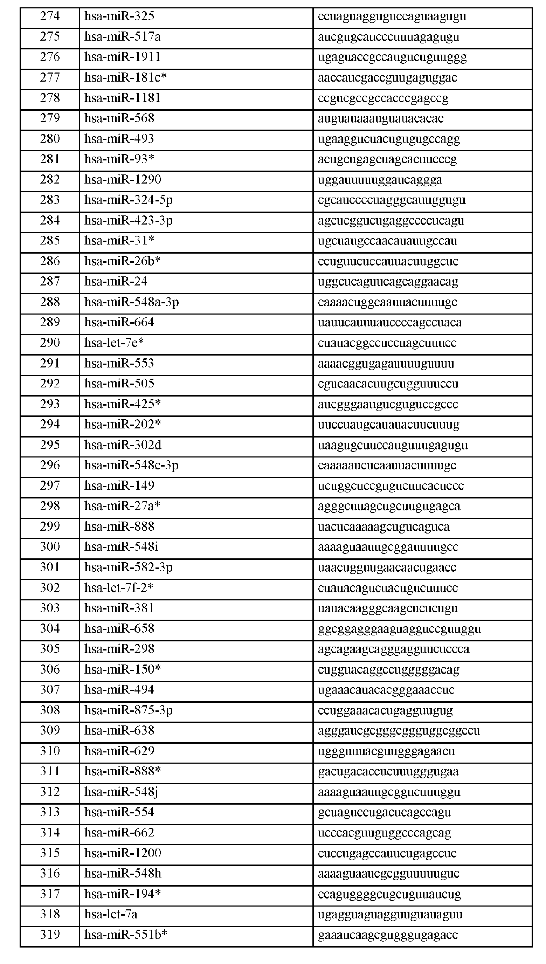

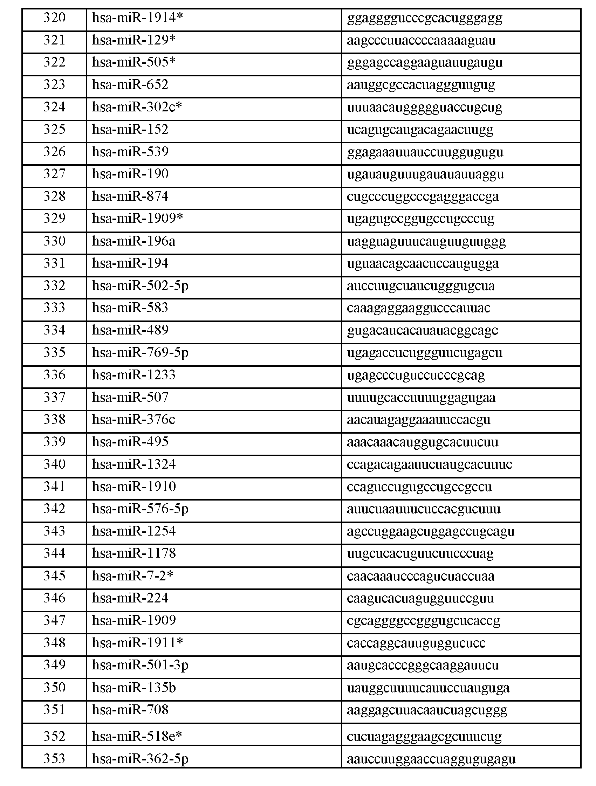

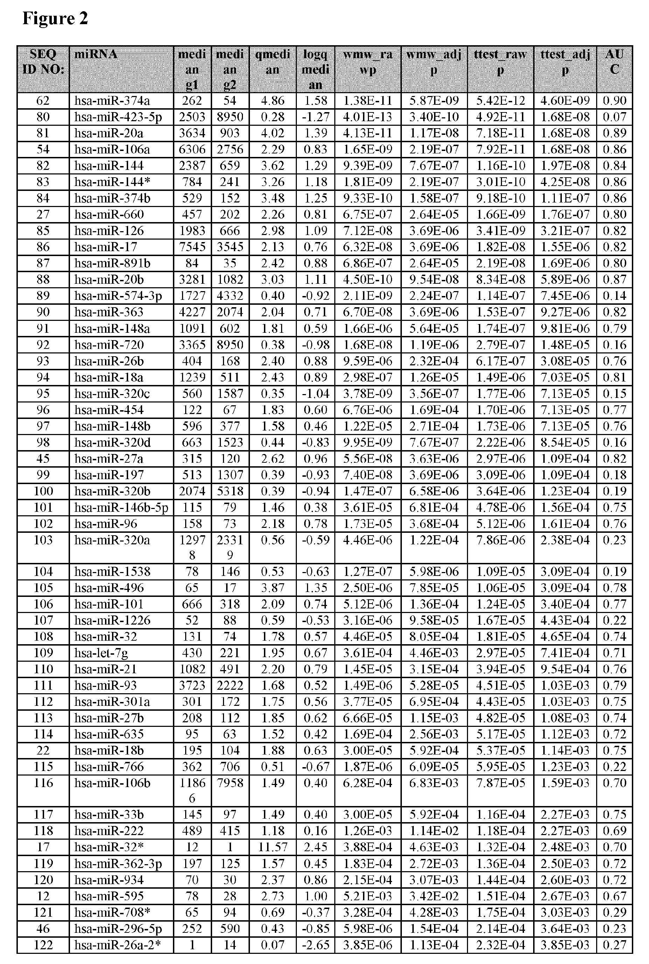

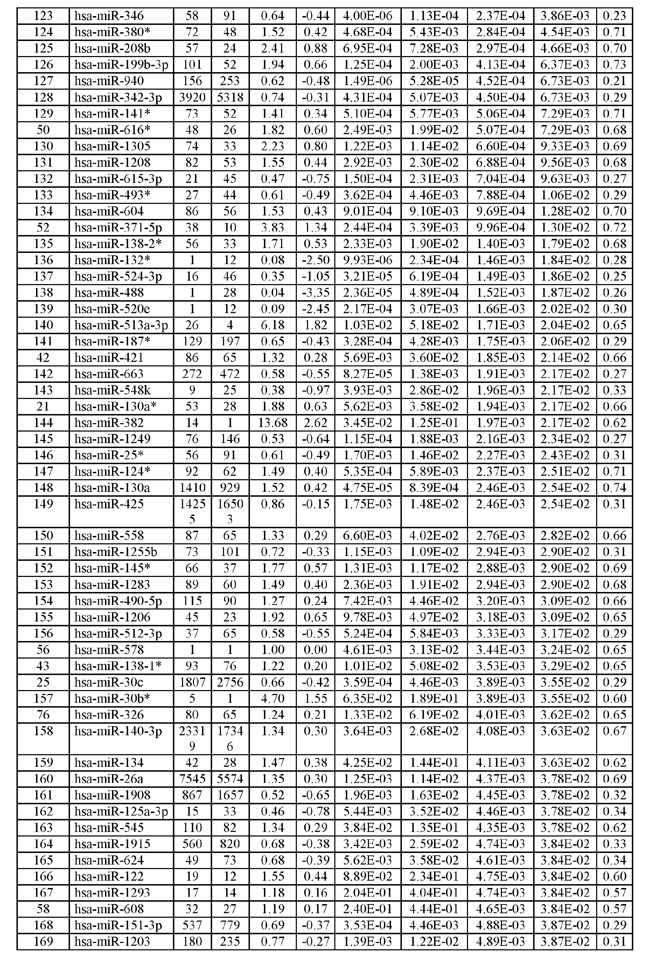

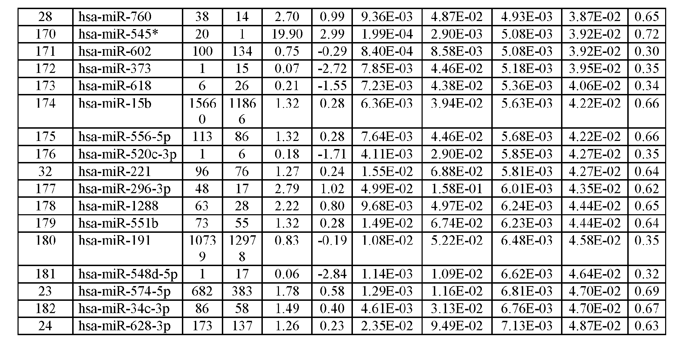

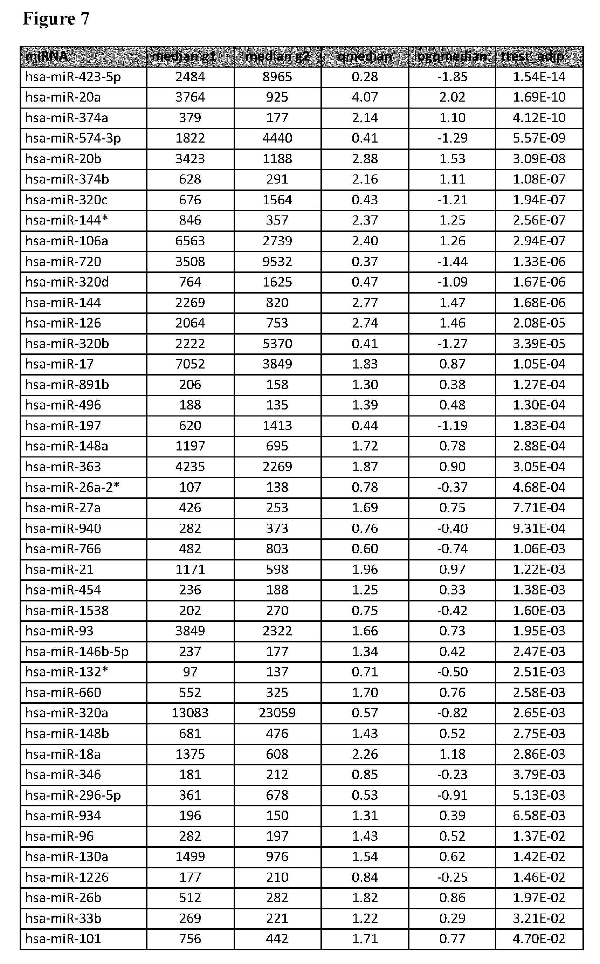

An overview of the miRNAs that are found to be significantly differentially regulated in biological samples of BPH versus healthy controls and that performed best according to t-test, limma-test or AUC is provided in Figure 2 or Figure 7 (healthy controls versus BPH subjects). (Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with BPH, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). The miRNAs are sorted in order of their t-test significance as described in more detail in the experimental section (see ttest_adjp= adjusted p-value calculated according to ttest). It should be noted that the lower the ttest_adjp value of a single miRNA, the higher is the diagnostic power of said miRNA for diagnosing of BPH.

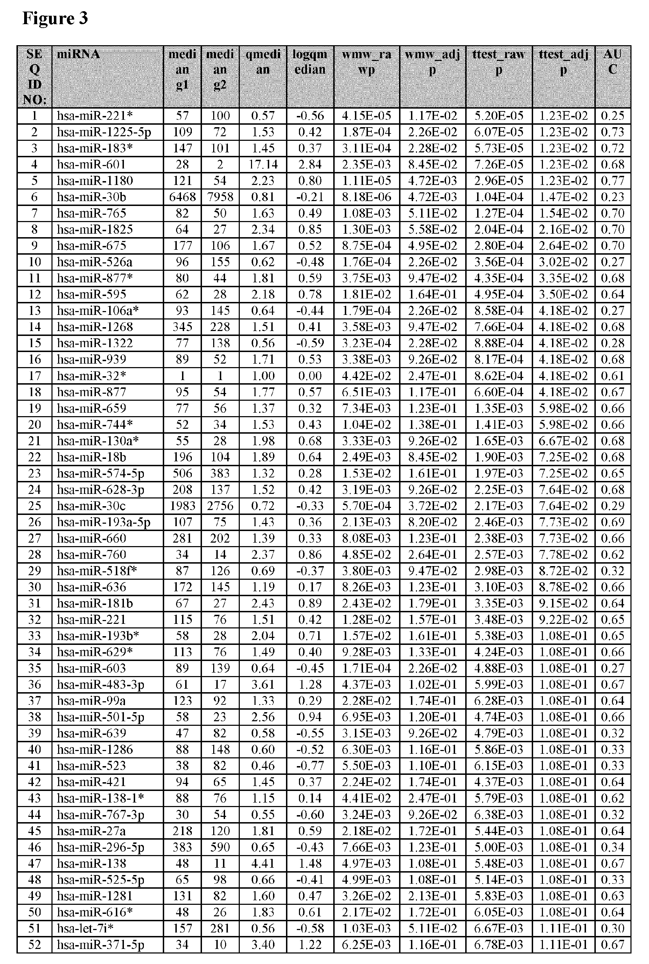

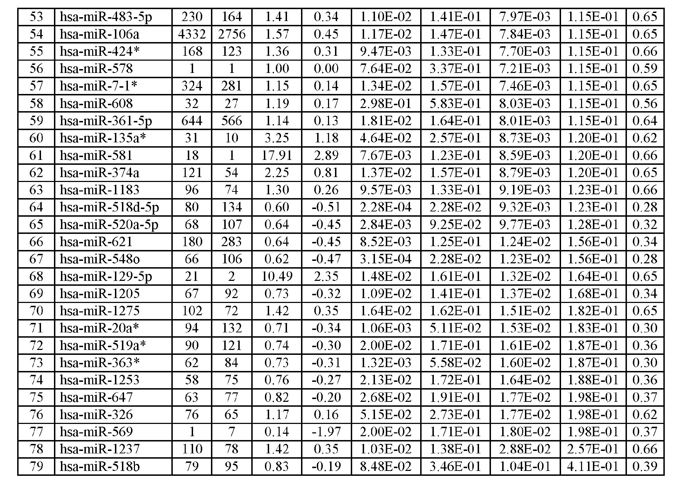

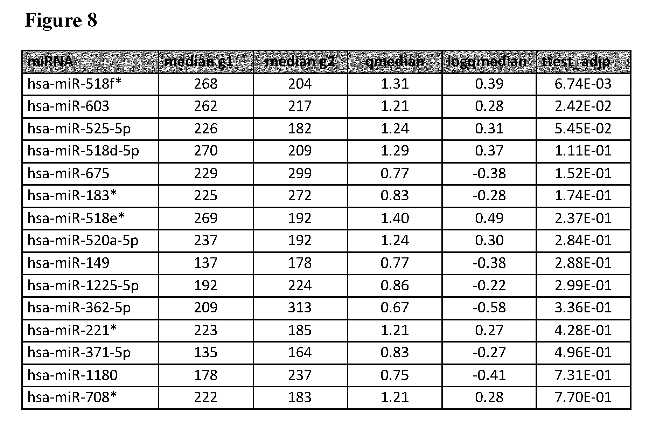

An overview of the miRNAs that are found to be significantly differentially regulated in biological samples of BPH versus prostate cancer patients and that performed best according to t-test, limma-test or AUC is provided Figure 3 (prostate cancer patients versus BPH subjects) (Experimental details: SEQ ID NO: sequence identification number, miRNA: identifier of the miRNA according to miRBase, median g1: median intensity obtained from microarray analysis for prostate cancer patients, median g2: median intensity obtained from microarray analysis for individuals with BPH, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). The miRNAs, i.e. miRNAs according to SEQ ID NO: 1 to 10, are sorted in order of their t-test significance as described in more detail in the experimental section (see ttest_adjp= adjusted p-value calculated according to ttest). It should be noted that the lower the ttest_adjp value of a single miRNA, the higher is the diagnostic power of said miRNA for diagnosing of BPH. Further miRNAs that are found to be significantly differentially regulated in biological samples of BPH versus prostate cancer patients are provided in Figure 8.

Usually the diagnostic power of a single miRNA biomarker is not sufficient to reach high accuracy, specificity and sensitivity for discrimination between healthy subjects or prostate cancer subjects (controls) and subjects suffering from BPH, hence no simple threshold method can be used for diagnosis and/or prognosis.
Therefore, the inventors of the present invention employed more than one miRNA biomarker, i.e. sets of miRNA biomarkers (signatures), to further increase and/or improve the performance for diagnosing of subjects suffering from BPH. This leads to a significant increase in sensitivity, specificity and accuracy when compared to the prior art.

In order to be able to discriminate, for example, between two or more clinical conditions, e.g. healthy and suffering from BPH, for a defined set of miRNA biomarkers, the inventors of the present invention applied a machine learning approach (e.g. t-test, AUC, support vector machine, hierarchical clustering, or k-means) which leads to an algorithm that is trained by reference data (i.e. data of reference miRNA expression profiles from the two clinical conditions, e.g. healthy and suffering from BPH, for the defined set of miRNA markers) to discriminate between the two statistical classes (i.e. two clinical conditions, e.g. healthy or suffering from BPH).

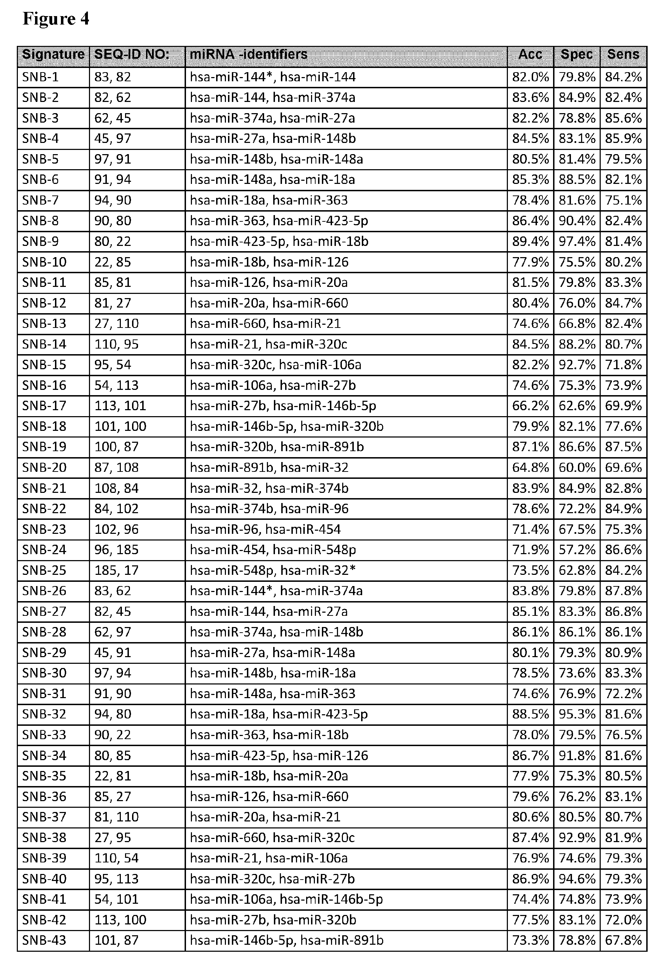

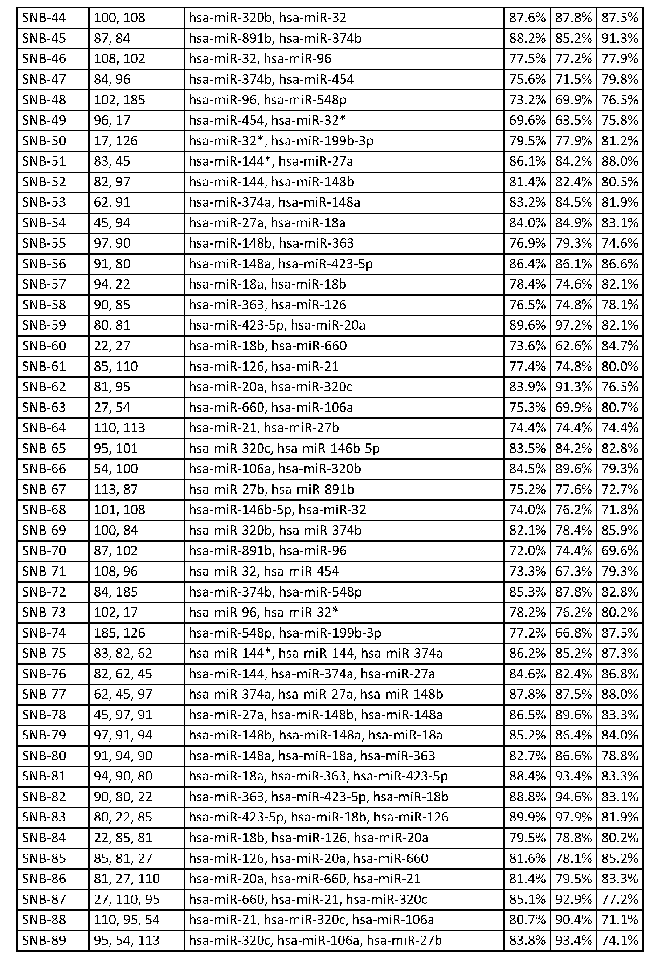

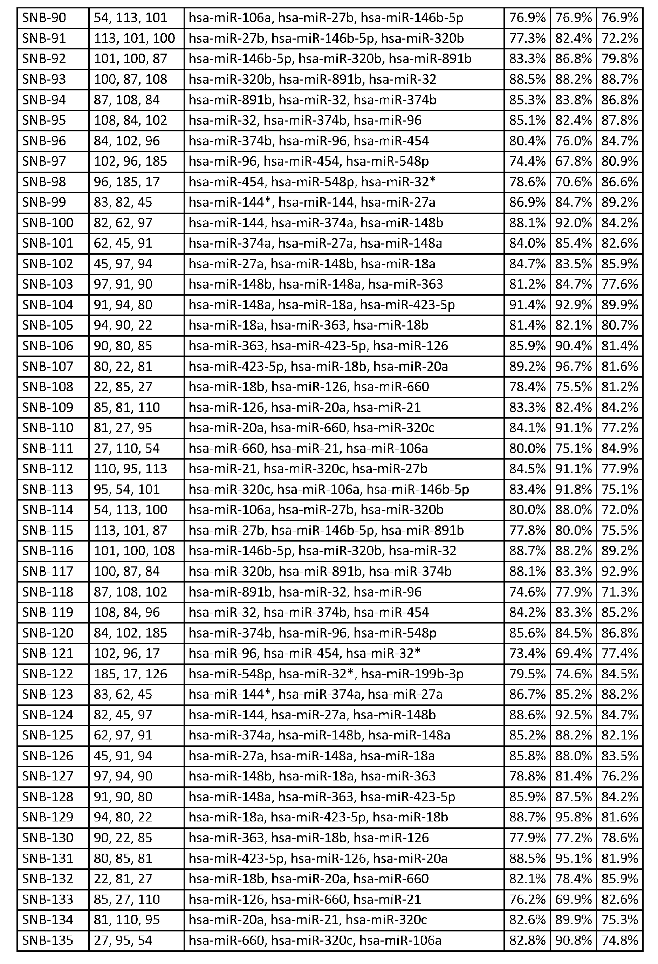

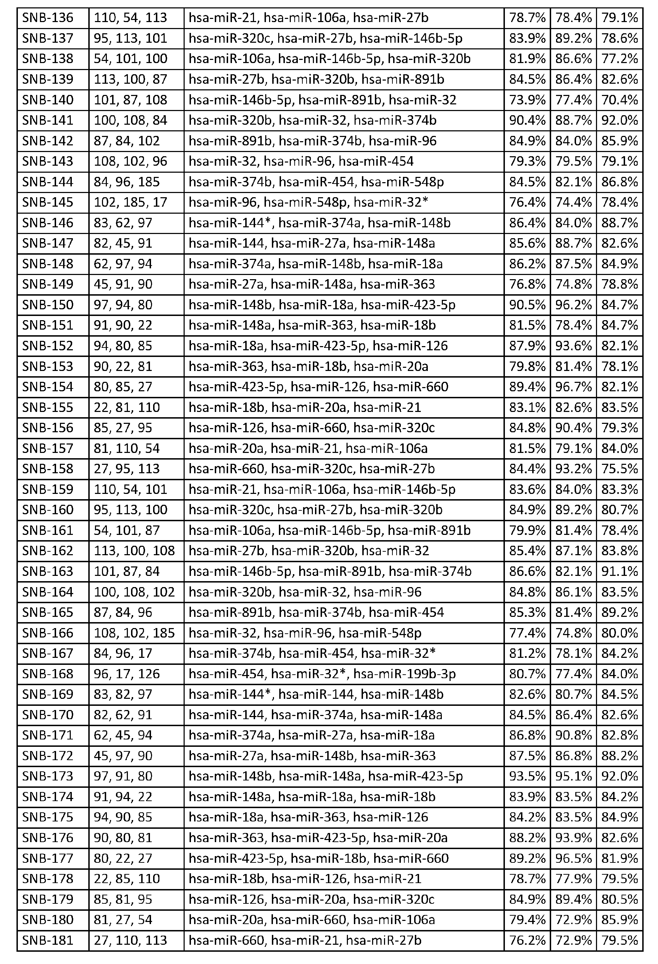

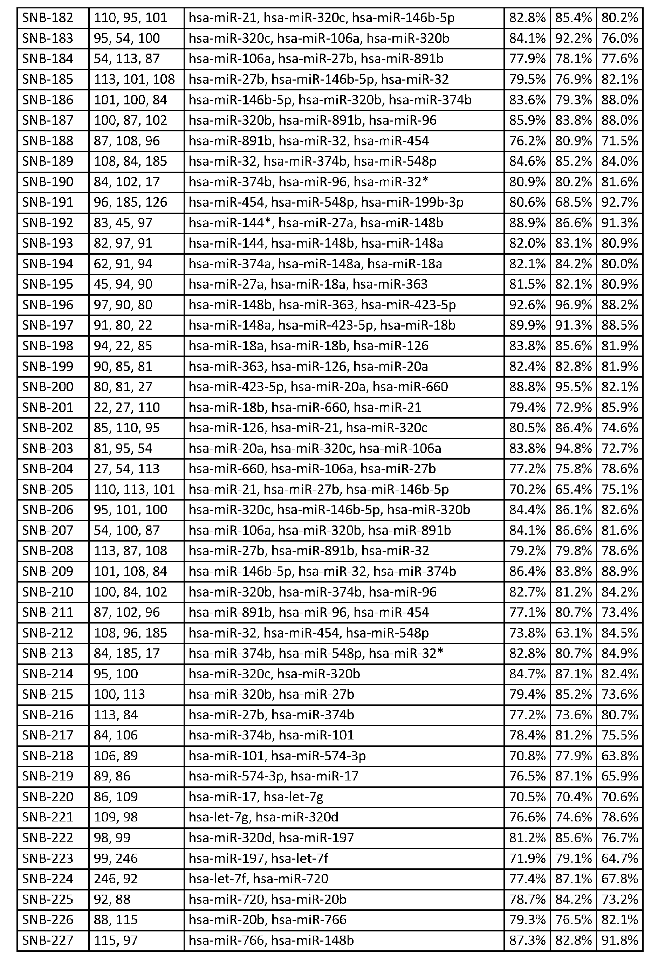

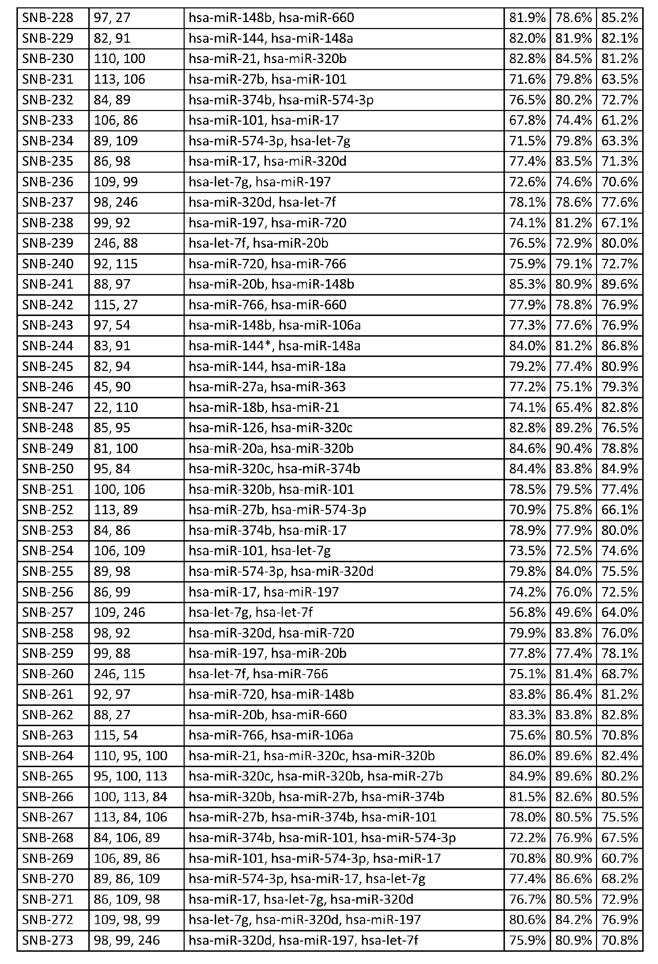

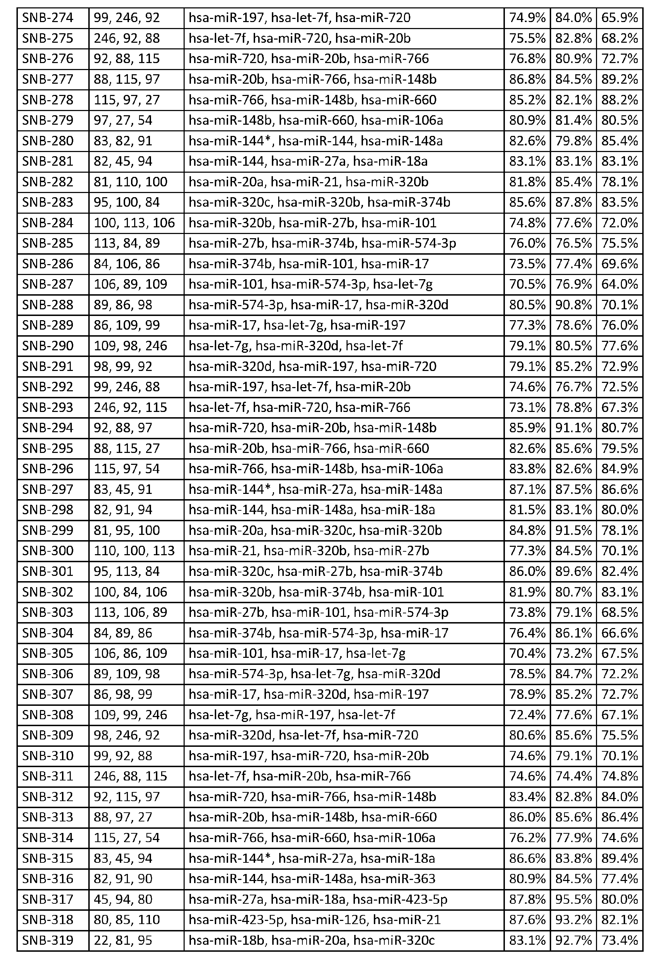

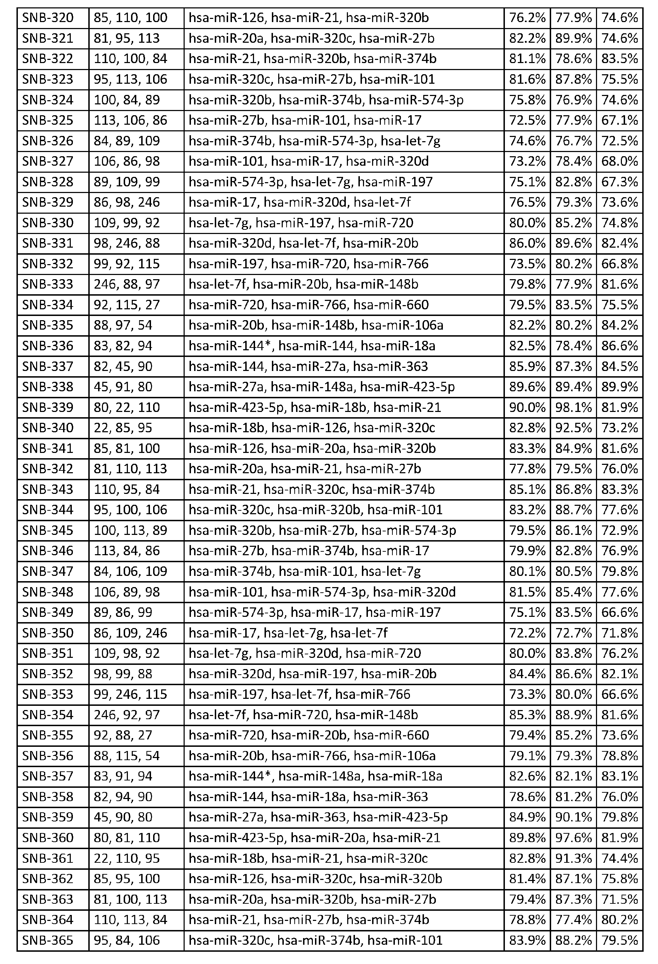

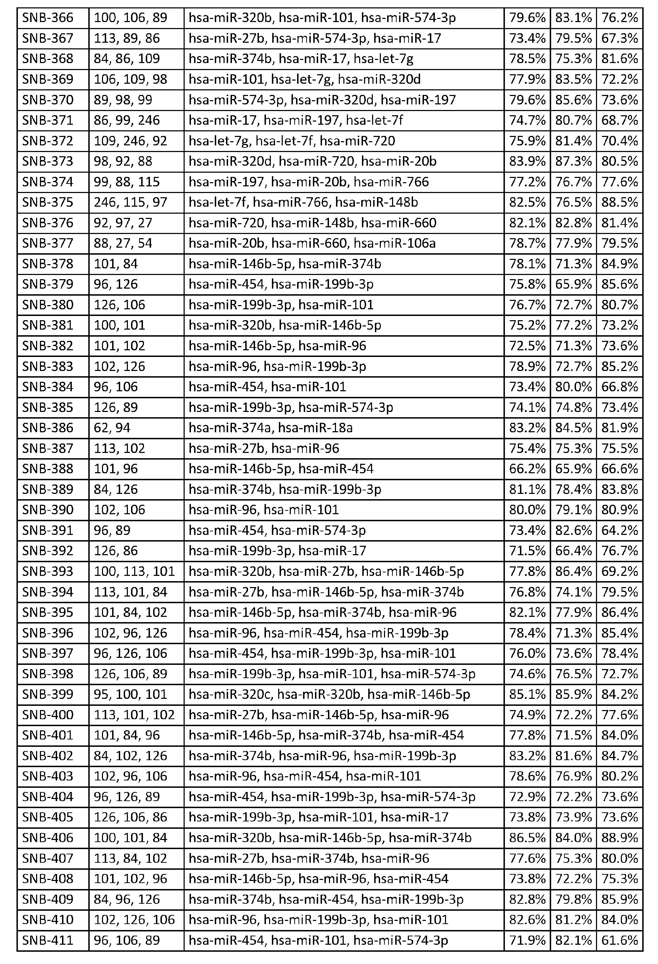

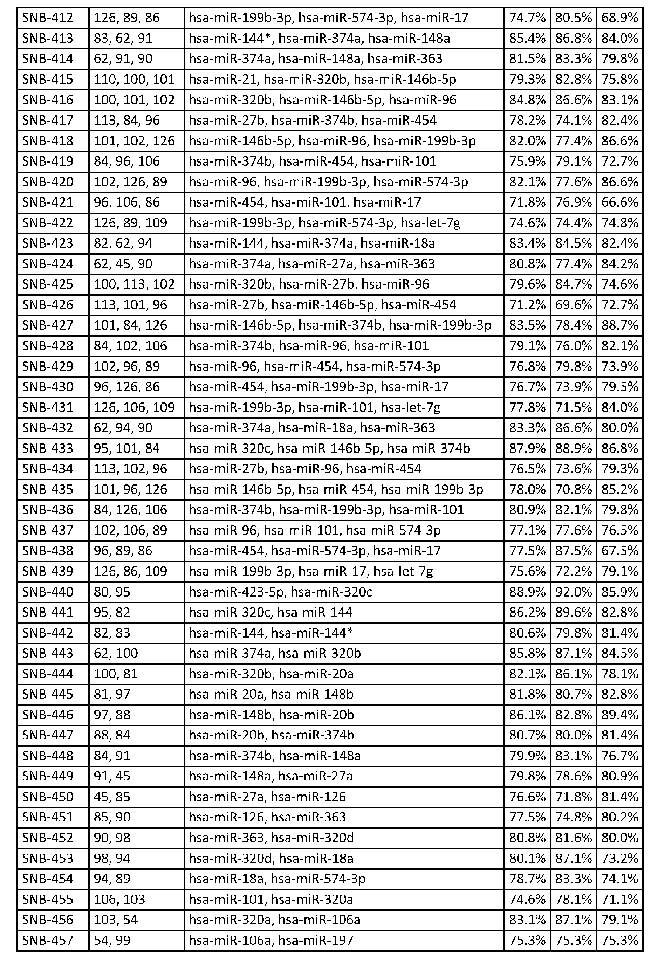

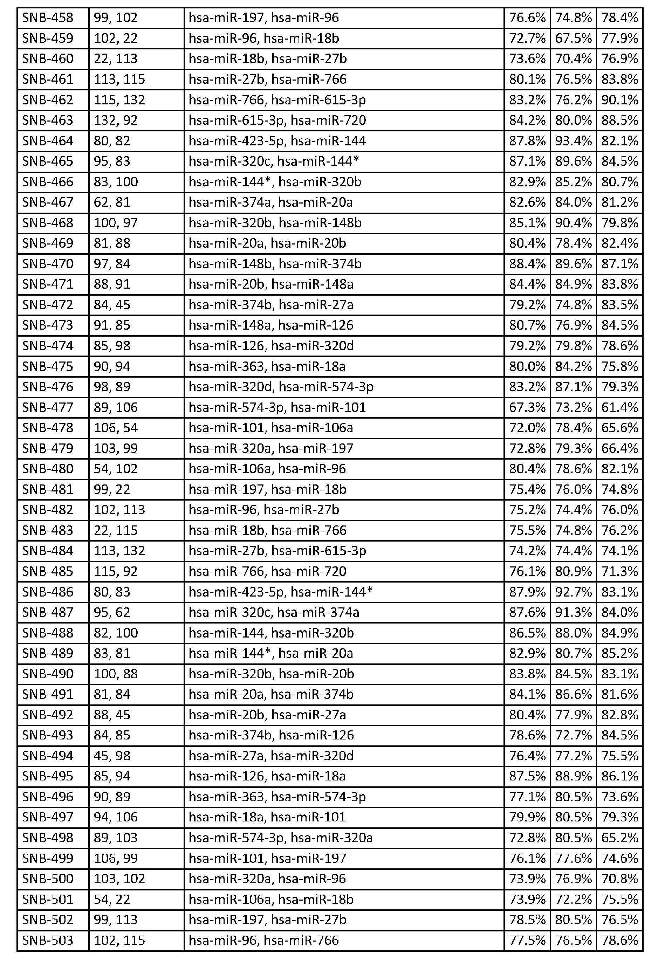

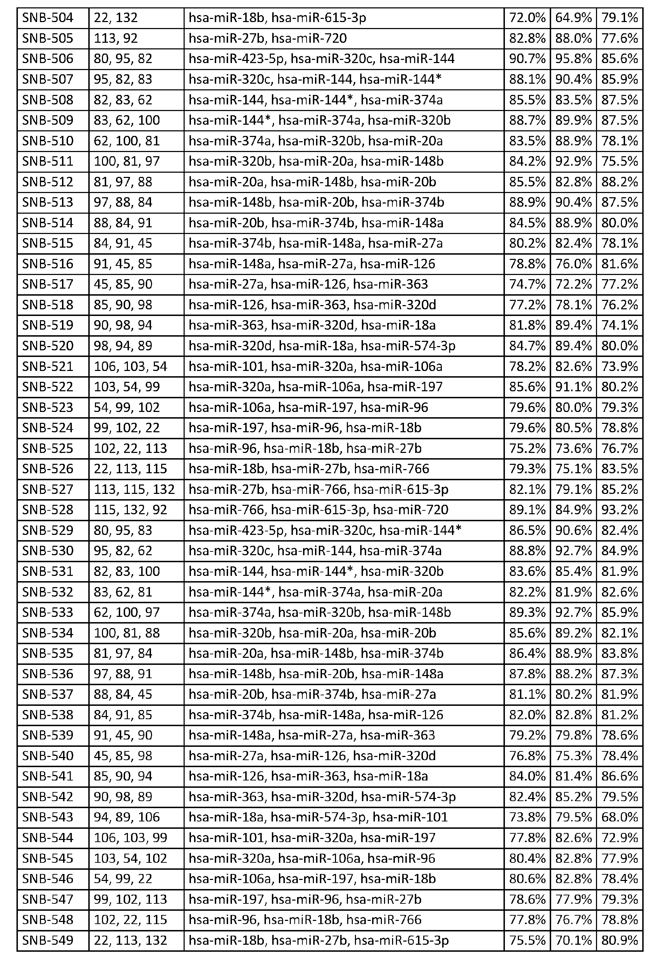

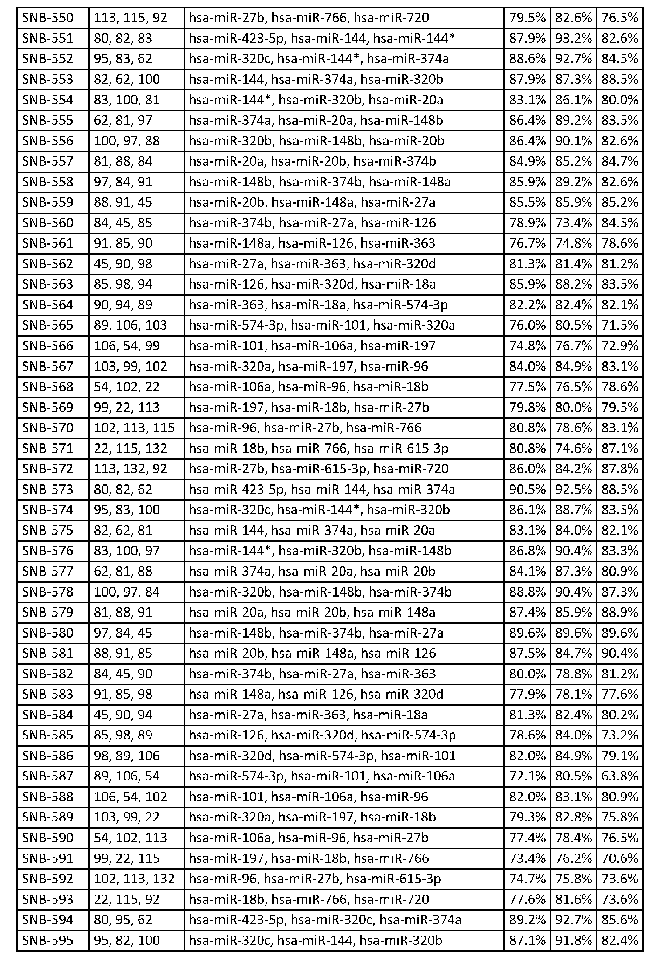

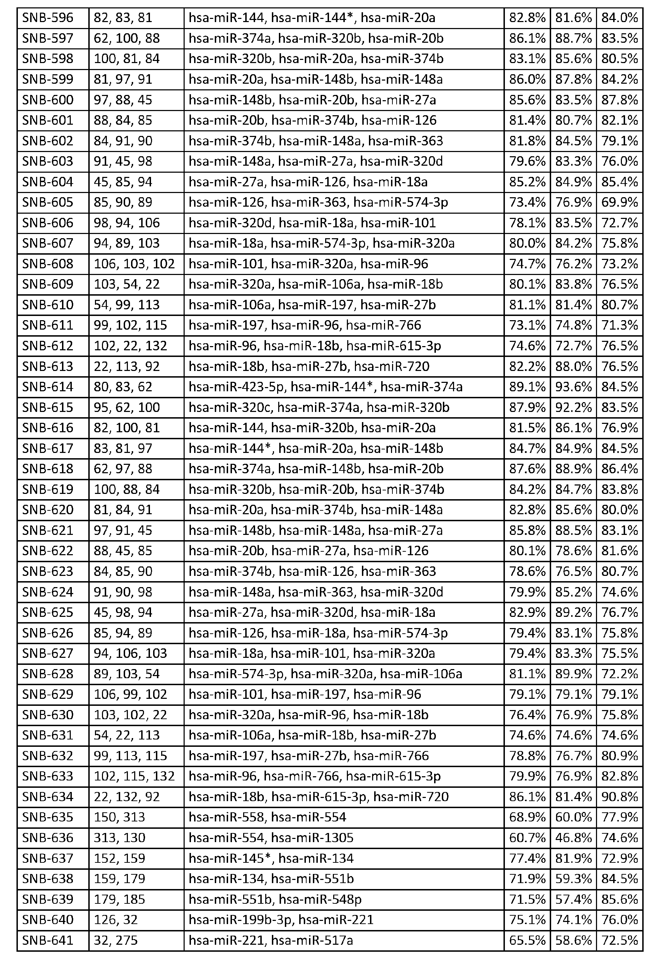

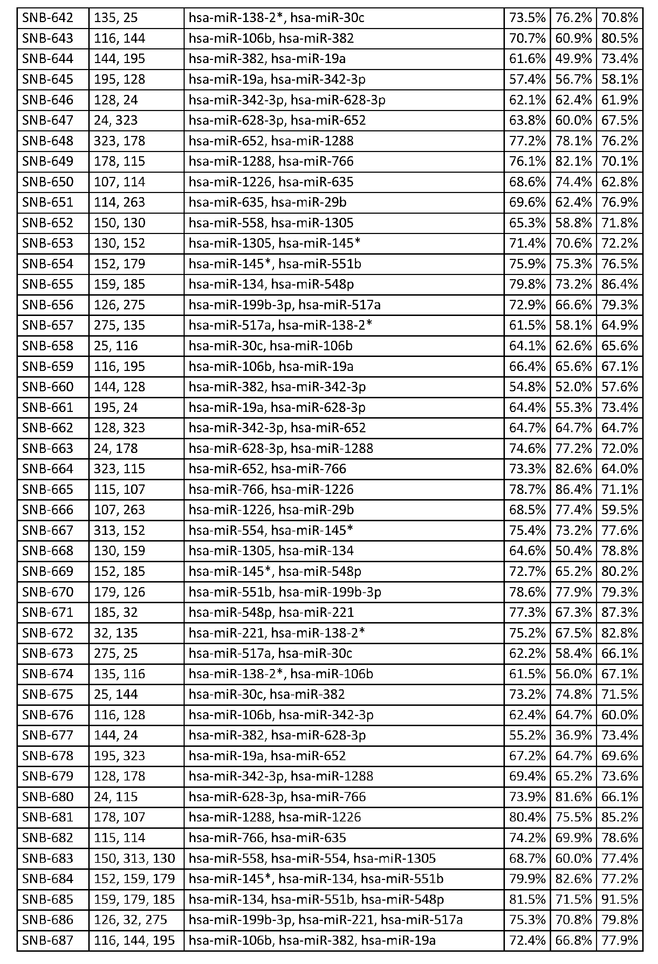

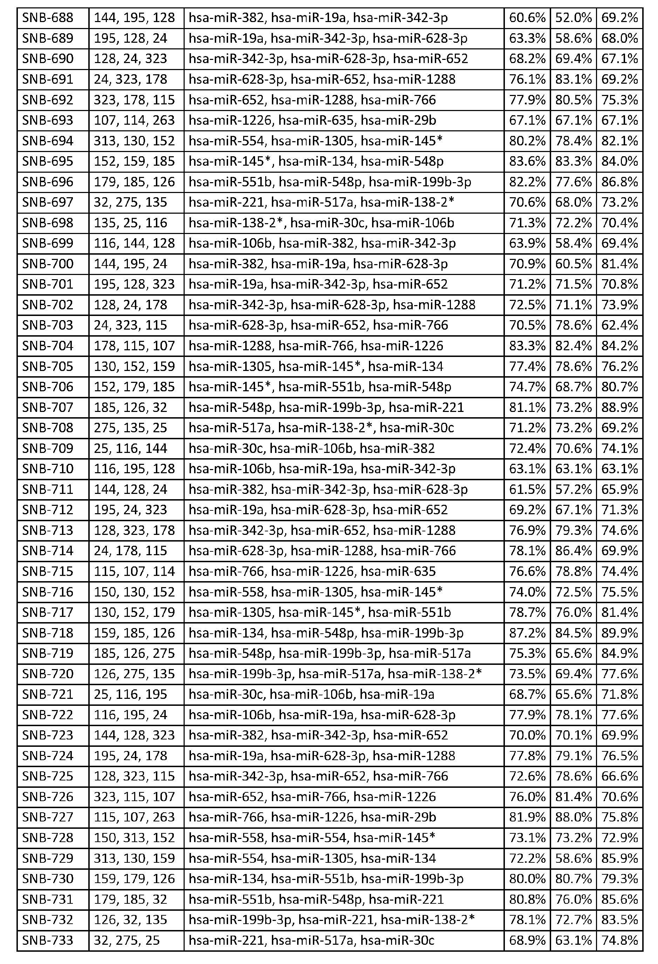

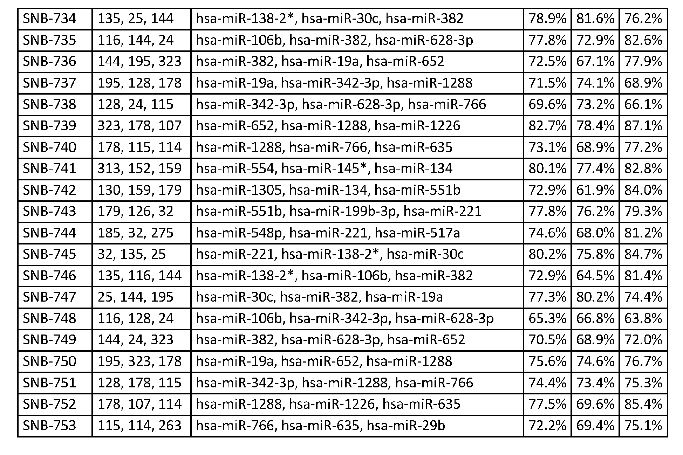

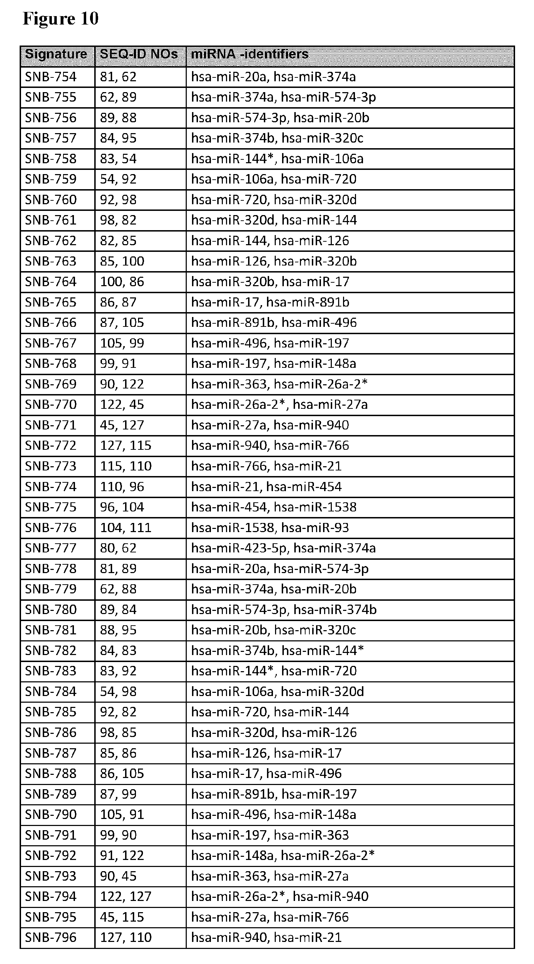

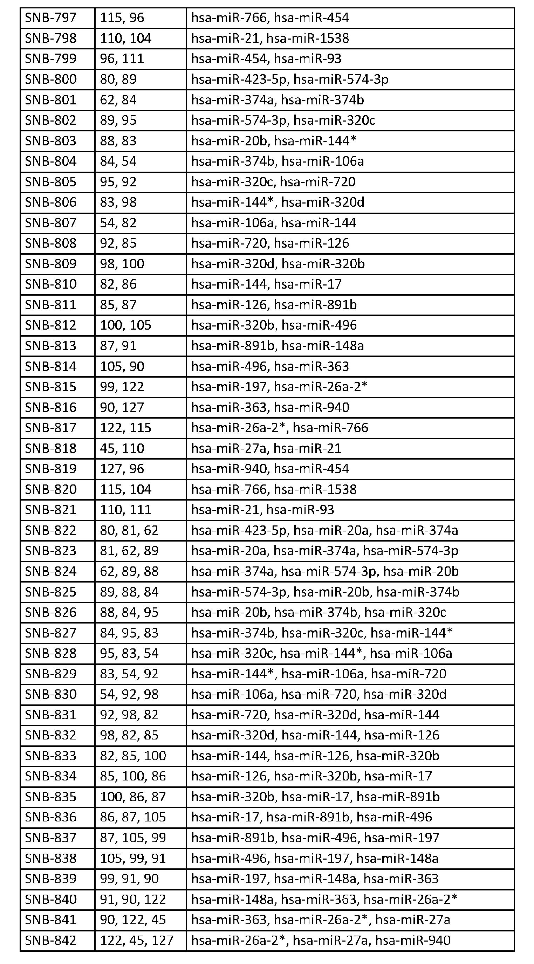

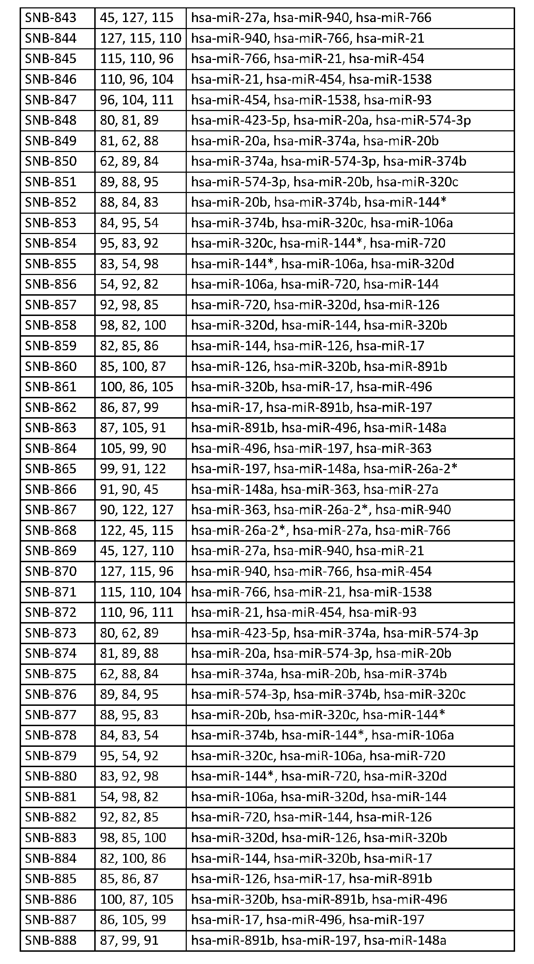

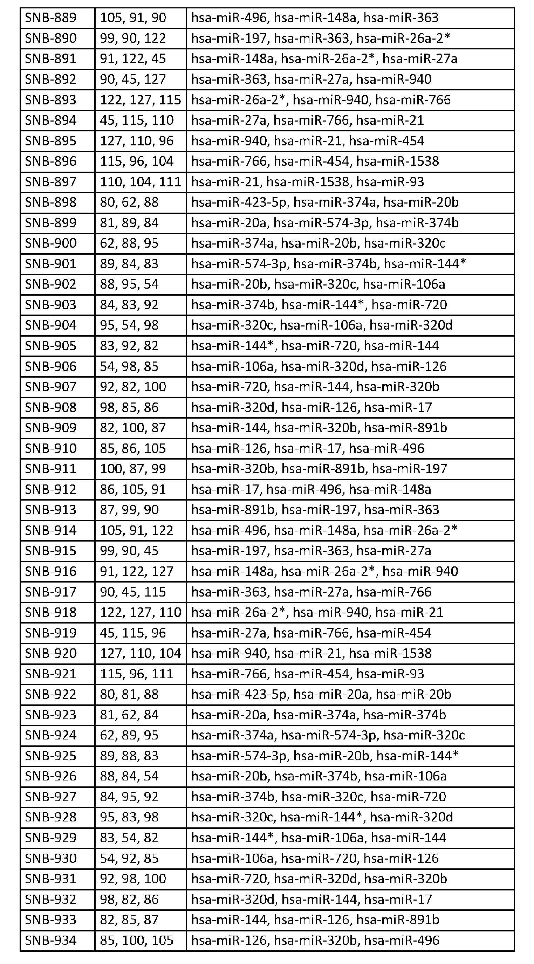

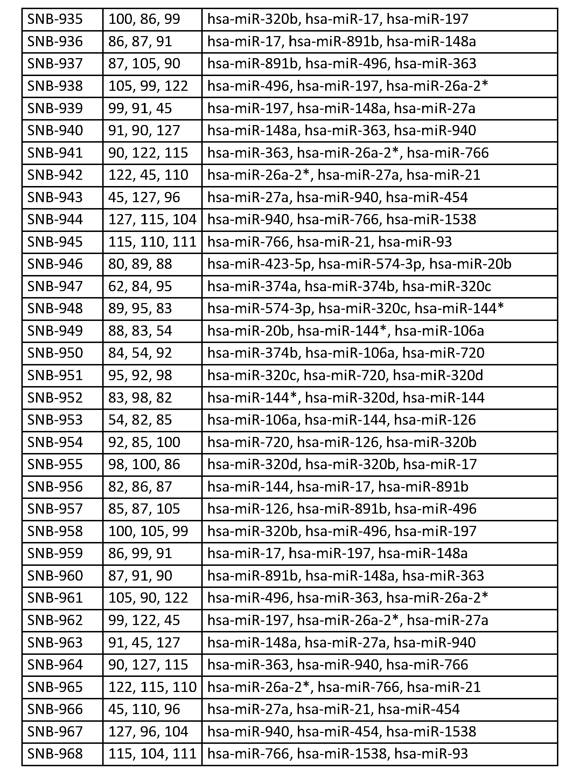

The inventors of the present invention surprisingly found that this approach yields in miRNA sets (signatures) that provide high diagnostic accuracy, specificity and sensitivity when comparing BPH patients and healthy controls (see miRNA sets SNB 1-968 in Figure 4 and Figure 10).

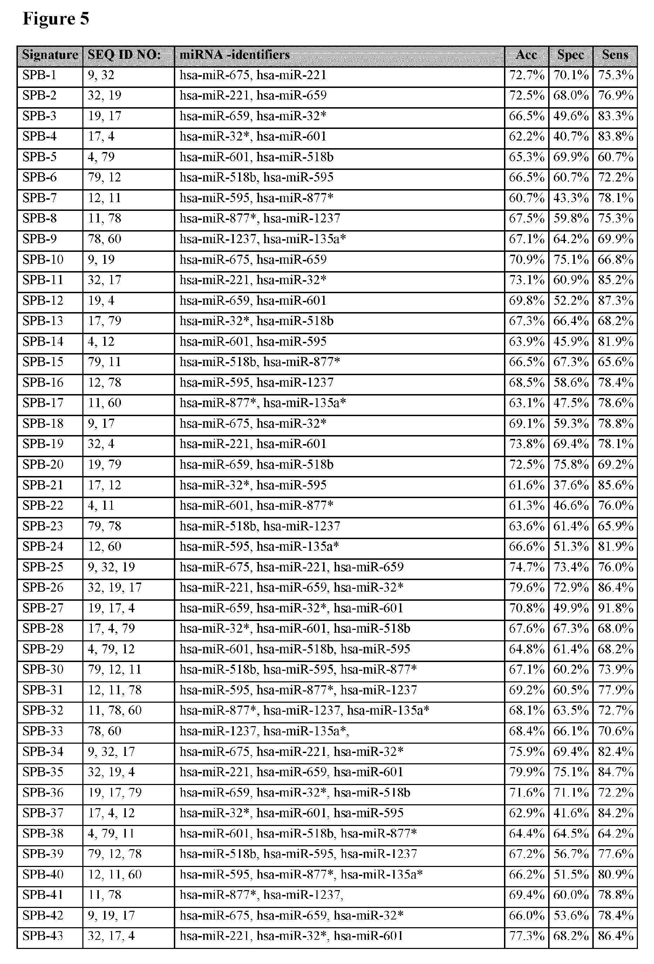

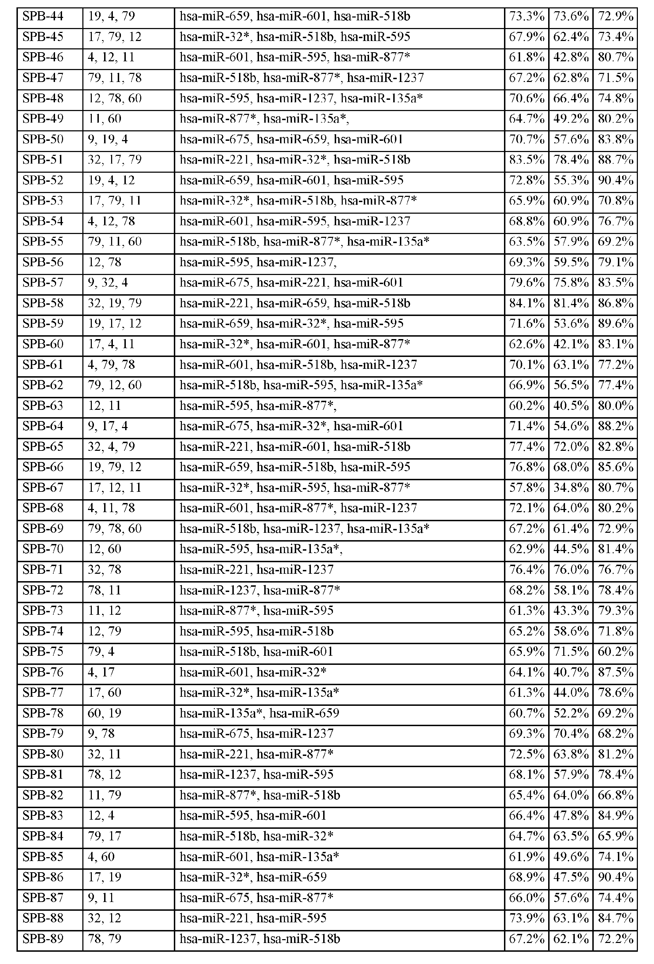

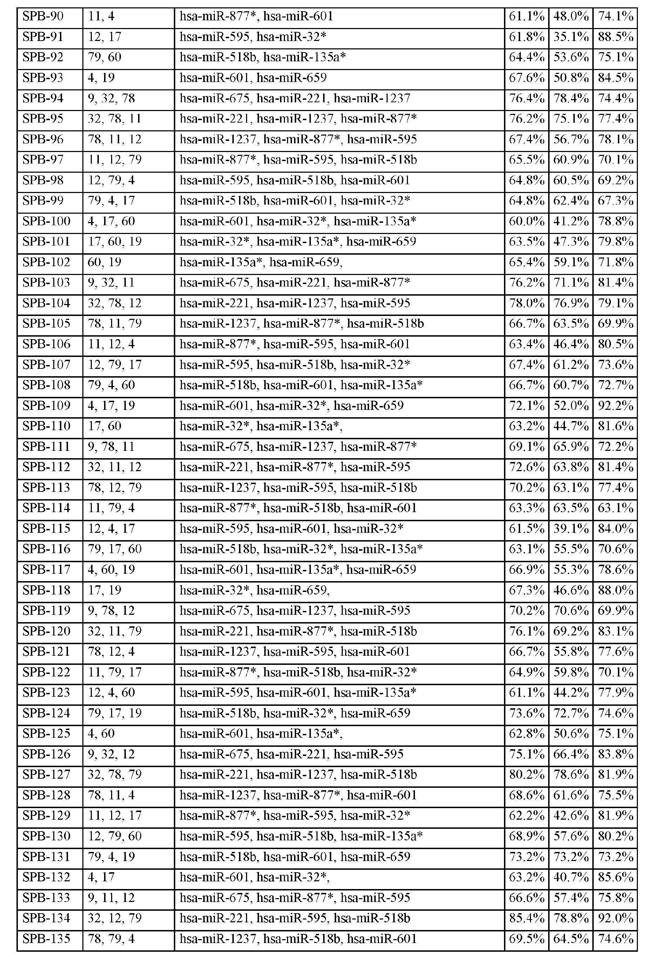

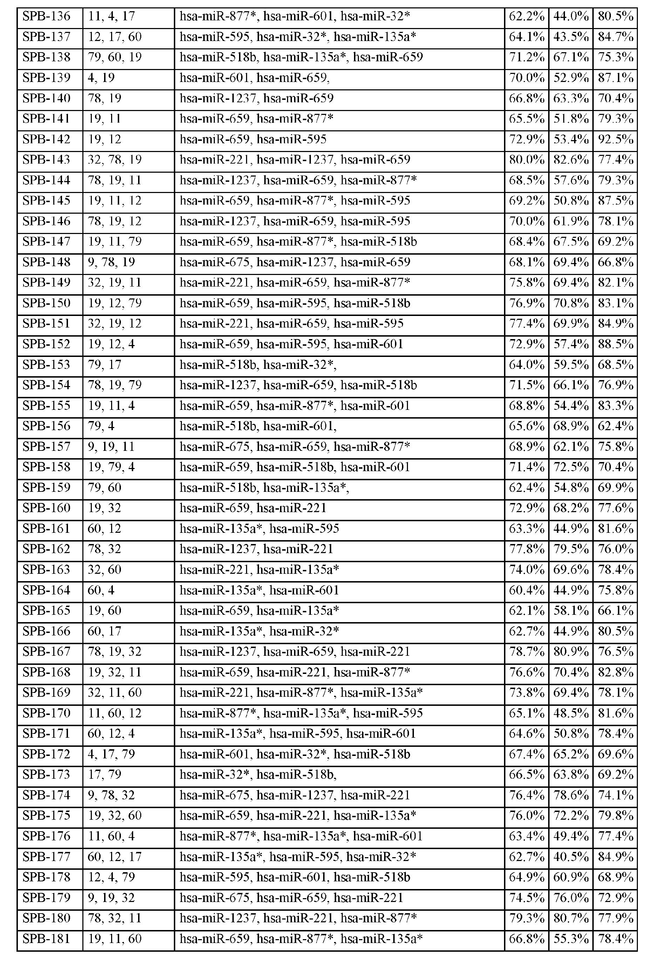

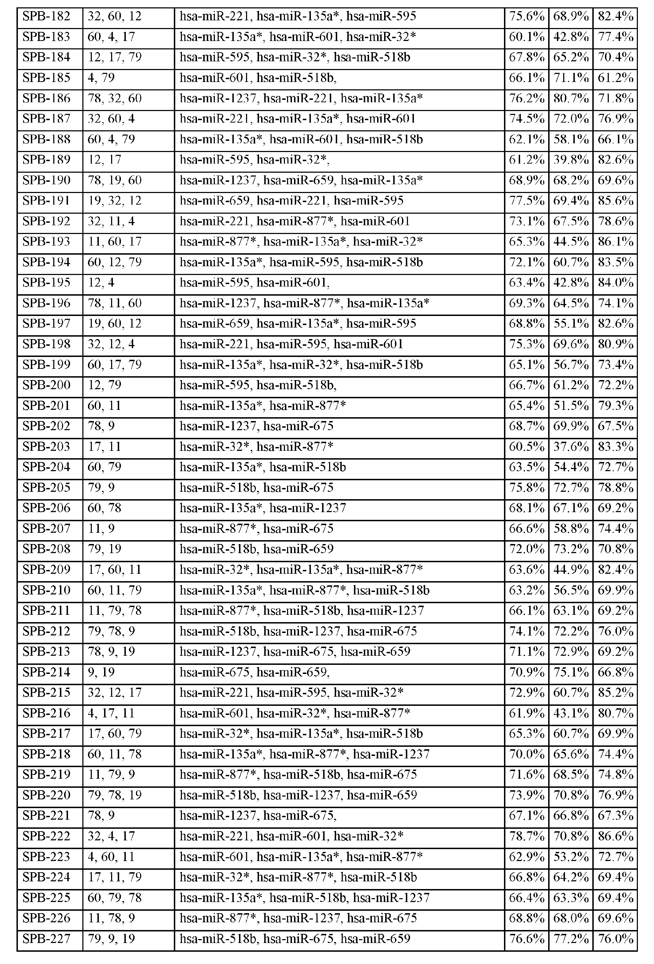

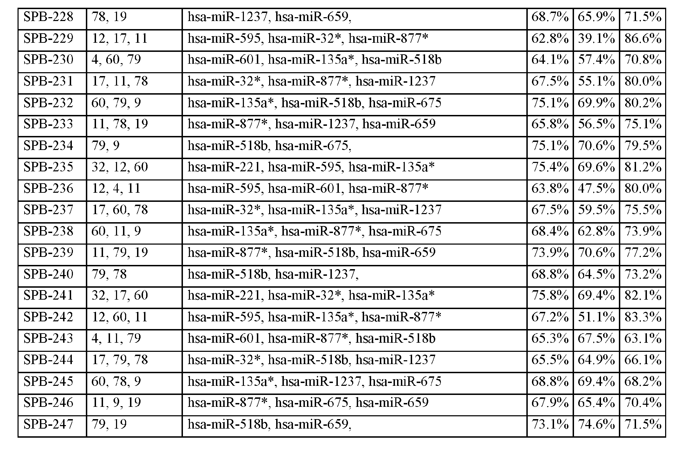

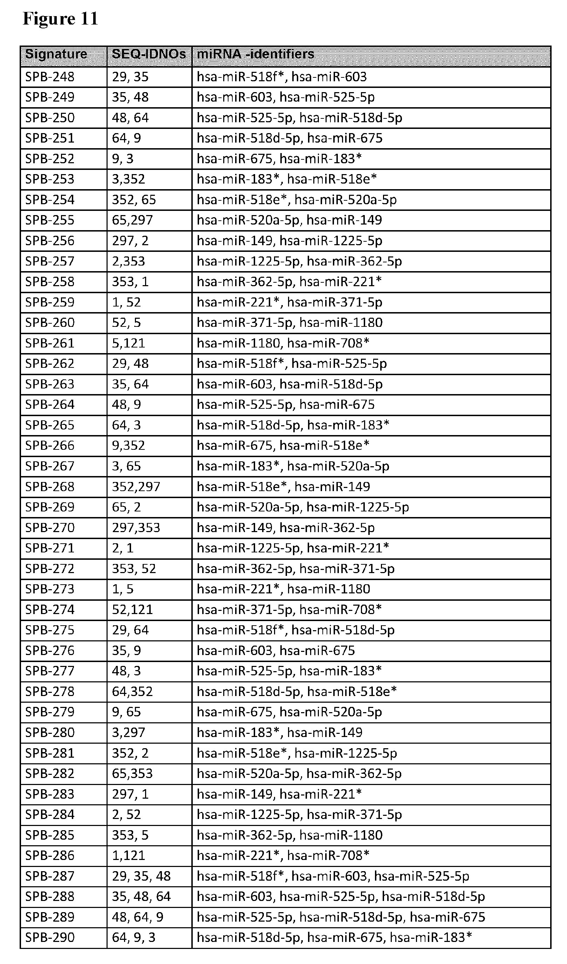

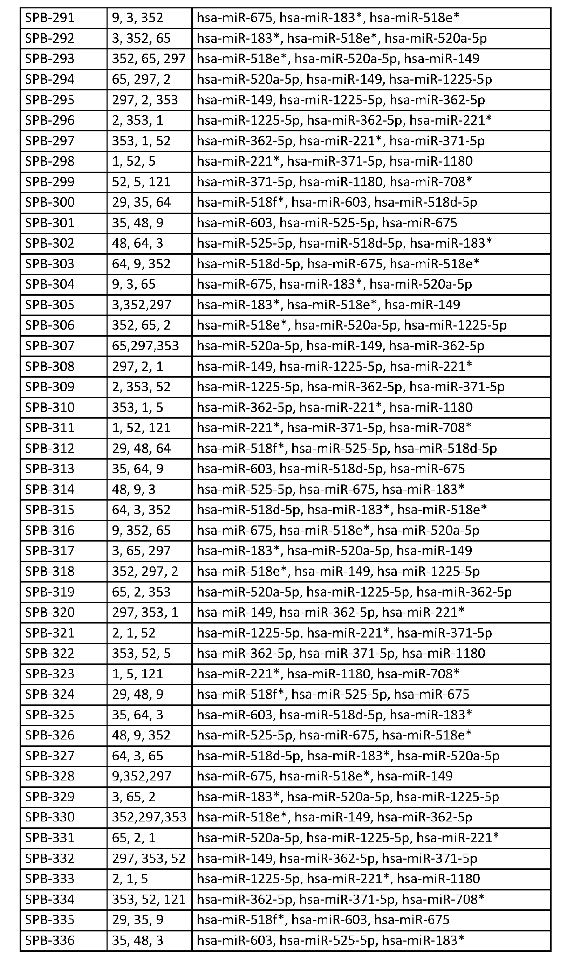

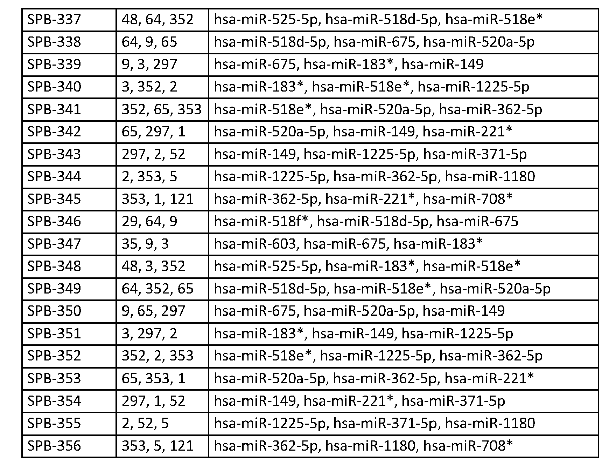

Further, the inventors of the present invention surprisingly found that this approach yields in miRNA sets (signatures) that provide high diagnostic accuracy, specificity and sensitivity when comparing BPH patients and prostate cancer patients (see miRNA sets SPB 1-356 in Figure 5 or Figure 11).

An exemplarily approach to arrive at miRNA sets/signatures that correlate with BPH is summarized below :
Step 1: Total RNA (or subfractions thereof) is extracted from the biological sample, e.g. a body fluid sample, preferably a blood sample (including plasma, serum, PBMC or other blood fractions), using suitable kits and/or purification methods.
Step 2: From the respective samples the quantity (expression level) of one miRNA or sets of at least two miRNAs, e.g. selected from the group listed in Figure 2 or Figure 7(BPH versus healthy control) or e.g. Figure 3 or Figure 8 (BPH versus prostate cancer) , is measured using experimental techniques. These techniques include but are not restricted to array based approaches, amplification methods (PCR, RT-PCR, qPCR), sequencing, next generation sequencing, flow cytometry and/or mass spectroscopy.
Step 3: In order to gather information on the diagnostic/prognostic value and the redundancy of each of the single miRNA biomarkers, mathematical methods are applied. These methods include, but are not restricted to, basic mathematic approaches (e.g. Fold Quotients, Signal to Noise ratios, Correlation), statistical methods as hypothesis tests (e.g. t-test, Wilcoxon-Mann-Whitney test), the Area under the Receiver operator Characteristics Curve, information theory approaches, (e.g. the Mutual Information, Cross-entropy), probability theory (e.g. joint and conditional probabilities) or combinations and modifications of the previously mentioned methods.
Step 4: The information gathered in step 3) is used to estimate for each miRNA biomarker the diagnostic content or value. Usually, however, this diagnostic value is too small to get a highly accurate diagnosis with accuracy rates, specificities and sensitivities beyond the 90% barrier. The diagnostic content of the miRNAs suitable for diagnosing/prognosing BPH is exemplarily listed in Figure 2 or 7 or Figure 3 or 8
Step 5: In order to increase the performance for diagnosing/prognosing of subjects suffering from BPH, more than one miRNA biomarker needs to be employed. Thus statistical learning / machine learning / bioinformatics / computational approaches are applied for set selection in order to select/define sets of miRNA biomarkers that are tailored for the detection of BPH. These techniques include, but are not restricted to, Wrapper subset selection techniques (e.g. forward step-wise, backward step-wise, combinatorial approaches, optimization approaches), filter subset selection methods (e.g. the methods mentioned in Step 3), principal component analysis, or combinations and modifications of such methods (e.g. hybrid approaches).
Step 6: The subsets, selected/defined in Step 5, which may range from only a small number (at least two for the set) to all measured biomarkers is then used to carry out a diagnosis/prognosis of BPH. To this end, statistical learning / machine learning / bioinformatics / computational approaches are applied that include but are not restricted to any type of supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.
Step 7: By combination of subset selection (Step 5) and machine learning (Step 6) an algorithm or mathematical function for diagnosing/prognosing BPH is obtained. This algorithm or mathematical function is applied to a miRNA expression profile of a subject to be diagnosed for BPH. This approach results in sets of miRNAs that are suitable for diagnosing/prognosing BPH, preferably when comparing healthy controls and BPH patients (see Figure 4 or 10 for suitable miRNA sets) or alternatively when comparing prostate cancer patients and BPH patients (see Figure 5 or 11 for suitable miRNA sets)

In a first aspect, the present invention relates to a method for diagnosing of BPH comprising the steps of:
(i) determining an expression profile of a set comprising at least two miRNAs a blood cell sample from a patient, and
(ii) comparing said expression profile to a reference,
wherein the set of miRNAs comprises hsa-miR-675 and hsa-miR-221 that are differentially regulated in blood cell samples from BPH patients as compared to prostate cancer patients and wherein the comparison of said expression profile to said reference allows to discriminate between BPH and prostate cancer.

It is preferred that the sample is blood cell sample or a leukocyte containing sample, particularly preferred it is a whole blood, PBMC, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

Further disclosed but not encompassed by the first aspect of the invention, the set of miRNAs comprises miRNAs that are differentially regulated in BPH patients as compared to healthy controls.

Further disclosed but not encompassed by the first aspect of the invention, the set comprising at least two miRNAs that are differentially regulated in BPH patients as compared to healthy controls is selected from the set of miRNAs listed in Figure 2 or Figure 7.

Further disclosed but not encompassed by the first aspect of the invention the set comprising at least two miRNAs that are differentially regulated in BPH patients as compared to healthy controls is selected from the sets of miRNAs listed in Figure 4 or Figure 10 (SNB-1 to SNB-968).

Further disclosed but not encompassed by the first aspect of the invention the set comprising at least two miRNAs that are differentially regulated in BPH patients as compared to healthy controls comprises at least one set of miRNAs listed in Figure 4 or Figure 10.

Further disclosed but not encompassed by the first aspect of the invention is a method for determining an expression profile of the set comprising at least two miRNAs that are differentially regulated in BPH patients as compared to healthy controls comprises the miRNAs from one set or a plurality of sets of miRNAs listed in Figure 4 or Figure 10.

For example, a set comprising 30 miRNAs that are differentially regulated in BPH patients as compared to healthy controls in a body fluid sample from a subject comprises at least the miRNAs from one set or several sets of miRNAs listed in Figure 4. Alternatively, a set comprising 29, 28, 27,26, 25, 24, 23, 22, 21, 20, 19, 18, 17, 16, 15, 14, 13, 12, 11, 10, 9, 8, 7, 6, 5, 4 or 3 miRNAs that are differentially regulated in BPH patients as compared to healthy controls comprises at least the miRNAs from one set or several sets of miRNAs listed in Figure 4 or Figure 10.

Further disclosed but not encompassed by the first aspect of the invention is a method for determining an expression profile of the set comprising at least two that are differentially regulated in BPH patients as compared to healthy controls in a body fluid sample from a subject comprises combinations of sets of miRNAs listed in Figure 4 or Figure 10.

For example, said set comprising 30 miRNAs that are differentially regulated in BPH patients as compared to healthy controls in a body fluid sample from a subject comprises at least 2, e.g. 2, 3, 4, 5 or 6, sets of miRNAs listed in Figure 4 or Figure 10. Alternatively, said set comprising 29, 28, 27 ,26, 25, 24, 23, 22, 21, 20, 19, 18, 17, 16, 15, 14, 13, 12, 11, 10, 9, 8, 7, 6, 5 or 4 miRNAs comprises a least 2, e.g. 2, 3, 4, 5 or 6, sets of miRNAs listed in Figure 4 or Figure 10.

The reference expression profile may be obtained from at least two subjects (e.g. human or animal). Preferably, the reference expression profile is an average expression profile (data) of at least 2 to 400 subjects, more preferably at least 20 to 200 subjects, and most preferably at least 40 to 150 subjects, with two known clinical conditions which are BPH or a specific form of BPH and healthy control.

According to the first aspect of the present invention, the set of miRNAs comprises miRNAs that are differentially regulated in BPH patients as compared to prostate cancer patients.

Preferably, the set comprising at least two miRNAs that are differentially regulated in BPH patients as compared to prostate cancer patients is selected from the set of miRNAs listed in Figure 3 or Figure 8.

It is preferred that the set comprising at least two miRNAs that are differentially regulated in BPH patients as compared to prostate cancer patients is selected from the sets of miRNAs listed in Figure 5 (SPB-1 to SPB-247) or Figure 11 (SPB-248-356).

It is also preferred that the set comprising at least two miRNAs that are differentially regulated in BPH patients as compared to prostate cancer patients comprises at least one set of miRNAs listed in Figure 5 or Figure 11.

Further, according to the method of the present invention, for determining an expression profile of the set comprising at least two miRNAs that are differentially regulated in BPH patients as compared to prostate cancer patients in a body fluid sample from a subject comprises the miRNAs from one set or a plurality of sets of miRNAs listed in Figure 5 or Figure 11.

For example, a set comprising 30 miRNAs that are differentially regulated BPH patients as compared to prostate cancer patients in a body fluid sample from a subject comprises at least the miRNAs from one set or several sets of miRNAs listed in Figure 5 or Figure 11. Alternatively, a set comprising 29, 28, 27,26, 25, 24, 23, 22, 21, 20, 19, 18, 17, 16, 15, 14, 13, 12, 11, 10, 9, 8, 7, 6, 5, 4 or 3 miRNAs that are differentially regulated in BPH patients as compared to prostate cancer patients comprises at least the miRNAs from one set or several sets of miRNAs listed in Figure 5 or Figure 11.

Further, according to the method of the present invention, for determining an expression profile of the set comprising at least two that are differentially regulated in BPH patients as compared to prostate cancer patients in a body fluid sample from a subject comprises combinations of sets of miRNAs listed in Figure 5 or Figure 11. For example, said set comprising 30 miRNAs that are differentially regulated in BPH patients as compared to prostate cancer patients in a body fluid sample from a subject comprises at least 2, e.g. 2, 3, 4, 5 or 6, sets of miRNAs listed in Figure 5 or Figure 11. Alternatively, said set comprising 29, 28, 27 ,26, 25, 24, 23, 22, 21, 20, 19, 18, 17, 16, 15, 14, 13, 12, 11, 10, 9, 8, 7, 6, 5 or 4 miRNAs comprises a least 2, e.g. 2, 3, 4, 5 or 6, sets of miRNAs listed in Figure 5 or Figure 11.

The reference may be a reference expression profile that may be obtained from at least two subjects (e.g. human or animal). Preferably the reference expression profile is an average expression profile (data) of at least 2 to 200 subjects, more preferably at least 10 to 150 subjects, and most preferably at least 20 to 100 subjects, with two known clinical conditions which are BPH or a specific form of BPH and prostate cancer or a specific form of prostate cancer.

It is particularly preferred that the reference is an algorithm or mathematical function. Preferably the algorithm or mathematical function is obtained from a reference expression profile (data) of at least two subjects, preferably the algorithm or mathematical function is obtained from an average reference expression profile (data) of at least 2 to 200 subjects, more preferably of at least 10 to 150 subjects, and most preferably of at least 20 to 100 subjects.

It is preferred that the algorithm or mathematical function is obtained using a machine learning approach
Preferably, the algorithm or mathematical function is saved on a data carrier comprised in the kit (according to the seventh aspect of the invention) or the computer program, wherein the algorithm or mathematical function is comprised, is saved on a data carrier comprised in the kit.

Preferably, the machine learning approach involves the following steps:
(i) inputting the reference expression profile(s) of (a) subject(s) with the known clinical condition of BPH or condition and/or with any other known clinical condition(s), preferably with the known clinical condition of healthy control or alternatively prostate cancer, and
(ii) computing an algorithm or a mathematical function based on said reference expression profile(s) that is suitable to distinguish between the (likely) clinical condition of BPH or any other (likely) clinical condition(s), preferably the clinical condition of healthy control or alternatively prostate cancer, or to decide if the clinical condition of BPH or any other condition, or no periodontal disease or condition is present or will likely be present in said patient.

It is preferred that the miRNA expression profile may be generated by any convenient means, e.g. nucleic acid hybridization (e.g. to a microarray), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX) and the like, that allow the analysis of differential miRNA expression levels between samples of a subject (e.g. diseased) and a control subject (e.g. healthy, reference sample).

Nucleic acid hybridization may be performed using a microarray/biochip or *in situ* hybridization. *In situ* hybridization is preferred for the analysis of a single miRNA or a set comprising a low number of miRNAs (e.g. a set of at least 2 to 50 miRNAs such as a set of 2, 5, 10, 20, 30, or 40 miRNAs). The microarray/biochip, however, allows the analysis of a single miRNA as well as a complex set of miRNAs (e.g. a all known miRNAs or subsets therof).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 2 to 50 miRNAs such as a set of 2, 5, 10, 20, 30, or 40 miRNAs), whereas high-throughput RT-PCR technologies (e.g. OpenArray from Applied Biosystems, SmartPCR from Wafergen, Biomark System from Fluidigm) are also able to measure large sets of miRNAS (e.g a set of 10, 20, 30, 50, 80, 100, 200 or more) or all known miRNAs in a high parallel fashion. RT-PCR is particularly suitable for detecting low abandoned miRNAs.

In a second aspect, the invention relates to the use of a set comprising polynucleotides for detecting a set comprising at least two miRNAs comprising hsa-miR-675 and hsa-miR-221 for diagnosing of BPH in a method for diagnosing BPH according to the first aspect of the invention.

It is preferred that the sample is a blood cell sample or a leukocyte containing sample, particularly preferred it is a whole blood, PBMC sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

Further disclosed but not encompassed by the second aspect of the invention, the set of miRNAs comprises miRNAs that are differentially regulated in BPH patients as compared to healthy controls.

Further disclosed but not encompassed by the second aspect of the invention, the set comprising at least two miRNAs that are differentially regulated in BPH patients as compared to healthy controls is selected from the set of miRNAs listed in Figure 2 or Figure 7.

Further disclosed but not encompassed by the second aspect of the invention the set comprising at least two miRNAs that are differentially regulated in BPH patients as compared to healthy controls.is selected from the set of miRNAs listed in Figure 4 or Figure 10 (SNB 1-968).

Further disclosed but not encompassed by the second aspect of the invention the set comprising at least two miRNAs that are differentially regulated in BPH patients as compared to healthy controls. comprises at least one set of miRNAs listed in Figure 4 or Figure 10.

It is preferred that the polynucleotides comprised in the set of the present invention are complementary to the miRNAs comprised in the set, wherein the nucleotide sequences of said miRNAs are preferably selected from the group consisting of miRNAs listed in Figure 2 or Figure 7 or set of miRNAs listed in Figure 4 or Figure 10, a fragment thereof, and a sequence having at least 80%, 85%, 90% or 95% sequence identity thereto.

For example, the polynucleotides of the present invention are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 miRNAs that are differentially regulated in BPH patients as compared to healthy controls.wherein the set of miRNAs comprises at least one, e.g. 1, 2, 3, 4, 5 or 6, of the set of miRNAs listed in Figure 4 or Figure 10.

According to the second aspect of the present invention, the set of miRNAs comprises miRNAs that are differentially regulated in BPH patients as compared to prostate cancer patients.

Preferably, the set comprising at least two miRNAs that are differentially regulated in BPH patients as compared to prostate cancer patients is selected from the set of miRNAs listed in Figure 3 or Figure 8.

It is preferred that the set comprising at least two miRNAs that are differentially regulated in BPH patients as compared to prostate cancer patients is selected from the set of miRNAs listed in Figure 5 or Figure 11.

It is preferred that the set comprising at least two miRNAs that are differentially regulated in BPH patients as compared to prostate cancer patients. comprises at least one set of miRNAs listed in Figure 5 or Figure 11.

It is preferred that the polynucleotides comprised in the set of the present invention are complementary to the miRNAs comprised in the set, wherein the nucleotide sequences of said miRNAs are preferably selected from the group consisting of miRNAs listed in Figure 3 or Figure 8 or set of miRNAs listed in Figure 5 or Figure 11, a fragment thereof, and a sequence having at least 80%, 85%, 90% or 95% sequence identity thereto.

For example, the polynucleotides of the present invention are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 miRNAs that are differentially regulated in BPH patients as compared to prostate cancer patients wherein the set of miRNAs comprises at least one, e.g. 1, 2, 3, 4, 5 or 6, of the set of miRNAs listed in Figure 5 or Figure 11.

In second aspect, the invention relates to the use of set of polynucleotides for diagnosing BPH in a subject.

Further described is a set of at least two primer pairs for determining the expression level of a set of miRNAs in a body fluid sample of a subject suffering or suspected of suffering from BPH.

It is preferred that the body fluid sample is a blood sample, preferably a blood cell sample or a leukocyte containing sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

In one embodiment of the third aspect of the present invention, the set of miRNAs comprises miRNAs that are differentially regulated in BPH patients as compared to healthy controls.

Further disclosed but not encompassed by present inventions is a set comprising at least two miRNAs that are differentially regulated in BPH patients as compared to healthy controls is selected from the set of miRNAs listed in Figure 2 or Figure 7.

Further disclosed but not encompassed by present inventions is a set comprising at least two miRNAs that are differentially regulated in BPH patients as compared to healthy controls is selected from the sets of miRNAs listed in Figure 4 or Figure 10.

Further disclosed but not encompassed by present inventions is a set comprising at least two miRNAs that are differentially regulated in BPH patients as compared to healthy controls comprises at least one set of miRNAs listed in Figure 4 or Figure 10.

Further disclosed but not encompassed by present inventions is a set of at least two primer pairs for determining the expression level of a set of miRNAs that are differentially regulated in BPH patients as compared to healthy controls are primer pairs that are specific for at least one miRNA listed in Figure 2 or Figure 7.

Further disclosed but not encompassed by present inventions is a set of at least two primer pairs for determining the expression level of a set of miRNAs that are differentially regulated in BPH patients as compared to healthy controls are primer pairs that are specific for at least one set of miRNAs listed in Figure 4 or Figure 10.

Further disclosed but not encompassed by present inventions is a set of at least two primer pairs are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, and wherein the set of miRNAs comprises at least one of the sets listed in Figure 4 or Figure 10.

For example, the set of at least two primer pairs are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 or 2 miRNAs wherein the set of miRNAs that are differentially regulated in BPH patients as compared to healthy controls comprises at least one of the set of miRNAs listed in Figure 4 or Figure 10.

Further disclosed but not encompassed by present inventions is that said primer pairs may be used for amplifying cDNA transcripts of the set of miRNAs that are differentially regulated in BPH patients as compared to healthy controls selected from the miRNAs listed in Figure 2 or Figure 7. Furthermore, the said primer pairs may be used for amplifying cDNA transcripts of the set of miRNAs listed in Figure 4 or Figure 10.

It is understood that the primer pairs for detecting a set of miRNAs may consist of specific and or non-specific primers. Additionally, the set of primer pairs may be complemented by other substances or reagents (e.g. buffers, enzymes, dye, labelled probes) known to the skilled in the art for conducting real time polymerase chain reaction (RT-PCR).

According to the third aspect of the present invention, the set of miRNAs comprises miRNAs that are differentially regulated in BPH patients as compared to prostate cancer patients.

Preferably, the set comprising at least two miRNAs that are differentially regulated in BPH patients as compared to prostate cancer patients is selected from the set of miRNAs listed in Figure 3 or Figure 8.

It is preferred that the set comprising at least two miRNAs that are differentially regulated in BPH patients as compared to prostate cancer patients is selected from the sets of miRNAs listed in Figure 5 or Figure 11.

It is preferred that the set comprising at least two miRNAs that are differentially regulated in BPH patients as compared to prostate cancer patients comprises at least one set of miRNAs listed in Figure 5 or Figure 11.

It is preferred that the set of at least two primer pairs for determining the expression level of a set of miRNAs that are differentially regulated in BPH patients as compared to prostate cancer patients are primer pairs that are specific for at least one miRNA listed in Figure 3 or Figure 8.

It is preferred that the set of at least two primer pairs for determining the expression level of a set of miRNAs that are differentially regulated in BPH patients as compared to prostate cancer patients are primer pairs that are specific for at least one set of miRNAs listed in Figure 5 or Figure 11.

It is preferred that the set of at least two primer pairs disclosed therein are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, and wherein the set of miRNAs comprises at least one of the sets listed in Figure 5 or Figure 11.

For example, the set of at least two primer pairs disclosed therein are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 or 2 miRNAs wherein the set of miRNAs that are differentially regulated in BPH patients as compared to prostate cancer patients comprises at least one of the set of miRNAs listed in Figure 5 or Figure 11.

Preferably, the said primer pairs may be used for amplifying cDNA transcripts of the set of miRNAs that are differentially regulated in BPH patients as compared to prostate cancer patients selected from the miRNAs listed in Figure 3 or Figure 8. Furthermore, the said primer pairs may be used for amplifying cDNA transcripts of the set of miRNAs listed in Figure 5 or Figure 11.

It is understood that the primer pairs for detecting a set of miRNAs may consist of specific and or non-specific primers. Additionally, the set of primer pairs may be complemented by other substances or reagents (e.g. buffers, enzymes, dye, labelled probes) known to the skilled in the art for conducting real time polymerase chain reaction (RT-PCR).

Further described is the use of a set of primer pairs for diagnosing BPH in a subject.

Still further described are means for diagnosing of BPH in a body fluid sample of a subject.

Further disclosed are means for diagnosing of BPH in a body fluid sample of a subject comprising
(i) a set of at least two polynucleotides according to the second aspect of the invention or
(ii) a set of at least two primer pairs according the fourth aspect of the invention.

It is preferred that the body fluid sample is a blood sample, preferably a blood cell sample or a leukocyte containing sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

It is further described but not encompassed by the present invention that the set of at least two polynucleotides or the set of at least 2 primer pairs are for detecting a set comprising at least two miRNAs that are differentially regulated in BPH patients as compared to healthy controls.in a body fluid sample, e.g. blood sample, from a subject, e.g. patient, human or animal, wherein the set of miRNAs is selected from the miRNAs listed in Figure 2 or Figure 7.

It is further described but not encompassed by the present invention that the set of at least two polynucleotides or the set of at least 2 primer pairs are for detecting a set comprising at least two miRNAs that are differentially regulated in BPH patients as compared to healthy controls.in a body fluid sample, e.g. blood sample, from a subject, e.g. patient, human or animal, wherein the set of miRNAs is selected from the sets of miRNAs listed in Figure 4 or Figure 10.

It is further described but not encompassed by the present invention that the set of at least two primer pairs for determining the expression level of a set of miRNAs that are differentially regulated in BPH patients as compared to healthy controls. are primer pairs that are specific for at least two miRNAs selected from the miRNAs listed in Figure 2 or Figure 7.

It is further described but not encompassed by the present invention that the set of at least two primer pairs for determining the expression level of a set of miRNAs that are differentially regulated in BPH patients as compared to healthy controls. are primer pairs that are specific for at least one set of miRNAs listed in Figure 4 or Figure 10.

Further, the set of at least two polynucleotides or the set of at least 2 primer pairs are for detecting a set comprising at least two miRNAs that are differentially regulated in BPH patients as compared to prostate cancer patients in a body fluid sample, e.g. blood sample, from a subject, e.g. patient, human or animal, wherein the set of miRNAs is selected from the miRNAs listed in Figure 3 or Figure 8.

Further, the set of at least two polynucleotides or the set of at least 2 primer pairs are for detecting a set comprising at least two miRNAs that are differentially regulated in BPH patients as compared to prostate cancer patients.in a body fluid sample, e.g. blood sample, from a subject, e.g. patient, human or animal, wherein the set of miRNAs is selected from the sets of miRNAs listed in Figure 5 or Figure 11.

It is preferred that the set of at least two primer pairs for determining the expression level of a set of miRNAs that are differentially regulated in BPH patients as compared to prostate cancer patients. are primer pairs that are specific for at least two miRNAs selected from the miRNAs listed in Figure 3 or Figure 8.

It is preferred that the set of at least two primer pairs for determining the expression level of a set of miRNAs that are differentially regulated in BPH patients as compared to prostate cancer patients. are primer pairs that are specific for at least one set of miRNAs listed in Figure 5 or Figure 11.

It is also preferred that said means for diagnosing of BPH comprise, of a set of beads comprising a at least two polynucleotides according to the second aspect of the present invention. It is especially preferred that the beads are employed within a flow cytometer setup for diagnosing of BPH, e.g. in a LUMINEX system (*www.luminexcorp.com).*

In a third aspect, the invention relates to the use of a kit for diagnosing of BPH in a subject as claimed in claim 7. Also disclosed is a kit for diagnosing of BPH in a method according to the first aspect of the invention comprising
(i) means for determining the miRNA expression profile of a set of at least two miRNAs that comprises hsa-miR-675 and hsa-miR-221,-comprising :
   (a) a set of at least two polynucleotides for detecting a set of at least two miRNAs comprising hsa-miR-675 and hsa-miR-221 or
   (b) a set of at least two primer pairs for detecting a set of at least two miRNAs comprising hsa-miR-675 and hsa-miR-221
      , and
(ii) at least one reference.

It is preferred that the sample is a blood cell sample or a leukocyte containing sample, particularly preferred it is a whole blood, PBMC sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

Further disclosed but not encompassed by the third aspect of the present invention, the set of miRNAs comprises miRNAs that are differentially regulated in BPH patients as compared to healthy controls.

According to the third aspect of the present invention, the set of miRNAs comprises miRNAs that are differentially regulated in BPH patients as compared to prostate cancer patients.

Said means may comprise of at least two polynucleotides according to the second aspect of the present invention, a set of at least 2 primer pairs; primers suitable to perform reverse transcriptase reaction and/or real time polymerase chain reaction such as quantitative polymerase chain reaction; and/or means for conducting next generation sequencing.

In fourth aspect, the invention relates to the use of a set of at least two miRNAs comprising hsa-miR-675 and hsa-miR-221-isolated from a blood cell sample from a subject for diagnosing of BPH according to the first aspect of the invention.

It is preferred that the sample is a blood cell sample or a leukocyte containing sample, particularly preferred it is a whole blood, PBMC, , more particularly preferred it is a whole blood sample.

It is preferred that the subject is a mammal including both a human and another mammal, e.g. an animal such as a mouse, a rat, a rabbit, or a monkey. It is particularly preferred that the subject is a human.

Further described, but not encompassed by the fourth aspect of the present invention, the set of miRNAs comprises miRNAs that are differentially regulated in BPH patients as compared to healthy controls.

Further described, but not encompassed by the fourth aspect of the present invention , the set comprising at least two miRNAs that are differentially regulated in BPH patients as compared to healthy controls is selected from the set of miRNAs listed in Figure 2 or Figure 7.

Further described, but not encompassed by the fourth aspect of the present invention, the set comprising at least two miRNAs that are differentially regulated in BPH patients as compared to healthy controls is selected from the set of miRNAs listed in Figure 4 or Figure 10.

Further described, but not encompassed by the fourth aspect of the present invention, the set comprising at least two miRNAs that are differentially regulated in BPH patients as compared to healthy controls comprises at least one set of miRNAs listed in Figure 4 or Figure 10.

According to the fourth aspect of the present invention, the set of miRNAs comprises miRNAs that are differentially regulated in BPH patients as compared to prostate cancer patients.

Preferably, the set comprising at least two miRNAs that are differentially regulated in BPH patients as compared to prostate cancer patients is selected from the set of miRNAs listed in Figure 3 or Figure 8.

It is preferred that the set comprising at least two miRNAs that are differentially regulated in BPH patients as compared to prostate cancer patients is selected from the set of miRNAs listed in Figure 5 or Figure 11.

It is preferred that the set comprising at least two miRNAs that are differentially regulated in BPH patients as compared to prostate cancer patients comprises at least one set of miRNAs listed in Figure 5 or Figure 11.

According to the fourth aspect, the invention relates to the use of a set of miRNAs according to the eighth aspect of the invention for diagnosing of BPH in a subject.

Further disclosure relates to diagnosing of prostate cancer in a in a body fluid sample, preferably in a blood sample, from a subject

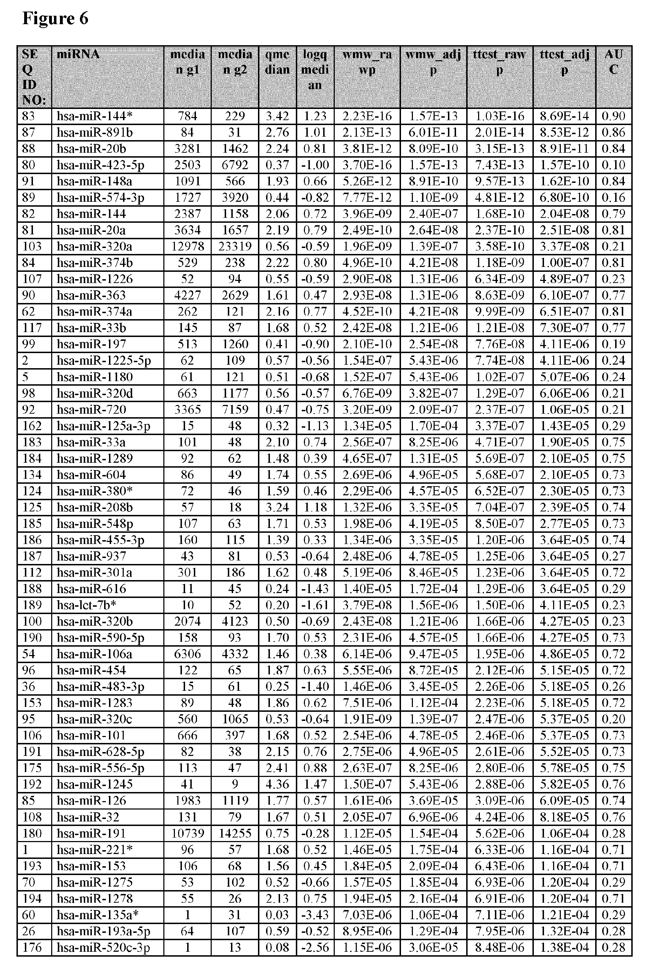

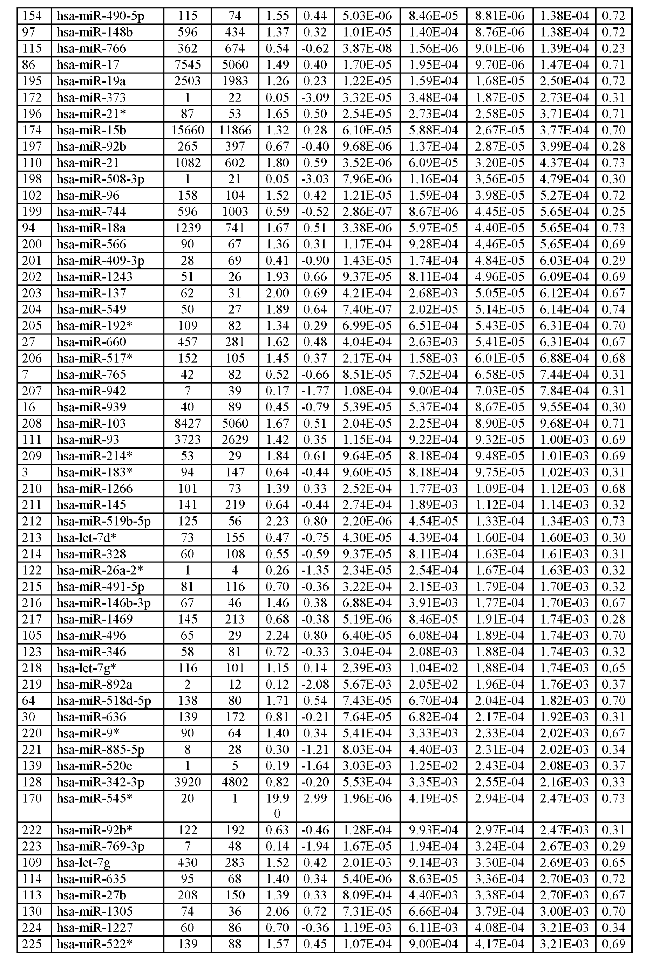

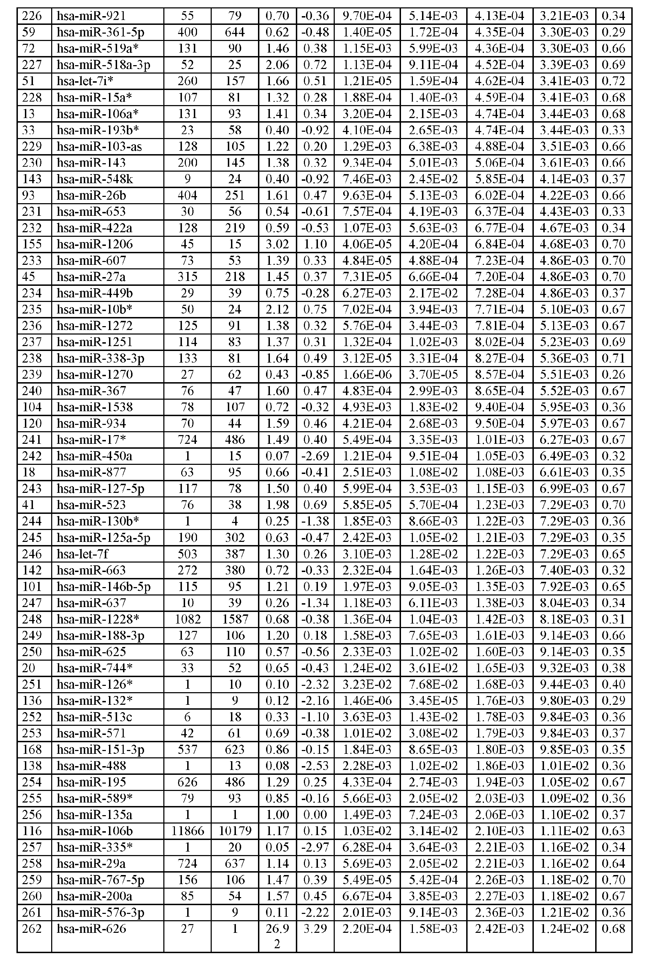

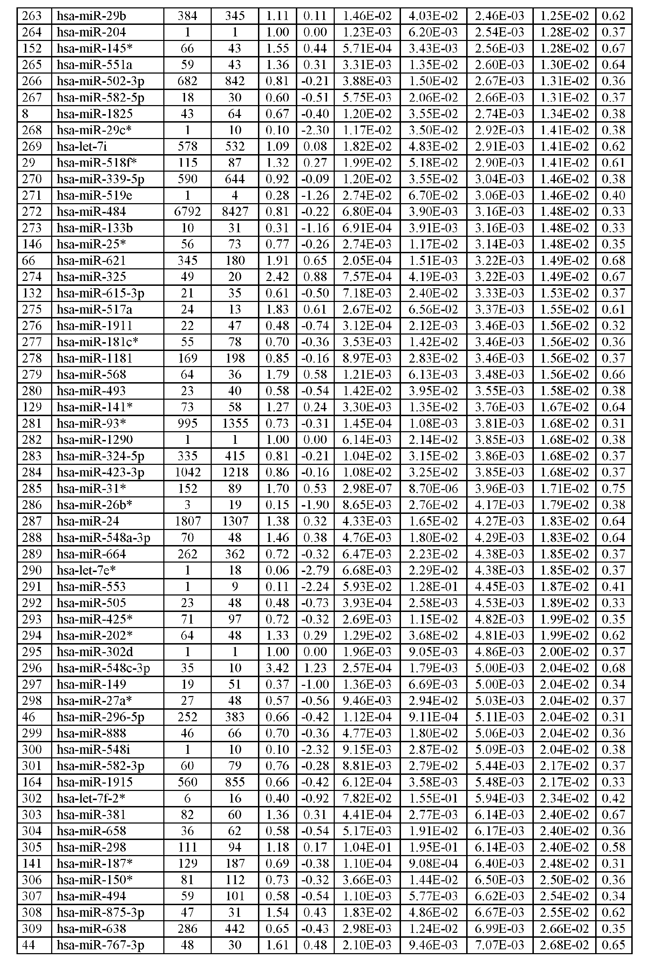

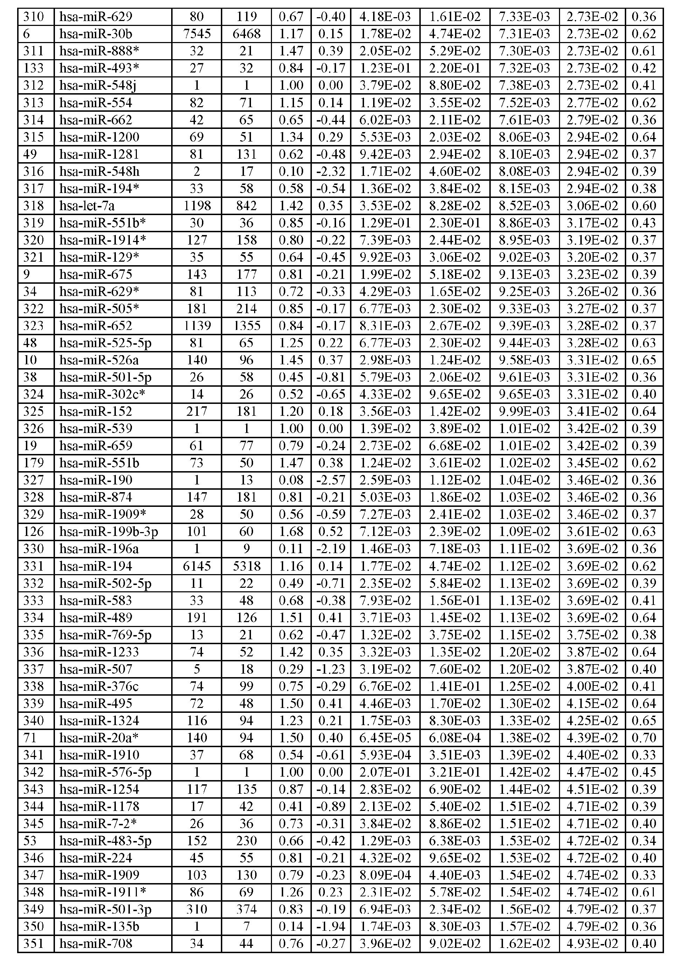

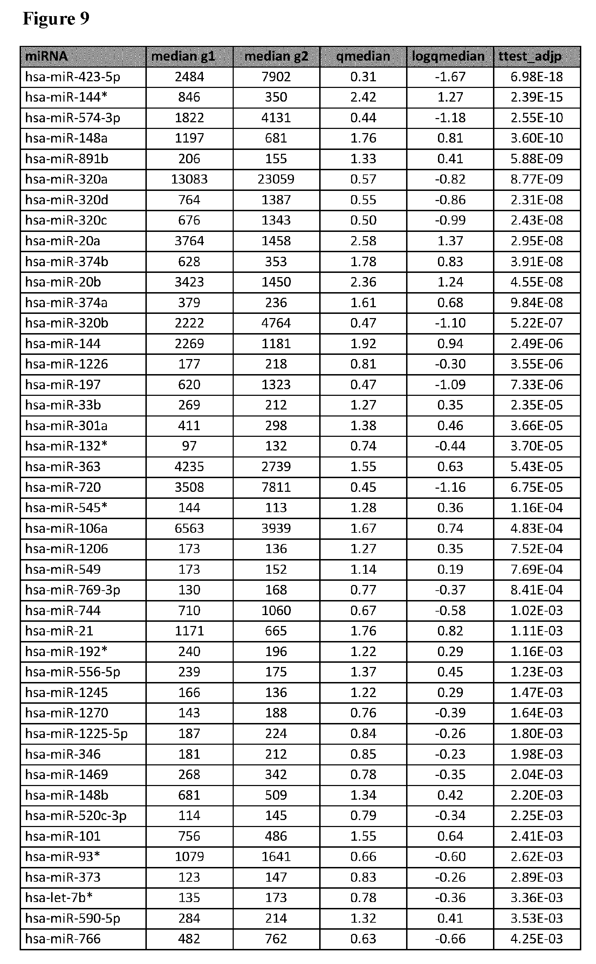

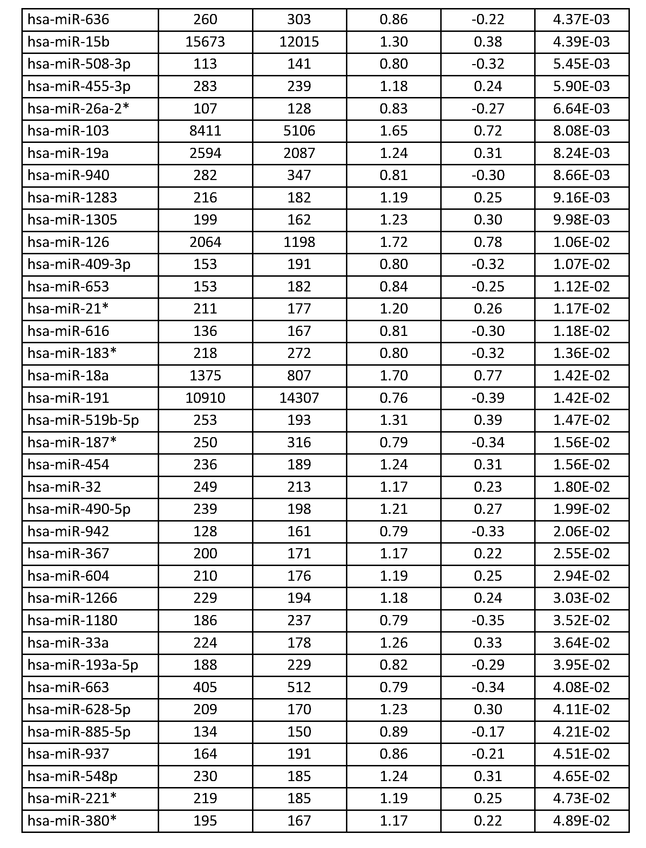

Surprisingly, the inventors found out that miRNAs are differentially regulated in samples from prostate cancer patients as compared to health controls. A complete overview of all miRNAs that are found to be differentially is provided in the table shown in Figure 6 or Figure 9. In Figure 6 in total, 279 miRNAs were found to be significantly deregulated (t-test significance <0.05) in blood cells of prostate cancer patients as compared to the healthy controls.

Said further disclosure relates to a method diagnosing and/or prognosing of prostate cancer comprising the steps of:
(i) determining an expression profile of a set comprising at least two miRNAs representative for prostate cancer in a body fluid sample from a subject, and
(ii) comparing said expression profile to a reference, wherein the comparison of said expression profile to said reference allows for the diagnosis and/or prognosis of prostate cancer,
wherein the set of miRNAs comprises miRNAs that are differentially regulated in blood samples of prostate cancer subjects as compared to healthy controls and wherein the miRNAs are selected from the tables in Figure 6 or Figure 9.

It is preferred that the body fluid sample according to the tenth aspect of the invention is a blood sample, preferably a blood cell sample or a leukocyte containing sample, particularly preferred it is a whole blood, PBMC, serum or plasma sample, more particularly preferred it is a whole blood sample.

Another further disclosure which is not encompassed by the present invention relates to a set comprising polynucleotides for detecting a set comprising at least two miRNAs for diagnosing and/or prognosing of prostate cancer in a body fluid sample from a subject, wherein the set of miRNAs comprises at least one miRNA listed in Figure 6 or Figure 9 or a set or a combination of sets of miRNAs listed in Figure 12.

Another further disclosure which is not encompassed by the present invention relates to the use of the aforementioned set of polynucleotides for diagnosing and/or prognosing prostate cancer in a subject.

Another further disclosure which is not encompassed by the present invention relates to a set of primer pairs for determining the expression level of a set of miRNAs in a body fluid sample of a subject suffering or suspected of suffering from prostate cancer, wherein the set of miRNAs comprises at least one miRNA listed in Figure 6 or Figure 9.

Another further disclosure which is not encompassed by the present invention relates to the use of set of the aforementioned primer pairs for diagnosing and/or prognosing prostate cancer in a subject.

Another further disclosure provides means for diagnosing and/or prognosing of prostate cancer in a body fluid sample of a subject comprising :
(i) a set of at least two polynucleotides, or
(ii) a set of primer pairs.

Another further disclosure provides a kit for diagnosing of prostate cancer comprising
(i) means for determining an expression profile of a set comprising at least two miRNAs representative for prostate cancer in a body fluid sample from a subject, and
(ii) at least one reference.

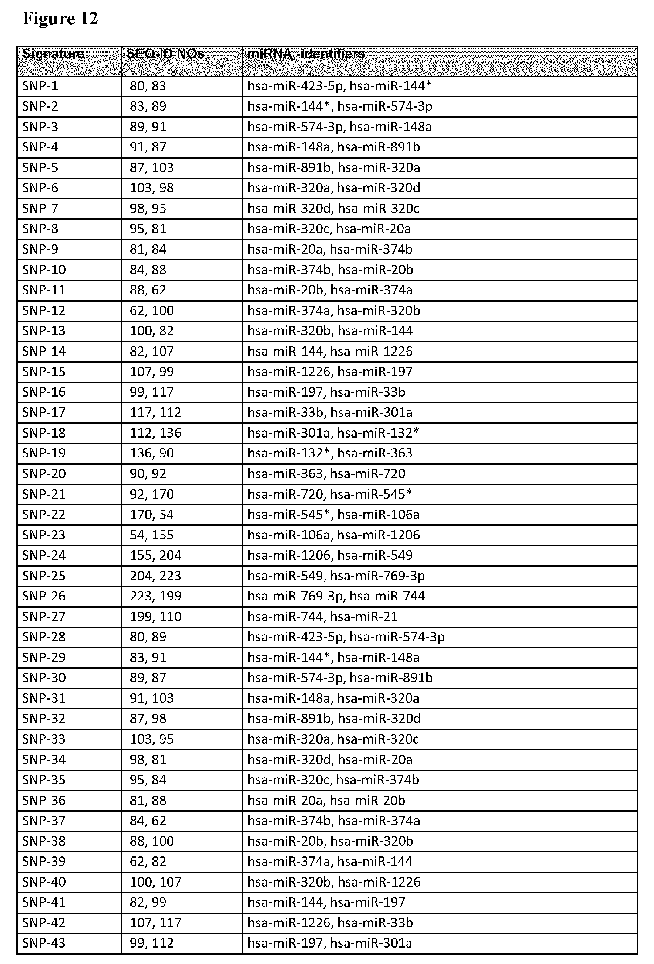

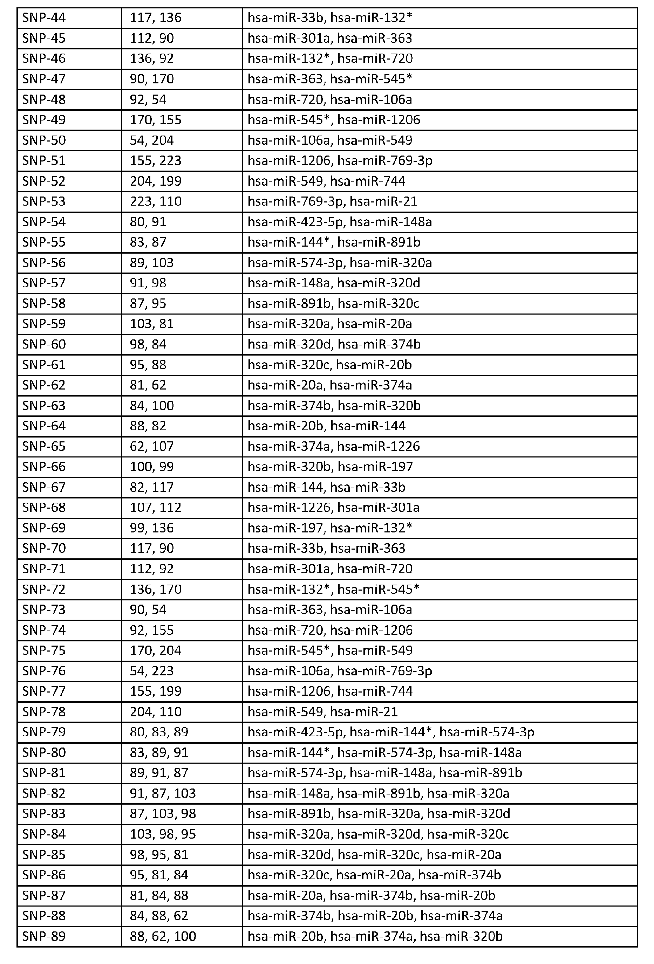

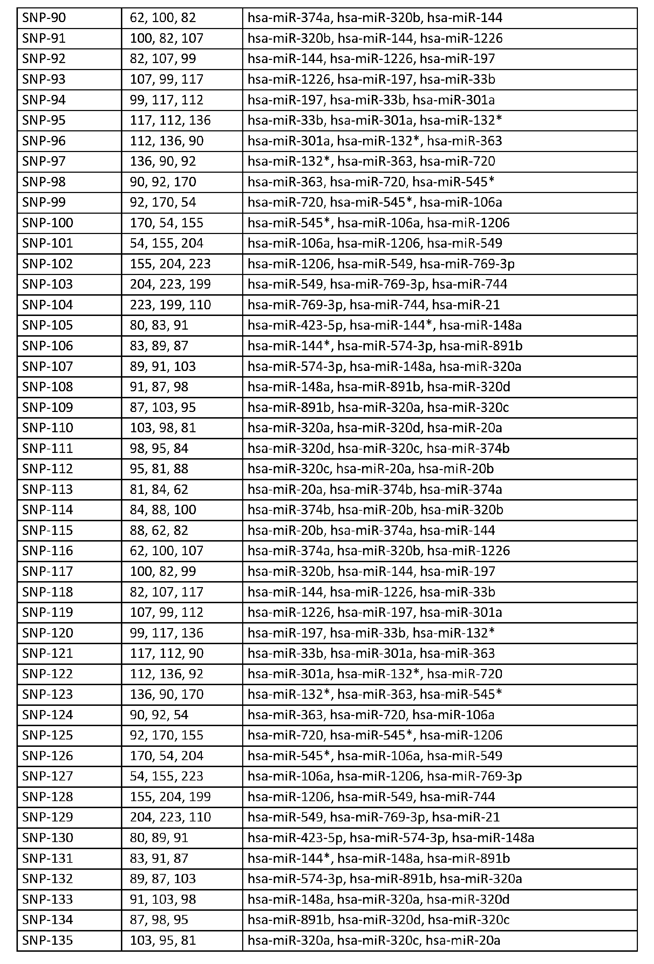

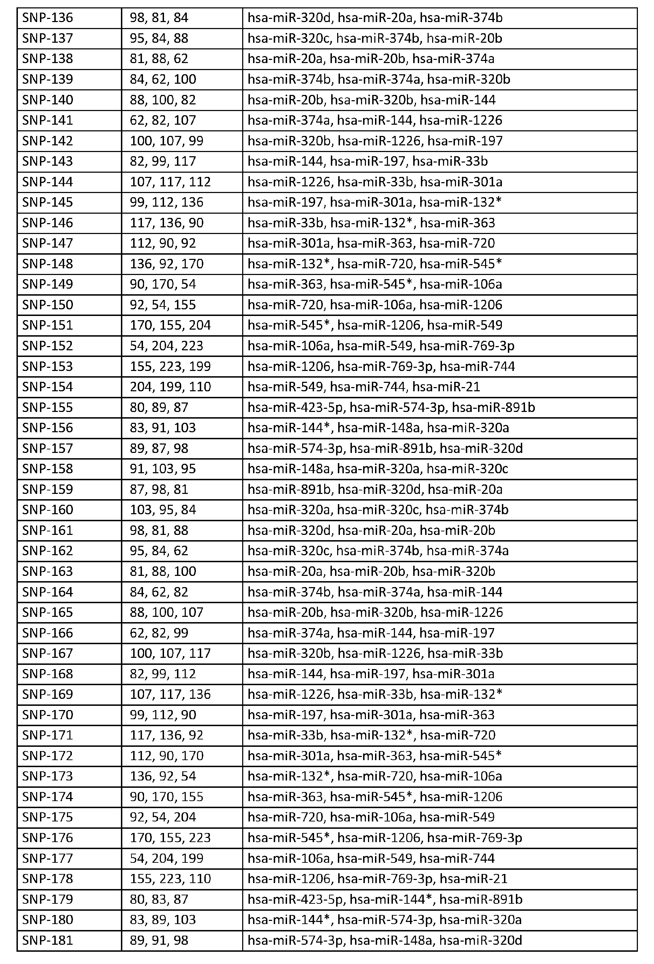

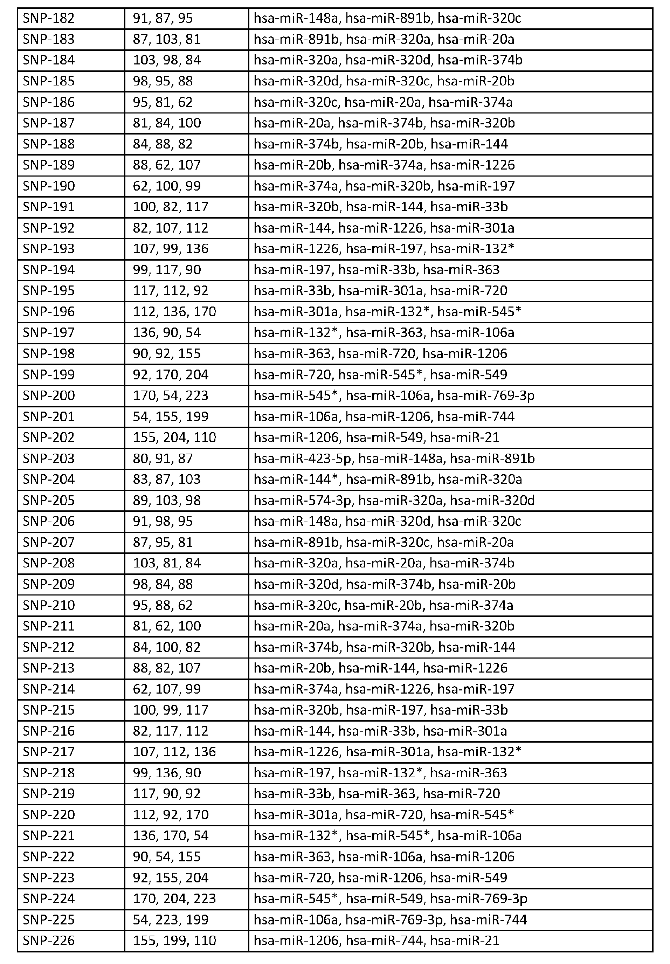

Another further disclosure which is not encompassed by the present invention provides a set of miRNAs in a body fluid sample isolated from a subject for diagnosing and/or prognosing of prostate cancer, wherein the set of miRNAs comprises at least one miRNA listed in Figure 6 or Figure 9 or a set or a combination of sets of miRNAs listed in Figure 12.

Another further disclosure which is not encompassed by the present invention provides a use of the aforementioned set of miRNAs for diagnosing and/or prognosing of prostate cancer in a subject,

### BRIEF DESCRIPTION OF THE DRAWINGS

Figure 1:
   Overview of miRNA sequences suitable for diagnosing prostate diseases.
Figure 2:
   Overview of miRNAs that are found to be differentially regulated between healthy controls and subjects suffering from BPH. Experimental details: SEQ ID NO: sequence identification number, miRNA: identifier of the miRNA according to miRBase, median g1: median intensity obtained from microarray analysis for healthy controls in counts/sec, median g2: median intensity obtained from microarray analysis for individuals with BPH in counts/sec, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 3:
   Overview of miRNAs that are found to be differentially regulated between prostate cancer subjects and subjects suffering from BPH. Experimental details: SEQ ID NO: sequence identification number, miRNA: identifier of the miRNA according to miRBase, median g1: median intensity obtained from microarray analysis for prostate cancer subjects in counts/sec, median g2: median intensity obtained from microarray analysis for individuals with BPH in counts/sec, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 4:
   Sets of miRNAs (miRNA signatures SNB-1 to SNB-753) that allow for effective diagnosis and/or prognosis of BPH when differentiating BPH and healthy controls. Experimental details: SEQ ID NO: sequence identification number, miRNA: identifier of the miRNA according to miRBase, Acc = accuracy, Spec = specificity, Sens = sensitivity
Figure 5:
   Sets of miRNAs (miRNA signatures SPB-1 to SPB-247) that allow for effective diagnosis and/or prognosis of BPH when differentiating prostate cancer and BPH. Experimental details: SEQ ID NO: sequence identification number, miRNA: identifier of the miRNA according to miRBase, Acc = accuracy, Spec = specificity, Sens = sensitivity.
Figure 6:
   Overview of miRNAs that are found to be differentially regulated between healthy controls and subjects suffering from prostate cancer. Experimental details: SEQ ID NO: sequence identification number, miRNA: identifier of the miRNA according to miRBase, median g1: median intensity obtained from microarray analysis for healthy controls in counts/sec, median g2: median intensity obtained from microarray analysis for individuals with prostate cancer in counts/sec, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 7:
   Overview of further miRNAs that are found to be differentially regulated between 68 healthy controls and 33 subjects suffering from BPH. Experimental details: SEQ ID NO: sequence identification number, miRNA: identifier of the miRNA according to miRBase, median g1: median intensity obtained from microarray analysis for healthy controls in counts/sec, median g2: median intensity obtained from microarray analysis for individuals with BPH in counts/sec, qmedian: ratio of median g1/median g2, logqmedian: log2 of qmedian, ttest_adjp: p-value obtained when applying t-test , adjusted p-value in order to reduce false discovery rate by Benjamini-Hochberg adjustment.
Figure 8:
   Overview of further miRNAs that are found to be differentially regulated between 64 prostate cancer subjects and 33 subjects suffering from BPH. Experimental details: SEQ ID NO: sequence identification number, miRNA: identifier of the miRNA according to miRBase, median g1: median intensity obtained from microarray analysis for individuals with BPH in counts/sec, median g2: median intensity obtained from microarray analysis for individuals with prostate cancer in counts/sec, qmedian: ratio of median g1/median g2, logqmedian: log2 of qmedian, ttest_adjp: p-value obtained when applying t-test , adjusted p-value in order to reduce false discovery rate by Benjamini-Hochberg adjustment.
Figure 9:
   Overview of further miRNAs that are found to be differentially regulated between 68 healthy controls and 64 subjects suffering from prostate cancer. Experimental details: SEQ ID NO: sequence identification number, miRNA: identifier of the miRNA according to miRBase, median g1: median intensity obtained from microarray analysis for healthy controls in counts/sec, median g2: median intensity obtained from microarray analysis for individuals with prostate cancer in counts/sec, qmedian: ratio of median g1/median g2, logqmedian: log2 of qmedian, ttest_adjp: p-value obtained when applying t-test , adjusted p-value in order to reduce false discovery rate by Benjamini-Hochberg adjustment.
Figure 10:
   Further sets of miRNAs (miRNA signatures SNB-754 to SNB-968) that allow for effective diagnosis and/or prognosis of BPH when differentiating BPH and healthy controls. Experimental details: SEQ ID NO: sequence identification number, miRNA: identifier of the miRNA according to miRBase.
Figure 11:
   Further sets of miRNAs (miRNA signatures SPB-248 to SPB-356) that allow for effective diagnosis and/or prognosis of BPH when differentiating prostate cancer and BPH. Experimental details: SEQ ID NO: sequence identification number, miRNA: identifier of the miRNA according to miRBase.
Figure 12:
   Sets of miRNAs (miRNA signatures SNP-1 to SNP-226) that allow for effective diagnosis and/or prognosis of prostate cancer when differentiating prostate cancer and healthy controls. Experimental details: SEQ ID NO: sequence identification number, miRNA: identifier of the miRNA according to miRBase.

### EXAMPLES

The Examples are designed in order to further illustrate the present invention and serve a better understanding. They are not to be construed as limiting the scope of the invention in any way.

### Materials and Methods

### miRNA extraction and microarray screening

Blood of patients has been extracted as previously described [1]. In brief, 2.5 to 5 ml blood was extracted in PAXgene Blood RNA tubes (BD, Franklin Lakes, New Jersey USA) and centrifuged at 5000 x g for 10 min at room temperature. The miRNeasy kit (Qiagen GmbH, Hilden) was used to isolate total RNA including miRNA from the resuspended pellet according to manufacturer's instructions. The eluted RNA was stored at -70°C.

All samples were shipped overnight on dry ice and analyzed with the Geniom RT Analyzer (febit biomed GmbH, Heidelberg, Germany) at the in-house genomic service department using the Geniom Biochip miRNA Homo sapiens. Each array contains 7 replicates of about 863 miRNAs and miRNA star sequences as annotated in the Sanger miRBase releases 12.0, 13.0 and 14.0. On-chip sample labeling with biotin was carried out by microfluidic-based primer extension labeling of miRNAs (MPEA [2]). Following hybridization for 16 hours at 42°C, the biochip was washed and a program for signal enhancement was carried out. All steps from sample loading to miRNA detection were processed without any manual intervention and inside the machine. The detection pictures were evaluated using the Geniom Wizard Software. For each feature, the median signal intensity was calculated. Following a background correction step, the median of the 7 replicates of each miRNA was computed. To normalize the data across different arrays, quantile normalization [3] was applied and all further analyses were carried out using the normalized and background subtracted intensity values. Since the miRBase has been upgraded twice in the past year from version 12.0 to 14, we used for the final data analysis the 863 miRNAs that were consistently present in all three versions.

### Statistical analysis

To estimate the value of single miRNAs, t-tests (unpaired, two-tailed) were carried out. The resulting p-values have been adjusted for multiple testing by Benjamini-Hochberg adjustment [4, 5]. In addition to this single biomarker analysis, we performed supervised classification of samples by using Support Vector Machines (SVM [6]) as implemented in the R e1071 package [7]. As parameters, we evaluated different kernel methods including linear, polynomial (degree 2 to 5), sigmoid and radial basis function kernels. The cost parameter was sampled from 0.01 to 10 in decimal powers. As subset selection technique, a filter approach based on t-test was carried out. In each iteration, the *s* miRNAs with lowest p-values were computed on the training set in each fold of a standard 10-fold cross validation, where *s* was sampled in regular intervals between 2 and 300. The respective subset was used to train the SVM and to carry out the prediction of the test samples in the cross validation. To compute probabilities for classes instead of class labels, a regression approach based on the output of the support vectors has been applied. To test for overtraining, non-parametric permutation tests have been applied. All computations were carried out using R [7], a freely available language for statistical tasks.

### REFERENCES

1. Keller A, Leidinger P, Borries A, Wendschlag A, Wucherpfennig F, Scheffler M, Huwer H, Lenhof HP, Meese E: miRNAs in lung cancer - studying complex fingerprints in patient's blood cells by microarray experiments. BMC Cancer 2009, 9:353.
2. Vorwerk S, Ganter K, Cheng Y, Hoheisel J, Stahler PF, Beier M: Microfluidic-based enzymatic on-chip labeling of miRNAs. N Biotechnol 2008, 25(2-3):142-149.
3. Bolstad BM, Irizarry RA, Astrand M, Speed TP: A comparison of normalization methods for high density oligonucleotide array data based on variance and bias. Bioinformatics 2003, 19(2):185-193.
4. Benjamini Y, Drai D, Elmer G, Kafkafi N, Golani I: Controlling the false discovery rate in behavior genetics research. Behav Brain Res 2001, 125(1-2):279-284.
5. Hochberg Y: A sharper bonferroni procedure for multiple tests of significance. Biometrica 1988, 75:185-193.
6. Vapnik V: The nature of statistical learning theory., 2nd edition edn. New York: Spinger; 2000.
7. Team R: R: A Language and Environment for Statistical Computing. In. Vienna: R Foundation for Statistical Computing; 2008.

## Claims

1. *A method of diagnosing BPH, comprising the steps*
*(a) determining an expression profile of a set of at least two miRNAs in a blood cell sample from a patient, and*
*(b) comparing said expression profile to a reference*
*wherein the set of miRNAs comprises hsa-miR-675 (SEQ ID NO: 9) and hsa-miR-221 (SEQ ID NO: 32) that are differentially regulated in blood cell samples from BPH patients as compared to prostate cancer patients and wherein the comparison of said determined expression profile to said reference allows to discriminate between BPH and prostate cancer.*

2. *The method according to claim 1, wherein blood cell sample is a blood cellular fraction including red blood cells, white blood cells, platelets.*

3. *The method according to any of the claims 1 or 2, wherein the set of miRNAs comprises miRNAs that are differentially regulated in blood samples from BPH patients as compared to prostate cancer patients and wherein the miRNAs are selected from Figure 3 or Figure 8.*

4. *The method according to any of the claims 1, 2 or 3, wherein the expression profile is determined from the total RNA isolated from the pellet obtained after centrifugation of a whole blood sample.*

5. *The method according to any one of claims 1 to 4, wherein the expression profile is determined by nucleic acid hybridization, nucleic acid amplification, polymerase extension, sequencing, mass spectroscopy, flow cytometry or any combinations thereof.*

6. *Use of a set of polynucleotides for detecting a set of at least two miRNAs comprising hsa-miR-675 (SEQ ID NO: 9) and hsa-miR-221 (SEQ ID NO: 32) in the method according to any of the claims 1 to 5.*

7. *Use of a kit in the method according to any of the claims 1 to 5, comprising:*
*(a) means for determining the miRNA expression profile of a set of at least two miRNAs that comprises hsa-miR-675 (SEQ ID NO: 9) and hsa-miR-221 (SEQ ID NO: 32) comprising :*
*i. a set of at least two polynucleotides for detecting a set of at least two miRNAs comprising hsa-miR-675 (SEQ ID NO: 9) and hsa-miR-221 (SEQ ID NO:* 32) *or*
*ii. a set of at least two primer pairs for detecting a set of at least two miRNAs comprising hsa-miR-675 (SEQ ID NO: 9) and hsa-miR-221 (SEQ ID NO: 32)*
*and*
*(b) at least one reference*

8. *The use of claim 7, wherein the reference is a reference expression profile that is obtained from a least 2 subjects with two known clinical conditions which are BPH and prostate cancer.*

9. *Use of set of at least two miRNAs comprising hsa-miR-675 (SEQ ID NO: 9) and hsa-miR-221 (SEQ ID NO: 32) isolated from a blood cell sample from a subject in the method according to any of the claims 1 to 5.*

## Patentansprüche

1. Verfahren zur Diagnose von BPH, umfassend die Schritte:
(a) Ermitteln eines Expressionsprofils eines Sets von mindestens zwei miRNAs in einer Blutzellprobe eines Patienten, und
(b) Vergleichen des Expressionsprofils mit einer Referenz,
wobei das Set von miRNAs hsa-miR-675 (SEQ ID NO: 9) und hsa-miR-221 (SEQ ID NO: 32) umfasst, die in Blutzellproben von BPH-Patienten im Vergleich zu Prostatakrebspatienten differentiell reguliert sind, und
wobei der Vergleich des ermittelten Expressionsprofils mit der Referenz die Unterscheidung zwischen BPH und Prostatakrebs erlaubt.

2. Verfahren gemäß Anspruch 1, wobei die Blutzellprobe eine zelluläre Fraktion des Blutes ist, die rote Blutzellen, weiße Blutzellen, Blutplättchen umfasst.

3. Verfahren gemäß einem der Ansprüche 1 oder 2, wobei das Set von miRNAs miRNAs umfasst, die in Blutproben von BPH-Patienten im Vergleich zu Prostatakrebspatienten differentiell reguliert sind und wobei die miRNAs aus der Figur 3 oder Figur 8 ausgewählt sind.

4. Verfahren gemäß einem der Ansprüche 1, 2 oder 3, wobei das Expressionsprofil anhand der Gesamt-RNA ermittelt wird, die aus dem Pellet isoliert wurde, welches nach der Zentrifugation einer Vollblutprobe erhalten wurde.

5. Verfahren gemäß einem der Ansprüche 1 bis 4, wobei das Expressionsprofil mittels Nukleinsäurehybridisierung, Nukleinsäureamplifikation, Polymeraseverlängerung, Sequenzierung, Massenspektrometrie, Durchflusszytometrie oder mittels jeder Kombination davon ermittelt wird.

6. Verwendung eines Sets von Polynukleotiden zum Nachweis eines Sets von mindestens zwei miRNAs, die hsa-miR-675 (SEQ ID NO: 9) und hsa-miR-221 (SEQ ID NO: 32) umfassen, in dem Verfahren gemäß einem der Ansprüche 1 bis 5.

7. Verwendung eines Kits in dem Verfahren gemäß einem der Ansprüche 1 bis 5, umfassend:
(a) Mittel zum Ermitteln des miRNA-Expressionsprofils eines Sets von mindestens zwei miRNAs, die hsa-miR-675 (SEQ ID NO: 9) und hsa-miR-221 (SEQ ID NO: 32) umfassen, umfassend:
i. ein Set von mindestens zwei Polynukleotiden zum Detektieren eines Sets von mindestens zwei miRNAs, die hsa-miR-675 (SEQ ID NO: 9) und hsa-miR-221 (SEQ ID NO: 32) umfassen, oder
ii. ein Set von mindestens zwei Primerpaaren zum Detektieren eines Sets von mindestens zwei miRNAs, die hsa-miR-675 (SEQ ID NO: 9) und hsa-miR-221 (SEQ ID NO: 32) umfassen,
und
(b) mindestens eine Referenz.

8. Verwendung nach Anspruch 7, wobei die Referenz ein Referenzexpressionsprofil ist, das von mindestens zwei Subjekten mit zwei bekannten klinischen Zuständen erhalten wurde, die BPH und Prostatakrebs sind.

9. Verwendung von mindestens zwei miRNAs, die hsa-miR-675 (SEQ ID NO: 9) und hsa-miR-221 (SEQ ID NO: 32) umfassen und die aus einer Blutzellprobe von einem Subjekt isoliert wurden, in dem Verfahren gemäß einem der Ansprüche 1 bis 5.

## Revendications

1. Procédé de diagnostic de l'hyperplasie prostatique bénigne, comprenant les étapes
(a) de détermination d'un profil d'expression d'un ensemble d'au moins deux ARNmi dans un échantillon de cellules sanguines d'un patient, et
(b) de comparaison dudit profil d'expression à une référence où l'ensemble des ARNmi comprend hsa-miR-675 (SEQ ID NO : 9) et hsa- miR-221 (SEQ ID NO: 32) qui sont régulés de façon différentielle dans des échantillons de cellules sanguines de patients atteints d'hyperplasie prostatique bénigne comparativement aux patients atteints du cancer de la prostate et où la comparaison dudit profil d'expression déterminé avec ladite référence permet de distinguer entre l'hyperplasie prostatique bénigne et le cancer de la prostate.

2. Procédé selon la revendication 1, où l'échantillon de cellules sanguines est une fraction cellulaire du sang comprenant des globules rouges, des globules blancs, des plaquettes.

3. Procédé selon l'une quelconque des revendications 1 ou 2, où l'ensemble d'ARNmi comprend des ARNmi qui sont régulés de façon différentielle dans des échantillons sanguins provenant de patients atteints d'hyperplasie prostatique bénigne comparativement aux patients atteints du cancer de la prostate et où les ARNmi sont choisis parmi la Figure 3 ou la Figure 8.

4. Procédé selon l'une quelconque des revendications 1, 2 ou 3, où le profil d'expression est déterminé à partir de l'ARN total isolé de la pastille obtenue après centrifugation d'un échantillon de sang entier.

5. Procédé selon l'une quelconque des revendications 1 à 4, où le profil d'expression est déterminé par hybridation d'acide nucléique, amplification d'acide nucléique, extension polymérase, séquençage, spectroscopie de masse, cytométrie de flux ou toute combinaison de ceux-ci.

6. Utilisation d'un ensemble de polynucléotides pour détecter un ensemble d'au moins deux ARNmi comprenant hsa-miR-675 (SEQ ID NO : 9) et hsa- miR-221 (SEQ ID NO: 32) dans le procédé selon l'une quelconque des revendications 1 à 5.

7. Utilisation d'un kit dans le procédé selon l'une quelconque des revendications 1 à 5, comprenant :
(a) un moyen pour déterminer le profil d'expression ARNmi d'un ensemble d'au moins deux ARNmi comprenant hsa-miR-675 (SEQ ID NO : 9) et hsa-miR-221 (SEQ ID NO: 32) comprenant :
i. un ensemble d'au moins deux polynucléotides pour détecter un ensemble d'au moins deux ARNmi comprenant hsa-miR-675 (SEQ ID NO : 9) et hsa- miR-221 (SEQ ID NO: 32) ou
ii. un ensemble d'au moins deux paires d'amorces pour détecter un ensemble d'au moins deux ARNmi comprenant hsa-miR-675 (SEQ ID NO : 9) et hsa- miR-221 (SEQ ID NO: 32)
et
(b) au moins une référence

8. Utilisation selon la revendication 7, où la référence est un profil d'expression de référence qui est obtenu à partir d'un moins 2 sujets présentant deux conditions cliniques connues qui sont l'hyperplasie prostatique bénigne et le cancer de la prostate.

9. Utilisation d'un ensemble d'au moins deux ARNmi comprenant hsa-miR-675 (SEQ ID NO : 9) et hsa- miR-221 (SEQ ID NO: 32) isolée d'un échantillon de cellules sanguines à partir d'un sujet dans le procédé selon l'une quelconque des revendications 1 à 5.


### SEQUENCE LISTING

<110> febit holding GmbH
<120> Complex sets of miRNAs as non-invasive biomarkers for prostate diseases
<130> FP-004
<160> 353
<170> PatentIn version 3.5
<210> 1
   <211> 22
   <212> RNA
   <213> Homo sapiens
<400> 1
   accuggcaua caauguagau uu 22
<210> 2
   <211> 22
   <212> RNA
   <213> Homo sapiens
<400> 2
   guggguacgg cccagugggg gg 22
<210> 3
   <211> 22
   <212> RNA
   <213> Homo sapiens
<400> 3
   gugaauuacc gaagggccau aa 22
<210> 4
   <211> 22
   <212> RNA
   <213> Homo sapiens
<400> 4
   uggucuagga uuguuggagg ag 22
<210> 5
   <211> 22
   <212> RNA
   <213> Homo sapiens
<400> 5
   uuuccggcuc gcgugggugu gu 22
<210> 6
   <211> 22
   <212> RNA
   <213> Homo sapiens
<400> 6
   uguaaacauc cuacacucag cu 22
<210> 7
   <211> 21
   <212> RNA
   <213> Homo sapiens
<400> 7
   uggaggagaa ggaaggugau g 21
<210> 8
   <211> 18
   <212> RNA
   <213> Homo sapiens
<400> 8
   uccagugccc uccucucc 18
<210> 9
   <211> 23
   <212> RNA
   <213> Homo sapiens
<400> 9
   uggugcggag agggcccaca gug 23
<210> 10
   <211> 22
   <212> RNA
   <213> Homo sapiens
<400> 10
   cucuagaggg aagcacuuuc ug 22
<210> 11
   <211> 21
   <212> RNA
   <213> Homo sapiens
<400> 11
   uccucuucuc ccuccuccca g 21
<210> 12
   <211> 21
   <212> RNA
   <213> Homo sapiens
<400> 12
   gaagugugcc gugguguguc u 21
<210> 13
   <211> 22
   <212> RNA
   <213> Homo sapiens
<400> 13
   cugcaaugua agcacuucuu ac 22
<210> 14
   <211> 18
   <212> RNA
   <213> Homo sapiens
<400> 14
   cgggcguggu gguggggg 18
<210> 15
   <211> 19
   <212> RNA
   <213> Homo sapiens
<400> 15
   gaugaugcug cugaugcug 19
<210> 16
   <211> 24
   <212> RNA
   <213> Homo sapiens
<400> 16
   uggggagcug aggcucuggg ggug 24
<210> 17
   <211> 22
   <212> RNA
   <213> Homo sapiens
<400> 17
   caauuuagug ugugugauau uu 22
<210> 18
   <211> 20
   <212> RNA
   <213> Homo sapiens
<400> 18
   guagaggaga uggcgcaggg 20
<210> 19
   <211> 22
   <212> RNA
   <213> Homo sapiens
<400> 19
   cuugguucag ggagggucccca 22
<210> 20
   <211> 22
   <212> RNA
   <213> Homo sapiens
<400> 20
   cuguugccac uaaccucaac cu 22
<210> 21
   <211> 22
   <212> RNA
   <213> Homo sapiens
<400> 21
   uucacauugu gcuacugucu gc 22
<210> 22
   <211> 23
   <212> RNA
   <213> Homo sapiens
<400> 22
   uaaggugcau cuagugcagu uag 23
<210> 23
   <211> 23
   <212> RNA
   <213> Homo sapiens
<400> 23
   ugagugugug ugugugagug ugu 23
<210> 24
   <211> 21
   <212> RNA
   <213> Homo sapiens
<400> 24
   ucuaguaaga guggcagucg a 21
<210> 25
   <211> 23
   <212> RNA
   <213> Homo sapiens
<400> 25
   uguaaacauc cuacacucuc age 23
<210> 26
   <211> 22
   <212> RNA
   <213> Homo sapiens
<400> 26
   ugggucuuug cgggcgagauga 22
<210> 27
   <211> 22
   <212> RNA
   <213> Homo sapiens
<400> 27
   uacccauugc auaucggagu ug 22
<210> 28
   <211> 20
   <212> RNA
   <213> Homo sapiens
<400> 28
   cggcucuggg ucugugggga 20
<210> 29
   <211> 22
   <212> RNA
   <213> Homo sapiens
<400> 29
   cucuagaggg aagcacuuuc uc 22
<210> 30
   <211> 23
   <212> RNA
   <213> Homo sapiens
<400> 30
   ugugcuugcu cgucccgccc gca 23
<210> 31
   <211> 23
   <212> RNA
   <213> Homo sapiens
<400> 31
   aacauucauu gcugucggug ggu 23
<210> 32
   <211> 23
   <212> RNA
   <213> Homo sapiens
<400> 32
   agcuacauug ucugcugggu uuc 23
<210> 33
   <211> 22
   <212> RNA
   <213> Homo sapiens
<400> 33
   cgggguuuug agggcgagau ga 22
<210> 34
   <211> 22
   <212> RNA
   <213> Homo sapiens
<400> 34
   guucucccaa cguaagccca gc 22
<210> 35
   <211> 22
   <212> RNA
   <213> Homo sapiens
<400> 35
   cacacacugc aauuacuuuu gc 22
<210> 36
   <211> 21
   <212> RNA
   <213> Homo sapiens
<400> 36
   ucacuccucu ccucccgucu u 21
<210> 37
   <211> 22
   <212> RNA
   <213> Homo sapiens
<400> 37
   aacccguaga uccgaucuug ug 22
<210> 38
   <211> 22
   <212> RNA
   <213> Homo sapiens
<400> 38
   aauccuuugu cccuggguga ga 22
<210> 39
   <211> 23
   <212> RNA
   <213> Homo sapiens
<400> 39
   aucgcugcgg uugcgagcgc ugu 23
<210> 40
   <211> 21
   <212> RNA
   <213> Homo sapiens
<400> 40
   ugcaggacca agaugagccc u 21
<210> 41
   <211> 23
   <212> RNA
   <213> Homo sapiens
<400> 41
   gaacgcgcuu cccuauagag ggu 23
<210> 42
   <211> 23
   <212> RNA
   <213> Homo sapiens
<400> 42
   aucaacagac auuaauuggg cgc 23
<210> 43
   <211> 22
   <212> RNA
   <213> Homo sapiens
<400> 43
   gcuacuucac aacaccaggg cc 22
<210> 44
   <211> 23
   <212> RNA
   <213> Homo sapiens
<400> 44
   ucugcucaua ccccaugguu ucu 23
<210> 45
   <211> 21
   <212> RNA
   <213> Homo sapiens
<400> 45
   uucacagugg cuaaguuccg c 21
<210> 46
   <211> 21
   <212> RNA
   <213> Homo sapiens
<400> 46
   agggcccccc cucaauccug u 21
<210> 47
   <211> 23
   <212> RNA
   <213> Homo sapiens
<400> 47
   agcugguguu gugaaucagg ccg 23
<210> 48
   <211> 21
   <212> RNA
   <213> Homo sapiens
<400> 48
   cuccagaggg augcacuuuc u 21
<210> 49
   <211> 17
   <212> RNA
   <213> Homo sapiens
<400> 49
   ucgccuccuc cucuccc 17
<210> 50
   <211> 22
   <212> RNA
   <213> Homo sapiens
<400> 50
   acucaaaacc cuucagugac uu 22
<210> 51
   <211> 22
   <212> RNA
   <213> Homo sapiens
<400> 51
   cugcgcaagc uacugccuug cu 22
<210> 52
   <211> 20
   <212> RNA
   <213> Homo sapiens
<400> 52
   acucaaacug ugggggcacu 20
<210> 53
   <211> 22
   <212> RNA
   <213> Homo sapiens
<400> 53
   aagacgggag gaaagaaggg ag 22
<210> 54
   <211> 23
   <212> RNA
   <213> Homo sapiens
<400> 54
   aaaagugcuu acagugcagg uag 23
<210> 55
   <211> 21
   <212> RNA
   <213> Homo sapiens
<400> 55
   caaaacguga ggcgcugcua u 21
<210> 56
   <211> 21
   <212> RNA
   <213> Homo sapiens
<400> 56
   cuucuugugc ucuaggauug u 21
<210> 57
   <211> 22
   <212> RNA
   <213> Homo sapiens
<400> 57
   caacaaauca cagucugcca ua 22
<210> 58
   <211> 25
   <212> RNA
   <213> Homo sapiens
<400> 58
   agggguggug uugggacagc uccgu 25
<210> 59
   <211> 22
   <212> RNA
   <213> Homo sapiens
<400> 59
   uuaucagaau cuccaggggu ac 22
<210> 60
   <211> 22
   <212> RNA
   <213> Homo sapiens
<400> 60
   uauagggauu ggagccgugg eg 22
<210> 61
   <211> 21
   <212> RNA
   <213> Homo sapiens
<400> 61
   ucuuguguuc ucuagaucag u 21
<210> 62
   <211> 22
   <212> RNA
   <213> Homo sapiens
<400> 62
   uuauaauaca accugauaag ug 22
<210> 63
   <211> 27
   <212> RNA
   <213> Homo sapiens
<400> 63
   cacuguaggu gauggugaga gugggca 27
<210> 64
   <211> 22
   <212> RNA
   <213> Homo sapiens
<400> 64
   cucuagaggg aagcacuuuc ug 22
<210> 65
   <211> 21
   <212> RNA
   <213> Homo sapiens
<400> 65
   cuccagaggg aaguacuuuc u 21
<210> 66
   <211> 21
   <212> RNA
   <213> Homo sapiens
<400> 66
   ggcuagcaac agcgcuuacc u 21
<210> 67
   <211> 22
   <212> RNA
   <213> Homo sapiens
<400> 67
   ccaaaacugc aguuacuuuu gc 22
<210> 68
   <211> 21
   <212> RNA
   <213> Homo sapiens
<400> 68
   cuuuuugcgg ucugggcuug c 21
<210> 69
   <211> 20
   <212> RNA
   <213> Homo sapiens
<400> 69
   cugcagggu uugcuuugag 20
<210> 70
   <211> 17
   <212> RNA
   <213> Homo sapiens
<400> 70
   gugggggaga ggcuguc 17
<210> 71
   <211> 22
   <212> RNA
   <213> Homo sapiens
<400> 71
   acugcauuau gagcacuuaa agvvv 22
<210> 72
   <211> 22
   <212> RNA
   <213> Homo sapiens
<400> 72
   cucuagaggg aagcgcuuuc ug 22
<210> 73
   <211> 22
   <212> RNA
   <213> Homo sapiens
<400> 73
   cggguggauc acgaugcaau uu 22
<210> 74
   <211> 21
   <212> RNA
   <213> Homo sapiens
<400> 74
   agagaagaag aucagccugc a 21
<210> 75
   <211> 21
   <212> RNA
   <213> Homo sapiens
<400> 75
   guggcugcac ucacuuccuu c 21
<210> 76
   <211> 20
   <212> RNA
   <213> Homo sapiens
<400> 76
   ccucugggcc cuuccuccag 20
<210> 77
   <211> 21
   <212> RNA
   <213> Homo sapiens
<400> 77
   aguuaaugaa uccuggaaag u 21
<210> 78
   <211> 21
   <212> RNA
   <213> Homo sapiens
<400> 78
   uccuucugcu ccguccccca g 21
<210> 79
   <211> 22
   <212> RNA
   <213> Homo sapiens
<400> 79
   caaagcgcuc cccuuuagag gu 22
<210> 80
   <211> 23
   <212> RNA
   <213> Homo sapiens
<400> 80
   ugaggggcag agagcgagac uuu 23
<210> 81
   <211> 23
   <212> RNA
   <213> Homo sapiens
<400> 81
   uaaagugcuu auagugcagg uag 23
<210> 82
   <211> 20
   <212> RNA
   <213> Homo sapiens
<400> 82
   uacaguauag augauguacu 20
<210> 83
   <211> 22
   <212> RNA
   <213> Homo sapiens
<400> 83
   ggauaucauc auauacugua ag 22
<210> 84
   <211> 22
   <212> RNA
   <213> Homo sapiens
<400> 84
   auauaauaca accugcuaag ug 22
<210> 85
   <211> 22
   <212> RNA
   <213> Homo sapiens
<400> 85
   ucguaccgug aguaauaaug cg 22
<210> 86
   <211> 23
   <212> RNA
   <213> Homo sapiens
<400> 86
   caaagugcuu acagugcagg uag 23
<210> 87
   <211> 22
   <212> RNA
   <213> Homo sapiens
<400> 87
   ugcaacuuac cugagucauu ga 22
<210> 88
   <211> 23
   <212> RNA
   <213> Homo sapiens
<400> 88
   caaagugcuc auagugcagg uag 23
<210> 89
   <211> 22
   <212> RNA
   <213> Homo sapiens
<400> 89
   cacgcucaug cacacaccca ca 22
<210> 90
   <211> 22
   <212> RNA
   <213> Homo sapiens
<400> 90
   aauugcacgg uauccaucug ua 22
<210> 91
   <211> 22
   <212> RNA
   <213> Homo sapiens
<400> 91
   ucagugcacu acagaacuuu gu 22
<210> 92
   <211> 17
   <212> RNA
   <213> Homo sapiens
<400> 92
   ucucgcuggg gccucca 17
<210> 93
   <211> 21
   <212> RNA
   <213> Homo sapiens
<400> 93
   uucaaguaau ucaggauagg u 21
<210> 94
   <211> 23
   <212> RNA
   <213> Homo sapiens
<400> 94
   uaaggugcau cuagugcaga uag 23
<210> 95
   <211> 20
   <212> RNA
   <213> Homo sapiens
<400> 95
   aaaagcuggg uugagagggu 20
<210> 96
   <211> 23
   <212> RNA
   <213> Homo sapiens
<400> 96
   uagugcaaua uugcuuauag ggu 23
<210> 97
   <211> 22
   <212> RNA
   <213> Homo sapiens
<400> 97
   ucagugcauc acagaacuuu gu 22
<210> 98
   <211> 19
   <212> RNA
   <213> Homo sapiens
<400> 98
   aaaagcuggg uugagagga 19
<210> 99
   <211> 22
   <212> RNA
   <213> Homo sapiens
<400> 99
   uucaccaccu ucuccaccca gc 22
<210> 100
   <211> 22
   <212> RNA
   <213> Homo sapiens
<400> 100
   aaaagcuggg uugagagggc aa 22
<210> 101
   <211> 22
   <212> RNA
   <213> Homo sapiens
<400> 101
   ugagaacuga auuccauagg cu 22
<210> 102
   <211> 23
   <212> RNA
   <213> Homo sapiens
<400> 102
   uuuggcacua gcacauuuuu gcu 23
<210> 103
   <211> 22
   <212> RNA
   <213> Homo sapiens
<400> 103
   aaaagcuggg uugagagggc ga 22
<210> 104
   <211> 23
   <212> RNA
   <213> Homo sapiens
<400> 104
   cggcccgggc ugcugcuguu ccu 23
<210> 105
   <211> 22
   <212> RNA
   <213> Homo sapiens
<400> 105
   ugaguauuac auggccaauc uc 22
<210> 106
   <211> 21
   <212> RNA
   <213> Homo sapiens
<400> 106
   uacaguacug ugauaacuga a 21
<210> 107
   <211> 22
   <212> RNA
   <213> Homo sapiens
<400> 107
   ucaccagccc uguguucccu ag 22
<210> 108
   <211> 22
   <212> RNA
   <213> Homo sapiens
<400> 108
   uauugcacau uacuaaguug ca 22
<210> 109
   <211> 22
   <212> RNA
   <213> Homo sapiens
<400> 109
   ugagguagua guuuguacag uu 22
<210> 110
   <211> 22
   <212> RNA
   <213> Homo sapiens
<400> 110
   uagcuuauca gacugauguu ga 22
<210> 111
   <211> 23
   <212> RNA
   <213> Homo sapiens
<400> 111
   caaagugcug uucgugcagg uag 23
<210> 112
   <211> 23
   <212> RNA
   <213> Homo sapiens
<400> 112
   cagugcaaua guauugucaa agc 23
<210> 113
   <211> 21
   <212> RNA
   <213> Homo sapiens
<400> 113
   uucacagugg cuaaguucug c 21
<210> 114
   <211> 23
   <212> RNA
   <213> Homo sapiens
<400> 114
   acuugggcac ugaaacaaug ucc 23
<210> 115
   <211> 22
   <212> RNA
   <213> Homo sapiens
<400> 115
   acuccagccc cacagccuca gc 22
<210> 116
   <211> 21
   <212> RNA
   <213> Homo sapiens
<400> 116
   uaaagugcug acagugcaga u 21
<210> 117
   <211> 20
   <212> RNA
   <213> Homo sapiens
<400> 117
   gugcauugcu guugcauugc 20
<210> 118
   <211> 21
   <212> RNA
   <213> Homo sapiens
<400> 118
   agcuacaucu ggcuacuggg u 21
<210> 119
   <211> 22
   <212> RNA
   <213> Homo sapiens
<400> 119
   aacacaccua uucaaggauu ca 22
<210> 120
   <211> 22
   <212> RNA
   <213> Homo sapiens
<400> 120
   ugucuacuac uggagacacu gg 22
<210> 121
   <211> 22
   <212> RNA
   <213> Homo sapiens
<400> 121
   caacuagacu gugagcuucu ag 22
<210> 122
   <211> 22
   <212> RNA
   <213> Homo sapiens
<400> 122
   ccuauucuug auuacuuguu uc 22
<210> 123
   <211> 23
   <212> RNA
   <213> Homo sapiens
<400> 123
   ugucugcccg caugccugcc ucu 23
<210> 124
   <211> 22
   <212> RNA
   <213> Homo sapiens
<400> 124
   ugguugacca uagaacaugc gc 22
<210> 125
   <211> 22
   <212> RNA
   <213> Homo sapiens
<400> 125
   auaagacgaa caaaagguuu gu 22
<210> 126
   <211> 22
   <212> RNA
   <213> Homo sapiens
<400> 126
   acaguagucu gcacauuggu ua 22
<210> 127
   <211> 21
   <212> RNA
   <213> Homo sapiens
<400> 127
   aaggcagggc ccccgcuccc c 21
<210> 128
   <211> 23
   <212> RNA
   <213> Homo sapiens
<400> 128
   ucucacacag aaaucgcacc cgu 23
<210> 129
   <211> 22
   <212> RNA
   <213> Homo sapiens
<400> 129
   caucuuccag uacaguguug ga 22
<210> 130
   <211> 22
   <212> RNA
   <213> Homo sapiens
<400> 130
   uuuucaacuc uaaugggaga ga 22
<210> 131
   <211> 20
   <212> RNA
   <213> Homo sapiens
<400> 131
   ucacuguuca gacaggcgga 20
<210> 132
   <211> 22
   <212> RNA
   <213> Homo sapiens
<400> 132
   uccgagccug ggucucccuc uu 22
<210> 133
   <211> 22
   <212> RNA
   <213> Homo sapiens
<400> 133
   uuguacaugg uaggcuuuca uu 22
<210> 134
   <211> 19
   <212> RNA
   <213> Homo sapiens
<400> 134
   aggcugcgga auucaggac 19
<210> 135
   <211> 22
   <212> RNA
   <213> Homo sapiens
<400> 135
   gcuauuucac gacaccaggg uu 22
<210> 136
   <211> 22
   <212> RNA
   <213> Homo sapiens
<400> 136
   accguggcuu ucgauuguua cu 22
<210> 137
   <211> 21
   <212> RNA
   <213> Homo sapiens
<400> 137
   gaaggcgcuu cccuuuggag u 21
<210> 138
   <211> 21
   <212> RNA
   <213> Homo sapiens
<400> 138
   uugaaaggcu auuucuuggu c 21
<210> 139
   <211> 21
   <212> RNA
   <213> Homo sapiens
<400> 139
   aaagugcuuc cuuuuugagg g 21
<210> 140
   <211> 23
   <212> RNA
   <213> Homo sapiens
<400> 140
   uaaauuucac cuuucugaga agg 23
<210> 141
   <211> 22
   <212> RNA
   <213> Homo sapiens
<400> 141
   ggcuacaaca caggacccgg gc 22
<210> 142
   <211> 22
   <212> RNA
   <213> Homo sapiens
<400> 142
   aggcggggcg ccgcgggacc gc 22
<210> 143
   <211> 22
   <212> RNA
   <213> Homo sapiens
<400> 143
   aaaaguacuu gcggauuuug cu 22
<210> 144
   <211> 22
   <212> RNA
   <213> Homo sapiens
<400> 144
   gaaguuguuc gugguggauu eg 22
<210> 145
   <211> 22
   <212> RNA
   <213> Homo sapiens
<400> 145
   acgcccuucc cccccuucuu ca 22
<210> 146
   <211> 21
   <212> RNA
   <213> Homo sapiens
<400> 146
   aggcggagac uugggcaauu g 21
<210> 147
   <211> 22
   <212> RNA
   <213> Homo sapiens
<400> 147
   cguguucaca gcggaccuug au 22
<210> 148
   <211> 22
   <212> RNA
   <213> Homo sapiens
<400> 148
   cagugcaaug uuaaaagggc au 22
<210> 149
   <211> 23
   <212> RNA
   <213> Homo sapiens
<400> 149
   aaugacacga ucacucccgu uga 23
<210> 150
   <211> 19
   <212> RNA
   <213> Homo sapiens
<400> 150
   ugagcugcug uaccaaaau 19
<210> 151
   <211> 22
   <212> RNA
   <213> Homo sapiens
<400> 151
   cggaugagca aagaaagugg uu 22
<210> 152
   <211> 22
   <212> RNA
   <213> Homo sapiens
<400> 152
   ggauuccugg aaauacuguu cu 22
<210> 153
   <211> 22
   <212> RNA
   <213> Homo sapiens
<400> 153
   ucuacaaagg aaagcgcuuu cu 22
<210> 154
   <211> 20
   <212> RNA
   <213> Homo sapiens
<400> 154
   ccauggaucu ccaggugggu 20
<210> 155
   <211> 21
   <212> RNA
   <213> Homo sapiens
<400> 155
   uguucaugua gauguuuaag c 21
<210> 156
   <211> 22
   <212> RNA
   <213> Homo sapiens
<400> 156
   aagugcuguc auagcugagg uc 22
<210> 157
   <211> 22
   <212> RNA
   <213> Homo sapiens
<400> 157
   cugggaggug gauguuuacu uc 22
<210> 158
   <211> 21
   <212> RNA
   <213> Homo sapiens
<400> 158
   uaccacaggg uagaaccacg g 21
<210> 159
   <211> 22
   <212> RNA
   <213> Homo sapiens
<400> 159
   ugugacuggu ugaccagagg gg 22
<210> 160
   <211> 22
   <212> RNA
   <213> Homo sapiens
<400> 160
   uucaaguaau ccaggauagg cu 22
<210> 161
   <211> 21
   <212> RNA
   <213> Homo sapiens
<400> 161
   cggcggggac ggcgauuggu c 21
<210> 162
   <211> 22
   <212> RNA
   <213> Homo sapiens
<400> 162
   acaggugagg uucuugggag cc 22
<210> 163
   <211> 22
   <212> RNA
   <213> Homo sapiens
<400> 163
   ucagcaaaca uuuauugugu gc 22
<210> 164
   <211> 20
   <212> RNA
   <213> Homo sapiens
<400> 164
   ccccagggcg acgcggcggg 20
<210> 165
   <211> 21
   <212> RNA
   <213> Homo sapiens
<400> 165
   cacaagguau ugguauuacc u 21
<210> 166
   <211> 22
   <212> RNA
   <213> Homo sapiens
<400> 166
   uggaguguga caaugguguu ug 22
<210> 167
   <211> 22
   <212> RNA
   <213> Homo sapiens
<400> 167
   uggguggucu ggagauuugu gc 22
<210> 168
   <211> 21
   <212> RNA
   <213> Homo sapiens
<400> 168
   cuagacugaa gcuccuugag g 21
<210> 169
   <211> 21
   <212> RNA
   <213> Homo sapiens
<400> 169
   cccggagcca ggaugcagcu c 21
<210> 170
   <211> 22
   <212> RNA
   <213> Homo sapiens
<400> 170
   ucaguaaaug uuuauuagau ga 22
<210> 171
   <211> 23
   <212> RNA
   <213> Homo sapiens
<400> 171
   gacacgggcg acagcugcgg ccc 23
<210> 172
   <211> 23
   <212> RNA
   <213> Homo sapiens
<400> 172
   gaagugcuuc gauuuugggg ugu 23
<210> 173
   <211> 23
   <212> RNA
   <213> Homo sapiens
<400> 173
   aaacucuacu uguccuucug agu 23
<210> 174
   <211> 22
   <212> RNA
   <213> Homo sapiens
<400> 174
   uagcagcaca ucaugguuua ca 22
<210> 175
   <211> 22
   <212> RNA
   <213> Homo sapiens
<400> 175
   gaugagcuca uuguaauaug ag 22
<210> 176
   <211> 22
   <212> RNA
   <213> Homo sapiens
<400> 176
   aaagugcuuc cuuuuagagg gu 22
<210> 177
   <211> 22
   <212> RNA
   <213> Homo sapiens
<400> 177
   gaggguuggg uggaggcucu cc 22
<210> 178
   <211> 21
   <212> RNA
   <213> Homo sapiens
<400> 178
   uggacugccc ugaucuggag a 21
<210> 179
   <211> 21
   <212> RNA
   <213> Homo sapiens
<400> 179
   gcgacccaua cuugguuuca g 21
<210> 180
   <211> 23
   <212> RNA
   <213> Homo sapiens
<400> 180
   caacggaauc ccaaaagcag cug 23
<210> 181
   <211> 22
   <212> RNA
   <213> Homo sapiens
<400> 181
   aaaaguaauu gugguuuuug cc 22
<210> 182
   <211> 22
   <212> RNA
   <213> Homo sapiens
<400> 182
   aaucacuaac cacacggcca gg 22
<210> 183
   <211> 21
   <212> RNA
   <213> Homo sapiens
<400> 183
   gugcauugua guugcauugc a 21
<210> 184
   <211> 23
   <212> RNA
   <213> Homo sapiens
<400> 184
   uggaguccag gaaucugcau uuu 23
<210> 185
   <211> 22
   <212> RNA
   <213> Homo sapiens
<400> 185
   uagcaaaaac ugcaguuacu uu 22
<210> 186
   <211> 21
   <212> RNA
   <213> Homo sapiens
<400> 186
   gcaguccaug ggcauauaca c 21
<210> 187
   <211> 22
   <212> RNA
   <213> Homo sapiens
<400> 187
   auccgcgcuc ugacucucug cc 22
<210> 188
   <211> 22
   <212> RNA
   <213> Homo sapiens
<400> 188
   agucauugga ggguuugagc ag 22
<210> 189
   <211> 22
   <212> RNA
   <213> Homo sapiens
<400> 189
   cuauacaacc uacugccuuc cc 22
<210> 190
   <211> 22
   <212> RNA
   <213> Homo sapiens
<400> 190
   gagcuuauuc auaaaagugc ag 22
<210> 191
   <211> 22
   <212> RNA
   <213> Homo sapiens
<400> 191
   augcugacau auuuacuaga gg 22
<210> 192
   <211> 21
   <212> RNA
   <213> Homo sapiens
<400> 192
   aagugaucua aaggccuaca u 21
<210> 193
   <211> 22
   <212> RNA
   <213> Homo sapiens
<400> 193
   uugcauaguc acaaaaguga uc 22
<210> 194
   <211> 22
   <212> RNA
   <213> Homo sapiens
<400> 194
   uaguacugug cauaucaucu au 22
<210> 195
   <211> 23
   <212> RNA
   <213> Homo sapiens
<400> 195
   ugugcaaauc uaugcaaaac uga 23
<210> 196
   <211> 21
   <212> RNA
   <213> Homo sapiens
<400> 196
   caacaccagu cgaugggcug u 21
<210> 197
   <211> 22
   <212> RNA
   <213> Homo sapiens
<400> 197
   uauugcacuc gucccggccu cc 22
<210> 198
   <211> 23
   <212> RNA
   <213> Homo sapiens
<400> 198
   ugauuguagc cuuuuggagu aga 23
<210> 199
   <211> 22
   <212> RNA
   <213> Homo sapiens
<400> 199
   ugcggggcua gggcuaacag ca 22
<210> 200
   <211> 19
   <212> RNA
   <213> Homo sapiens
<400> 200
   gggcgccugu gaucccaac 19
<210> 201
   <211> 22
   <212> RNA
   <213> Homo sapiens
<400> 201
   gaauguugcu cggugaaccc cu 22
<210> 202
   <211> 22
   <212> RNA
   <213> Homo sapiens
<400> 202
   aacuggauca auuauaggag ug 22
<210> 203
   <211> 23
   <212> RNA
   <213> Homo sapiens
<400> 203
   uuauugcuua agaauacgcg uag 23
<210> 204
   <211> 21
   <212> RNA
   <213> Homo sapiens
<400> 204
   ugacaacuau ggaugagcuc u 21
<210> 205
   <211> 22
   <212> RNA
   <213> Homo sapiens
<400> 205
   cugccaauuc cauaggucac ag 22
<210> 206
   <211> 22
   <212> RNA
   <213> Homo sapiens
<400> 206
   ccucuagaug gaagcacugu cu 22
<210> 207
   <211> 22
   <212> RNA
   <213> Homo sapiens
<400> 207
   ucuucucugu uuuggccaug ug 22
<210> 208
   <211> 23
   <212> RNA
   <213> Homo sapiens
<400> 208
   agcagcauug uacagggcua uga 23
<210> 209
   <211> 22
   <212> RNA
   <213> Homo sapiens
<400> 209
   ugccugucua cacuugcugu gc 22
<210> 210
   <211> 23
   <212> RNA
   <213> Homo sapiens
<400> 210
   ccucagggcu guagaacagg gcu 23
<210> 211
   <211> 23
   <212> RNA
   <213> Homo sapiens
<400> 211
   guccaguuuu cccaggaauc ccu 23
<210> 212
   <211> 22
   <212> RNA
   <213> Homo sapiens
<400> 212
   cucuagaggg aagcgcuuuc ug 22
<210> 213
   <211> 22
   <212> RNA
   <213> Homo sapiens
<400> 213
   cuauacgacc ugcugccuuu cu 22
<210> 214
   <211> 22
   <212> RNA
   <213> Homo sapiens
<400> 214
   cuggcccucu cugcccuucc gu 22
<210> 215
   <211> 22
   <212> RNA
   <213> Homo sapiens
<400> 215
   aguggggaac ccuuccauga gg 22
<210> 216
   <211> 22
   <212> RNA
   <213> Homo sapiens
<400> 216
   ugcccugugg acucaguucu gg 22
<210> 217
   <211> 22
   <212> RNA
   <213> Homo sapiens
<400> 217
   cucggcgcgg ggcgcgggcu cc 22
<210> 218
   <211> 21
   <212> RNA
   <213> Homo sapiens
<400> 218
   cuguacaggc cacugccuug c 21
<210> 219
   <211> 21
   <212> RNA
   <213> Homo sapiens
<400> 219
   cacugugucc uuucugcgua g 21
<210> 220
   <211> 22
   <212> RNA
   <213> Homo sapiens
<400> 220
   auaaagcuag auaaccgaaa gu 22
<210> 221
   <211> 22
   <212> RNA
   <213> Homo sapiens
<400> 221
   uccauuacac uacccugccu cu 22
<210> 222
   <211> 22
   <212> RNA
   <213> Homo sapiens
<400> 222
   agggacggga cgcggugcag ug 22
<210> 223
   <211> 23
   <212> RNA
   <213> Homo sapiens
<400> 223
   cugggaucuc cggggucuug guu 23
<210> 224
   <211> 20
   <212> RNA
   <213> Homo sapiens
<400> 224
   cgugccaccc uuuuccccag 20
<210> 225
   <211> 22
   <212> RNA
   <213> Homo sapiens
<400> 225
   cucuagaggg aagcgcuuuc ug 22
<210> 226
   <211> 25
   <212> RNA
   <213> Homo sapiens
<400> 226
   cuagugaggg acagaaccag gauuc 25
<210> 227
   <211> 22
   <212> RNA
   <213> Homo sapiens
<400> 227
   gaaagcgcuu cccuuugcug ga 22
<210> 228
   <211> 22
   <212> RNA
   <213> Homo sapiens
<400> 228
   caggccauau ugugcugccu ca 22
<210> 229
   <211> 23
   <212> RNA
   <213> Homo sapiens
<400> 229
   ucauagcccu guacaaugcu gcu 23
<210> 230
   <211> 21
   <212> RNA
   <213> Homo sapiens
<400> 230
   ugagaugaag cacuguagcu c 21
<210> 231
   <211> 21
   <212> RNA
   <213> Homo sapiens
<400> 231
   guguugaaac aaucucuacu g 21
<210> 232
   <211> 22
   <212> RNA
   <213> Homo sapiens
<400> 232
   acuggacuua gggucagaag gc 22
<210> 233
   <211> 21
   <212> RNA
   <213> Homo sapiens
<400> 233
   guucaaaucc agaucuauaa c 21
<210> 234
   <211> 22
   <212> RNA
   <213> Homo sapiens
<400> 234
   aggcagugua uuguuagcug gc 22
<210> 235
   <211> 22
   <212> RNA
   <213> Homo sapiens
<400> 235
   acagauucga uucuagggga au 22
<210> 236
   <211> 26
   <212> RNA
   <213> Homo sapiens
<400> 236
   gaugaugaug gcagcaaauu cugaaa 26
<210> 237
   <211> 21
   <212> RNA
   <213> Homo sapiens
<400> 237
   acucuagcug ccaaaggcgc u 21
<210> 238
   <211> 22
   <212> RNA
   <213> Homo sapiens
<400> 238
   uccagcauca gugauuuugu ug 22
<210> 239
   <211> 23
   <212> RNA
   <213> Homo sapiens
<400> 239
   cuggagauau ggaagagcug ugu 23
<210> 240
   <211> 22
   <212> RNA
   <213> Homo sapiens
<400> 240
   aauugcacuu uagcaauggu ga 22
<210> 241
   <211> 22
   <212> RNA
   <213> Homo sapiens
<400> 241
   acugcaguga aggcacuugu ag 22
<210> 242
   <211> 22
   <212> RNA
   <213> Homo sapiens
<400> 242
   uuuugcgaug uguuccuaau au 22
<210> 243
   <211> 22
   <212> RNA
   <213> Homo sapiens
<400> 243
   cugaagcuca gagggcucug au 22
<210> 244
   <211> 21
   <212> RNA
   <213> Homo sapiens
<400> 244
   acucuuuccc uguugcacua c 21
<210> 245
   <211> 24
   <212> RNA
   <213> Homo sapiens
<400> 245
   ucccugagac ccuuuaaccu guga 24
<210> 246
   <211> 22
   <212> RNA
   <213> Homo sapiens
<400> 246
   ugagguagua gauuguauag uu 22
<210> 247
   <211> 24
   <212> RNA
   <213> Homo sapiens
<400> 247
   acugggggcu uucgggcucu gcgu 24
<210> 248
   <211> 21
   <212> RNA
   <213> Homo sapiens
<400> 248
   gugggcgggg gcaggugugu g 21
<210> 249
   <211> 21
   <212> RNA
   <213> Homo sapiens
<400> 249
   cucccacaug caggguuugc a 21
<210> 250
   <211> 21
   <212> RNA
   <213> Homo sapiens
<400> 250
   agggggaaag uucuauaguc c 21
<210> 251
   <211> 21
   <212> RNA
   <213> Homo sapiens
<400> 251
   cauuauuacu uuugguacgc g 21
<210> 252
   <211> 22
   <212> RNA
   <213> Homo sapiens
<400> 252
   uucucaagga ggugucguuu au 22
<210> 253
   <211> 21
   <212> RNA
   <213> Homo sapiens
<400> 253
   ugaguuggcc aucugaguga g 21
<210> 254
   <211> 21
   <212> RNA
   <213> Homo sapiens
<400> 254
   uagcagcaca gaaauauugg c 21
<210> 255
   <211> 24
   <212> RNA
   <213> Homo sapiens
<400> 255
   ucagaacaaa ugccgguucc caga 24
<210> 256
   <211> 23
   <212> RNA
   <213> Homo sapiens
<400> 256
   uauggcuuuu uauuccuaug uga 23
<210> 257
   <211> 22
   <212> RNA
   <213> Homo sapiens
<400> 257
   uuuuucauua uugcuccuga cc 22
<210> 258
   <211> 22
   <212> RNA
   <213> Homo sapiens
<400> 258
   uagcaccauc ugaaaucggu ua 22
<210> 259
   <211> 23
   <212> RNA
   <213> Homo sapiens
<400> 259
   ugcaccaugg uugucugagc aug 23
<210> 260
   <211> 22
   <212> RNA
   <213> Homo sapiens
<400> 260
   uaacacuguc ugguaacgau gu 22
<210> 261
   <211> 22
   <212> RNA
   <213> Homo sapiens
<400> 261
   aagaugugga aaaauuggaa uc 22
<210> 262
   <211> 19
   <212> RNA
   <213> Homo sapiens
<400> 262
   agcugucuga aaaugucuu 19
<210> 263
   <211> 23
   <212> RNA
   <213> Homo sapiens
<400> 263
   uagcaccauu ugaaaucagu guu 23
<210> 264
   <211> 22
   <212> RNA
   <213> Homo sapiens
<400> 264
   uucccuuugu cauccuaugc cu 22
<210> 265
   <211> 21
   <212> RNA
   <213> Homo sapiens
<400> 265
   gcgacccacu cuugguuucc a 21
<210> 266
   <211> 22
   <212> RNA
   <213> Homo sapiens
<400> 266
   aaugcaccug ggcaaggauu ca 22
<210> 267
   <211> 23
   <212> RNA
   <213> Homo sapiens
<400> 267
   uuacaguugu ucaaccaguu acu 23
<210> 268
   <211> 22
   <212> RNA
   <213> Homo sapiens
<400> 268
   ugaccgauuu cuccuggugu uc 22
<210> 269
   <211> 22
   <212> RNA
   <213> Homo sapiens
<400> 269
   ugagguagua guuugugcug uu 22
<210> 270
   <211> 23
   <212> RNA
   <213> Homo sapiens
<400> 270
   ucccuguccu ccaggagcuc acg 23
<210> 271
   <211> 22
   <212> RNA
   <213> Homo sapiens
<400> 271
   aagugccucc uuuuagagug uu 22
<210> 272
   <211> 22
   <212> RNA
   <213> Homo sapiens
<400> 272
   ucaggcucag uccccucccg au 22
<210> 273
   <211> 22
   <212> RNA
   <213> Homo sapiens
<400> 273
   uuuggucccc uucaaccagc ua 22
<210> 274
   <211> 23
   <212> RNA
   <213> Homo sapiens
<400> 274
   ccuaguaggu guccaguaag ugu 23
<210> 275
   <211> 22
   <212> RNA
   <213> Homo sapiens
<400> 275
   aucgugcauc ccuuuagagu gu 22
<210> 276
   <211> 23
   <212> RNA
   <213> Homo sapiens
<400> 276
   ugaguaccgc caugucuguu ggg 23
<210> 277
   <211> 22
   <212> RNA
   <213> Homo sapiens
<400> 277
   aaccaucgac cguugagugg ac 22
<210> 278
   <211> 21
   <212> RNA
   <213> Homo sapiens
<400> 278
   ccgucgccgc cacccgagcc g 21
<210> 279
   <211> 20
   <212> RNA
   <213> Homo sapiens
<400> 279
   auguauaaau guauacacac 20
<210> 280
   <211> 22
   <212> RNA
   <213> Homo sapiens
<400> 280
   ugaaggucua cugugugcca gg 22
<210> 281
   <211> 22
   <212> RNA
   <213> Homo sapiens
<400> 281
   acugcugagc uagcacuucc cg 22
<210> 282
   <211> 19
   <212> RNA
   <213> Homo sapiens
<400> 282
   uggauuuuug gaucaggga 19
<210> 283
   <211> 23
   <212> RNA
   <213> Homo sapiens
<400> 283
   cgcauccccu agggcauugg ugu 23
<210> 284
   <211> 23
   <212> RNA
   <213> Homo sapiens
<400> 284
   agcucggucu gaggccccuc agu 23
<210> 285
   <211> 22
   <212> RNA
   <213> Homo sapiens
<400> 285
   ugcuaugcca acauauugcc au 22
<210> 286
   <211> 22
   <212> RNA
   <213> Homo sapiens
<400> 286
   ccuguucucc auuacuuggc uc 22
<210> 287
   <211> 22
   <212> RNA
   <213> Homo sapiens
<400> 287
   uggcucaguu cagcaggaac ag 22
<210> 288
   <211> 22
   <212> RNA
   <213> Homo sapiens
<400> 288
   caaaacuggc aauuacuuuu gc 22
<210> 289
   <211> 23
   <212> RNA
   <213> Homo sapiens
<400> 289
   uauucauuua uccccagccu aca 23
<210> 290
   <211> 22
   <212> RNA
   <213> Homo sapiens
<400> 290
   cuauacggcc uccuagcuuu cc 22
<210> 291
   <211> 21
   <212> RNA
   <213> Homo sapiens
<400> 291
   aaaacgguga gauuuuguuu u 21
<210> 292
   <211> 22
   <212> RNA
   <213> Homo sapiens
<400> 292
   cgucaacacu ugcugguuuc cu 22
<210> 293
   <211> 22
   <212> RNA
   <213> Homo sapiens
<400> 293
   aucgggaaug ucguguccgc cc 22
<210> 294
   <211> 22
   <212> RNA
   <213> Homo sapiens
<400> 294
   uuccuaugca uauacuucuu ug 22
<210> 295
   <211> 23
   <212> RNA
   <213> Homo sapiens
<400> 295
   uaagugcuuc cauguuugag ugu 23
<210> 296
   <211> 22
   <212> RNA
   <213> Homo sapiens
<400> 296
   caaaaaucuc aauuacuuuu gc 22
<210> 297
   <211> 23
   <212> RNA
   <213> Homo sapiens
<400> 297
   ucuggcuccg ugucuucacu ccc 23
<210> 298
   <211> 22
   <212> RNA
   <213> Homo sapiens
<400> 298
   agggcuuagc ugcuugugag ca 22
<210> 299
   <211> 21
   <212> RNA
   <213> Homo sapiens
<400> 299
   uacucaaaaa gcugucaguc a 21
<210> 300
   <211> 22
   <212> RNA
   <213> Homo sapiens
<400> 300
   aaaaguaauu gcggauuuug cc 22
<210> 301
   <211> 22
   <212> RNA
   <213> Homo sapiens
<400> 301
   uaacugguug aacaacugaa cc 22
<210> 302
   <211> 22
   <212> RNA
   <213> Homo sapiens
<400> 302
   cuauacaguc uacugucuuu cc 22
<210> 303
   <211> 22
   <212> RNA
   <213> Homo sapiens
<400> 303
   uauacaaggg caagcucucu gu 22
<210> 304
   <211> 25
   <212> RNA
   <213> Homo sapiens
<400> 304
   ggcggaggga aguagguccg uuggu 25
<210> 305
   <211> 24
   <212> RNA
   <213> Homo sapiens
<400> 305
   agcagaagca gggagguucu ccca 24
<210> 306
   <211> 22
   <212> RNA
   <213> Homo sapiens
<400> 306
   cugguacagg ccugggggac ag 22
<210> 307
   <211> 22
   <212> RNA
   <213> Homo sapiens
<400> 307
   ugaaacauac acgggaaacc uc 22
<210> 308
   <211> 21
   <212> RNA
   <213> Homo sapiens
<400> 308
   ccuggaaaca cugagguugu g 21
<210> 309
   <211> 25
   <212> RNA
   <213> Homo sapiens
<400> 309
   agggaucgcg ggcggguggc ggccu 25
<210> 310
   <211> 21
   <212> RNA
   <213> Homo sapiens
<400> 310
   uggguuuacg uugggagaac u 21
<210> 311
   <211> 22
   <212> RNA
   <213> Homo sapiens
<400> 311
   gacugacacc ucuuugggug aa 22
<210> 312
   <211> 22
   <212> RNA
   <213> Homo sapiens
<400> 312
   aaaaguaauu gcggucuuug gu 22
<210> 313
   <211> 21
   <212> RNA
   <213> Homo sapiens
<400> 313
   gcuaguccug acucagccag u 21
<210> 314
   <211> 21
   <212> RNA
   <213> Homo sapiens
<400> 314
   ucccacguug uggcccagca g 21
<210> 315
   <211> 22
   <212> RNA
   <213> Homo sapiens
<400> 315
   cuccugagcc auucugagcc uc 22
<210> 316
   <211> 22
   <212> RNA
   <213> Homo sapiens
<400> 316
   aaaaguaauc gcgguuuuug uc 22
<210> 317
   <211> 22
   <212> RNA
   <213> Homo sapiens
<400> 317
   ccaguggggc ugcuguuauc ug 22
<210> 318
   <211> 22
   <212> RNA
   <213> Homo sapiens
<400> 318
   ugagguagua gguuguauag uu 22
<210> 319
   <211> 22
   <212> RNA
   <213> Homo sapiens
<400> 319
   gaaaucaagc gugggugaga cc 22
<210> 320
   <211> 22
   <212> RNA
   <213> Homo sapiens
<400> 320
   ggaggggucc cgcacuggga gg 22
<210> 321
   <211> 22
   <212> RNA
   <213> Homo sapiens
<400> 321
   aagcccuuac cccaaaaagu au 22
<210> 322
   <211> 22
   <212> RNA
   <213> Homo sapiens
<400> 322
   gggagccagg aaguauugau gu 22
<210> 323
   <211> 21
   <212> RNA
   <213> Homo sapiens
<400> 323
   aauggcgcca cuaggguugu g 21
<210> 324
   <211> 22
   <212> RNA
   <213> Homo sapiens
<400> 324
   uuuaacaugg ggguaccugc ug 22
<210> 325
   <211> 21
   <212> RNA
   <213> Homo sapiens
<400> 325
   ucagugcaug acagaacuug g 21
<210> 326
   <211> 22
   <212> RNA
   <213> Homo sapiens
<400> 326
   ggagaaauua uccuuggugu gu 22
<210> 327
   <211> 22
   <212> RNA
   <213> Homo sapiens
<400> 327
   ugauauguuu gauauauuag gu 22
<210> 328
   <211> 22
   <212> RNA
   <213> Homo sapiens
<400> 328
   cugcccuggc ccgagggacc ga 22
<210> 329
   <211> 21
   <212> RNA
   <213> Homo sapiens
<400> 329
   ugagugccgg ugccugcccu g 21
<210> 330
   <211> 22
   <212> RNA
   <213> Homo sapiens
<400> 330
   uagguaguuu cauguuguug gg 22
<210> 331
   <211> 22
   <212> RNA
   <213> Homo sapiens
<400> 331
   uguaacagca acuccaugug ga 22
<210> 332
   <211> 21
   <212> RNA
   <213> Homo sapiens
<400> 332
   auccuugcua ucugggugcu a 21
<210> 333
   <211> 21
   <212> RNA
   <213> Homo sapiens
<400> 333
   caaagaggaa ggucccauua c 21
<210> 334
   <211> 22
   <212> RNA
   <213> Homo sapiens
<400> 334
   gugacaucac auauacggca gc 22
<210> 335
   <211> 22
   <212> RNA
   <213> Homo sapiens
<400> 335
   ugagaccucu ggguucugag cu 22
<210> 336
   <211> 20
   <212> RNA
   <213> Homo sapiens
<400> 336
   ugagcccugu ccucccgcag 20
<210> 337
   <211> 21
   <212> RNA
   <213> Homo sapiens
<400> 337
   uuuugcaccu uuuggaguga a 21
<210> 338
   <211> 21
   <212> RNA
   <213> Homo sapiens
<400> 338
   aacauagagg aaauuccacg u 21
<210> 339
   <211> 22
   <212> RNA
   <213> Homo sapiens
<400> 339
   aaacaaacau ggugcacuuc uu 22
<210> 340
   <211> 24
   <212> RNA
   <213> Homo sapiens
<400> 340
   ccagacagaa uucuaugcac uuuc 24
<210> 341
   <211> 21
   <212> RNA
   <213> Homo sapiens
<400> 341
   ccaguccugu gccugccgcc u 21
<210> 342
   <211> 22
   <212> RNA
   <213> Homo sapiens
<400> 342
   auucuaauuu cuccacgucu uu 22
<210> 343
   <211> 24
   <212> RNA
   <213> Homo sapiens
<400> 343
   agccuggaag cuggagccug cagu 24
<210> 344
   <211> 21
   <212> RNA
   <213> Homo sapiens
<400> 344
   uugcucacug uucuucccua g 21
<210> 345
   <211> 22
   <212> RNA
   <213> Homo sapiens
<400> 345
   caacaaaucc cagucuaccu aa 22
<210> 346
   <211> 21
   <212> RNA
   <213> Homo sapiens
<400> 346
   caagucacua gugguuccgu u 21
<210> 347
   <211> 22
   <212> RNA
   <213> Homo sapiens
<400> 347
   cgcaggggcc gggugcucac cg 22
<210> 348
   <211> 20
   <212> RNA
   <213> Homo sapiens
<400> 348
   caccaggcau uguggucucc 20
<210> 349
   <211> 22
   <212> RNA
   <213> Homo sapiens
<400> 349
   aaugcacccg ggcaaggauu cu 22
<210> 350
   <211> 23
   <212> RNA
   <213> Homo sapiens
<400> 350
   uauggcuuuu cauuccuaug uga 23
<210> 351
   <211> 23
   <212> RNA
   <213> Homo sapiens
<400> 351
   aaggagcuua caaucuagcu ggg 23
<210> 352
   <211> 22
   <212> RNA
   <213> Homo sapiens
<400> 352
   cucuagaggg aagcgcuuuc ug 22
<210> 353
   <211> 24
   <212> RNA
   <213> Homo sapiens
<400> 353
   aauccuugga accuaggugu gagu 24